# EUROPEAN PATENT SPECIFICATION

(11) **EP 1 549 742 B1**
(45) Date of publication and mention of the grant of the patent: **08.01.2014**
(21) Application number: 03811277.7
(22) Date of filing: 07.10.2003
(51) Int. Cl.: G01N 33/50

(54) **METHOD FOR SELECTION OF COMPOUNDS WHICH INHIBIT CLONAL CELL GROWTH AND USE THEREOF**
VERFAHREN ZUR SELEKTION VON DAS KLONZELLWACHSTUM HEMMENDEN VERBINDUNGEN SOWIE VERWENDUNG DAVON
PROCEDE DE SELECTION DE COMPOSES INHIBANT LA CROISSANCE CELLULAIRE CLONALE ET UTILISATION DE CES COMPOSES

(30) Priority: 08.10.2002 NO 20024853
(43) Date of publication of application: 06.07.2005
(73) Proprietor: Tjötta, Enok, 0667 Oslo (NO)
(72) Inventor: Tjötta, Enok, 0667 Oslo (NO)
(74) Representative: Kristiansen, Ewy
(86) International application number: PCT/NO2003/000335
(87) International publication number: WO 2004/055175

(56) References cited:
- WO-A1-01/00585
- SVEN DE VOS ET AL.: 'Effects of retinoid X receptor-selective ligands on proliferation of prostate cancer cells' THE PROSTATE vol. 32, 1997, pages 115 - 121, XP008008733
- MACPHERSON I. ET AL.: 'Agar suspension culture for the selective assay of cells transformed by Polyoma virus' VIROLOGY vol. 23, no. 2, 1964, pages 291 - 294, XP002986691
- DATABASE BIOSIS [Online] BAIRD W.M. ET AL.: 'Comparison of the metabolism of benzo a pyrene and its activation to biologically active metabolites by low passage hamster and rat embryo cells', XP002986680 Database accession no. (PREV198172018181) & CARCINOGENESIS vol. 2, no. 2, 1981, pages 81 - 88
- MEISCHEN S.J. ET AL.: 'Synthesis and anti tumor activity of N phosphonoacetyl-L-aspartato-1 2-diamino cyclo hexane platinum II' JOURNAL OF CLINICAL HEMATOLOGY AND ONCOLOGY vol. 12, no. 3, 1982, pages 67 - 76, XP002987606
- WEPPELMANN B. ET AL.: 'The influence of prostaglandin antagonists on radiation therapy of carcinoma of the cervix' GYNECOLOGIC ONCOLOGY vol. 17, no. 2, 1984, pages 196 - 199, XP002986692
- STOLLER DIANE K. ET AL.: 'Reduction of atherosclerosis with nonsteroidal anti-inflammatory drugs' JOURNAL OF SURGICAL RESEARCH vol. 54, no. 1, January 1993, pages 7 - 11, XP002986693
- BAILEY J.M. ET AL.: 'Anti-inflammatory drugs in experimental atherosclerosis. Part 6. Combination therapy with steroid and non-steroid agents' ATEROSCLEROSIS vol. 54, no. 2, 1985, pages 205 - 212, XP002987607
- IWANUMA O ET AL: "Effects of mechanical stretching on Caspase and IGF-1 expression during the proliferation process of myoblasts", ZOOLOG SCI., vol. 25, no. 3, March 2008 (2008-03), pages 242-247,
- SONG H ET AL: "Emerging role of IGF-1 in stretch-induced neointimal hyperplasia in venous grafts", ARTERIOSCLEROSIS, THROMBOSIS AND VASCULAR BIOLOGY, vol. 27, 2007, page 1679,
- SCHWARTZ S ET AL: ANNU.REV.MED., vol. 49, 1998, page 437.60,
- TJOTTA E ET AL: J.ARTERIOSCLEROSI RES., vol. 3, 1963, pages 253-261,
- WRAIGHT J ET AL: NATURE BIOTECHNOLOGY, vol. 18, 2000, pages 521-526,
- HAS XU S ET AL: "Altered Insulin-like Growth Factor-II (IGF-II) Level and IGF-Binding Protein-3 (IGFBP-3) Protease Activity in Interstitial Fluid Taken from the Skin Lesion of Psoriasis", J.INVEST.DERMATOL., vol. 106, no. 1, January 1996 (1996-01), pages 109-112,

## Description

### FIELD OF THE INVENTION

The present invention relates to the use of 4-OH-OPB for preparing a pharmaceutical preparation for the treatment of psoriasis or for the treatment or prophylactics of arteriosclerosis or cancer, with the provision that said cancer is not malignancies derived from CD4 lymphocytes and HIV related Kaposis sarcoma.

A method for selection and testing of compounds inhibiting clonal cell growth and use of such compounds is described herein. Drugs harbouring said compounds are able to decrease or stop cloning of normal cells or tumour cells, it should therefore be possible to decrease or even abolish the development of malignant disease, local infiltration, metastasis from primary tumours and probably prevent atheromatosis, a monoclonal proliferation, in its start.

The export of migrating tumour cells transplanted to a subcutaneous location has been shown to be inhibited or stopped by using the same substance that inhibited clonal growth. But this effect did not show relation to cell density, as did clonal inhibition. It is anticipated that dcted clonal inhibitors or stimulators also will be active against other diseases where cloning might be involved in the pathogenesis (e.g. atheromatosis). Finally, clonal stimulators might be detected by the same kind of tests as clonal inhibitors and the responsible agents might be carcinogenic. It is important to know if drugs, internal physiological and pathological substances, or pathological agents with such activity were generated in the body or might originate from food or other external sources.

### BACKGROUND OF THE INVENTION

A basic assumption for the development of malignant tumours or its metastases is that they usually are developed from single cells that had to be cloned before developing a primary tumour or a metastasis and that inhibiting these processes should be beneficial.

The process of cloning is a "weak point" or a period in the life of a cell that has special needs. In cell culture the cloning procedure is facilitated by growth factors e.g. conditioned medium, insulin, insulin like growth factor, growth hormone, cytokines, serum extenders (e.g. Mito+) etc. in addition to serum (experiments number 3,25)

During organogenesis in the embryo, several cell clones are created. Later these clones develop organs that all together constitute the individual.

When tumours arise later in life, also these are clones. New clones in a developed individual seem not to fit in among normal organs, and tumours with consequences ranging from none to lethal might be the result.

With a drug that is able to decrease or stop cloning of normal cells or tumour cells, it should be possible to decrease or even abolish the development of malignant disease or metastases from primary tumours.

However, it is important to be aware of, that if a drug only acts against cloning, growth of existing multi-cellular tumours don't need to be stopped since no extra cloning of these multi-cellular tumours is necessary for their further expansion.

Therefore, treatment of malignant growth using selective clonal inhibitors will only stop cells that were apart from each other, not multi-cellular tumours. The latter have to be removed or inactivated by irradiation or other conventional treatment. The cytostatic activity of clonal inhibitors, if any at all, will probably be much less than for regular cytostatics. Therefore, it is anticipated that it will be possible to continue the treatment over much longer time periods with selective clonal inhibitors that do not possess regular cytostatic activity. Continuous treatment of selected cancer patients might protect against recurrences in a way not possible with classical cytostatics.

Cancer patients expected to profit most on anti-clonal treatment are those being radically treated for a malignancy without known metastases or where detected metastases had been removed or otherwise neutralised. Statistically some of the conventionally and radically treated patients will get recurrences or metastases later. It is expected that use of the compounds of the invention will reduce or in some instances stop these recurrences.

Tumours with known rest tumour might also benefit, since the export of metastatic cells from the rest-tumour and local infiltration might be stopped. This will probably induce a more benign appearance of the disease.

Leukaemia with no solid tumours should be a good choice for treatment since collocation inhibition is expected to be small and development of resistance against chemotherapy reduced since new clones should be significantly reduced in number.

Treatment with the aim of reducing or stopping spread of metastatic cells might be desirable during conventional treatment. Otherwise conventional treatment might loose control of metastatic cells.

In persons with high risk of getting malignant tumours (e.g. familiar breast or colon cancer) suitable drugs detected by the method of the invention might probably have valuable prophylactic effect.

Arteriosclerosis has been shown to contain cloned cells either developed de novo or from previously cloned cells. Growth enhancers might be an important pathogenic factor and it has been shown that stretching of cultured cells induced liberation of such (Standley, PR et al (1999) Am J Physiol, 276, E697-705). Stretching of vascular cells might occur chiefly in the curves of thicker parts of coronary arteries (E. Tjøtta, The distribution of Atheromatosis in the Coronary Arteries, J. Atheroscler. Res. 3 (1963) 253-261). These parts are also affected significantly more by atheromatosis than straight parts that probably were bended less. Thinner parts of the arteries were not affected by atheromatosis probably because bending will provide less local stretching of the wall. In addition mechanical factors and infections might increase inner pores of arteries witch resulting oozing inn of growth factors from serum. Experiments described later clearly show that growth factors increase the clonal growth of normal cells in agar (Experiments 3, 25).

Moreover, recurrences of Herpes virus infections may be initiated by some local or general cell destruction as mediated through UV light, low temperature, menstruation etc. It is known that ultrasound and freezing and thawing can induce liberation of insulin like growth factor. Stretching of vascular cells can as well liberate this factor as indicated above. Therefore, Herpes simlex virus that has been found in the arterial wall, might "seek" the parts of the vessel where bending induces these factors and possibly participate in the local pathogenesis.

Bigger clones or atheromas will probably be hard to attack by said substances because of collocation inhibition since this inhibition of the reduction of clonal growth is shown also for normal cells in culture. Prophylactics probably are easier. Such therapy might prevent restenosis in connection with introducing stents, bypass operations or endarterectomies.

For patients with familiar high-risk, specific prophylactics seem desirable. However, for the man in the street, prophylactic measures through food, vitamins or natural products as plant extracts seems better, but foregoing specific testing of such products is needed.

The sensitivity of individual malignant tumours might be tested in assays based on cloning. It is the inventor's view that the consequences of the findings in existing tests have not been interpreted correctly. An example of this is an article written by David H. Kern and Carl A. Bertelsen in International Advances in Surgical Oncology 7:187-213 (1984); Present Status of Chemo sensitivity Assays. In this article four studies were summarised in Table II on page 198 with the following conclusion: *"It is evident from each of these studies that the assay is very accurate for predicting tumor resistance. However, the assay is only about 60% accurate for predicting tumor sensitivity to a given agent. "* The opinion of the inventor is that this discrepancy might have a special explanation. When a drug is testing positive for activity against a certain tumour, the result indicates that the drug is active as a clonal inhibitor since the test used was a test where clones were inhibited by the drug. The drug, however, does not need to be fully effective, or effective at all, against tumours having reached a multi-cellular stage. Only compounds possessing general anti-mitotic or other cytostatic activity in addition to clonal inhibition would be expected to be able to stop both post-clonal and clonal growth.

However, when testing negatively in a clonal test, the result indicates no effect on cloning and no other anti-growth effect that may be effective against any stage in tumour development.

Therefore, a positive result in a clonal inhibitory test might mislead the oncologist to believe that the drug could be used also for treating tumours that are bigger than a certain maximum.

In the following there will be presented data indicating that an inhibitor of cloning is more active when the cells are seeded at low density compared to higher density. Thus high collocation of cells seemed to inhibit the effect of clonal inhibitors and was called collocation inhibition of clonal inhibitors. It is supposed that the less the collocation inhibition of the clonal inhibitor is, the more toxic the substance would be for the body with its multicellular organs.

Further, data indicating inhibition of the initiation of cell migration or export of malignant cells to other locations in the body by candidate anti-cancer drugs is also included (Experiment 9).

The method described herein comprises a three-phasic test for screening or testing the effect of drugs, potential drugs, food, food additives, toxins, potential toxins, pollutants, chemical compounds or physiological and pathological components from the body for specific inhibition or stimulation of clonal growth.

In prior art testing for drugs or potential drugs for anti-cancer effect only a single phasic test (e.g. Macpherson and Montagniers soft agar cloning technique, 1964, Virology, 23, 291-294) has been used for this purpose. It is the inventors opinion that said test is inaccurate and has in addition been misinterpreted. The three-phasic test will in contrast to prior art give more precise data of the expected clinical effect of the candidate drug that is tested. The data obtained from the method might indicate the optimal clinical stage for a particular drug and also give an indication of which drug would be optimal for a particular patient and if and how conventional treatment has to be included in the therapy.

### SUMMARY OF THE INVENTION

The present invention is based on the assumption that the easiest way to stop a malignant tumour would be to:
1. Stop the cloning that initiates tumour growth.
2. On a later stage to stop single cells outside an established tumour in a growth that is thought to be more or less clonal and would lead to local infiltration.
3. To stop the export of single cells from the primary tumour trough the vascular system.
4. In addition it is important to stop the establishment of single cells or groups of very few cells arriving in other organs before they settle as new metastatic clones.

All these qualities of the malignant process might be attacked if there could be found a drug with the general ability to stop the cloning procedure without toxic effect on the organism or densely collocated cells.

To detect such compounds a method has been established that is able to control the qualities of substances found by screening. This method might have many modifications.

The present invention comprises:
A method of selection and testing of drugs, potential drugs, food, food additives, toxins, potential toxins, components from physiological or pathological processes, chemical compounds including microbes, the outcome of physical effects (e.g. radioactivity or ultrasound) on the body, parts of the body or on said substances for specific anti-cloning or clonal stimulating effect, the said method comprises the following:
   a clonal test to study the effect on cloning of said substances and;
   a collocation inhibition test to study how increase of local cell concentration (increase of cell collocation) may decrease or abrogate the effect of said substances or physical effects on the process of cloning and on toxicity and;
   tests for the ability of said substances or physical effects to influence the development of metastases in other ways than on cloning, e.g. on export of metastatic cells from a malignant tumour or location containing tumour cells.

The present invention relates to the use of 4-OH-OPB for preparing a pharmaceutical preparation for the treatment of psoriasis or for the treatment or prophylactics of arteriosclerosis or cancer, with the provision that said cancer is not malignancies derived from CD4 lymphocytes and HIV related Kaposis sarcoma.

The present invention is based on the assumption that cloning is the "weak point" in the genesis of a malignant tumour or its metastases and that drugs can be selected in cell cultures when showing a sufficient inhibition of the process of cloning.

The export of metastatic cells from a tumour cell containing location is shown to be stopped by a clonal inhibitor, 4-OH-OPB. However, other collocated tumour cells did not inhibit this effect.

The method described herein is suitable for screening many substances and will probably make it possible to detect a number of non-toxic active inhibitors of cloning.

After the candidate drug has been selected in tissue culture, there is also need for studying the toxic effects in cell culture, animals or man and to test the inhibiting potential on tumours and the effect on tumour cell spread in the body.

With an effective clonal inhibition, the therapy of malignant tumours could probably be much more aggressive than before, since the chance of getting new metastases as a result, will be reduced or abolished. Instead of palliative measures, sub-radical tumour treatment could enter the scene since clonal inhibition and anti-migratory drug effects might be stopping the feared dissemination of malignant cells.

After conventional radical cancer treatment, many patients will develop recurrences or distant metastases probably mostly from silent single tumour cells. Clonal inhibitors are expected to arrest such seeds. In the described experiments in mice, at least100000 tumour cells transplanted to peritoneum were stopped.

The other aspect of the test is to detect clonal stimulators. The inventor has shown the effect of some; insulin, conditioned medium, serum and Mito+ (experiment number 3). Clonal stimulation might well have carcinogenic properties or stimulate the development of atheromatosis and might therefore yield results of interest for development of new drugs, control of existing drugs, food, food additives, preserving agents, microbes and chemicals.

The results would also be of interest when studying physiological processes, body fluids in healthy individuals and people having a disease, especially during tumour disease and in infections. But also arteriosclerosis might depend on cloning of cells in the inner arterial wall. People with increased risk of getting tumours with genetic, environmental, toxic or physical aetiology and arteriosclerosis or other diseases that might be related to cloning, could be studied with the aim of finding effective drugs among the detected compounds having effects against these pathological mechanisms.

Since metastases might arise from single cells, each of them represents a subclone. It is the experience of the inventor, after having done many cloning experiments, that nearly each new clone consists of cells that behave differently compared to the parent cell strain or other clones. It is expected that this would also happen in the body when the malignant disease progresses. To avoid such tumours consisting of a library of different properties, the production of new metastatic clones should be inhibited as indicated. This will probably also inhibit the development of therapy resistant clones. Then, if needed, conventional chemotherapy might be more effective.

Still conventional cancer treatment is considered necessary at the time of diagnosis since at the time of diagnosis the tumour will always be bigger than single cells or a few cells. But the indications for treatment, with the aim of curing the disease, might be widened since the clonal inhibitors might save those already having small metastases and inhibit export of new metastatic cells. This last effect might induce a less malignant or even benign appearance of a remaining tumour.

If tumours or tumour cells persist in the body the clonal inhibitor should be administrated over long time periods in order to inhibit or stop further cloning or spread of malignant cells. Tumours that still manage to grow during treatment should preferably be eliminated by using improved diagnostic procedures and using conventional measures as surgery, irradiation etc.

### Legends to figures of experiments 1-26:

### Legend experiment 1:

### Fig.1

It was important to find the agar concentration that supported the growth of polyoma virus transformed baby hamster kidney cells, but not the normal parental cell line (BHK21/c13).

In this experiment no significant growth of the normal BHK21/c13 cells in agar was found 5 days after seeding 600000 cells in 0.3 ml soft top agarose layer (Sigma type IX ultra-low gelling temperature) in wells of Falcon 24 wells tissue culture plates. The bottom layer consisted of 0.6 ml of 1.65% of the same agarose. The polyoma virus transformed BHK21/c13, however, showed clonal growth in 0.8% agarose, not in 1.2%.

### Legend, experiment 2:

### Fig.1

1 ml cultures of 400000 polyoma virus transformed BHK21/c13 cells, clone S100T1, were seeded in each Falcon centrifuge tube of 50 ml. The medium contained 1.5% Sigma type IX, ultra-low gelling temperature agarose and RPMI 1640 with 2.8% FBS and 0.22% Mito+. The treated cultures received 4-OH-OPB in final concentrations from 500 to 3000 nM.

After seeding and mixing, the tubes were placed in the refrigerator with 5% CO₂ in air for about 1 hour to consolidate the ultra low melting point agarose.

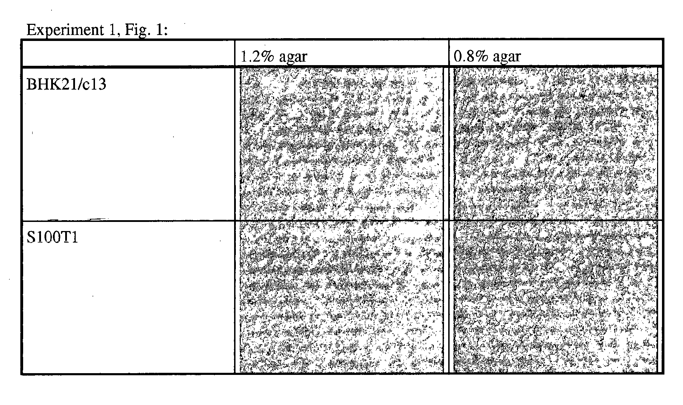

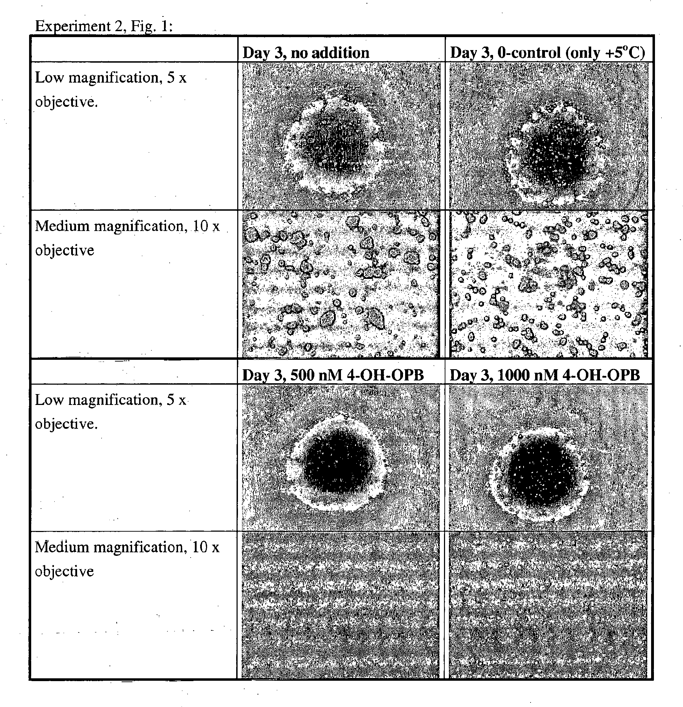

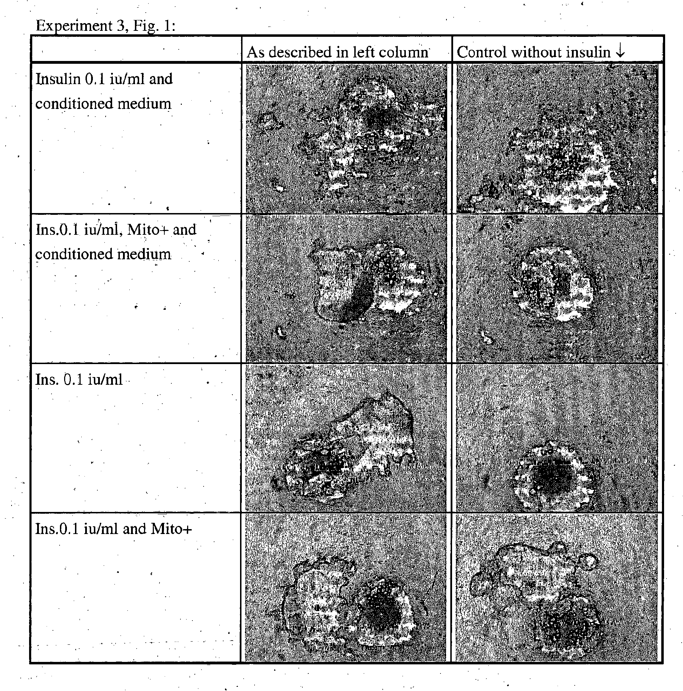

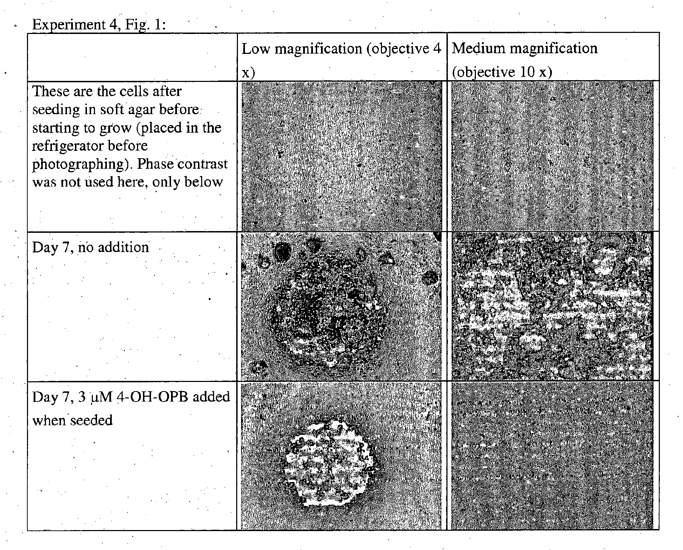

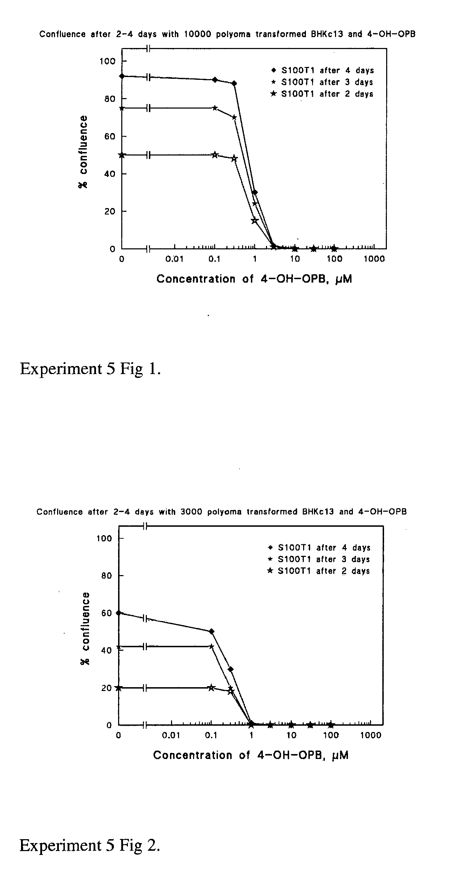

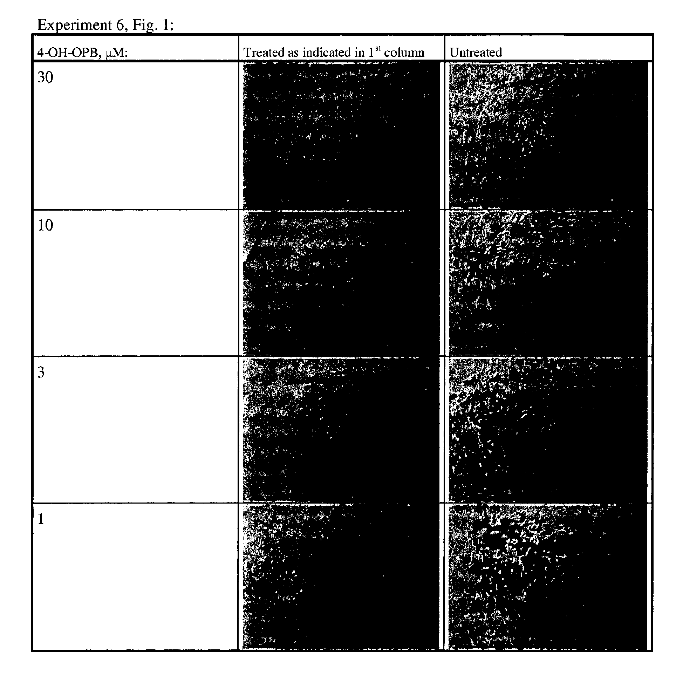

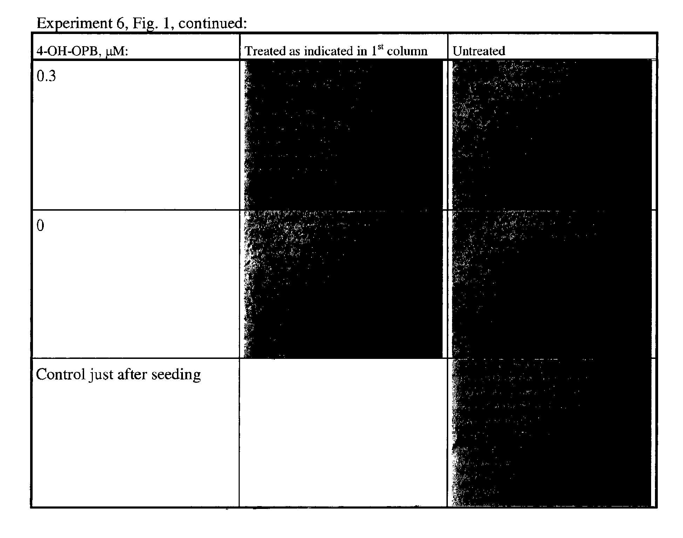

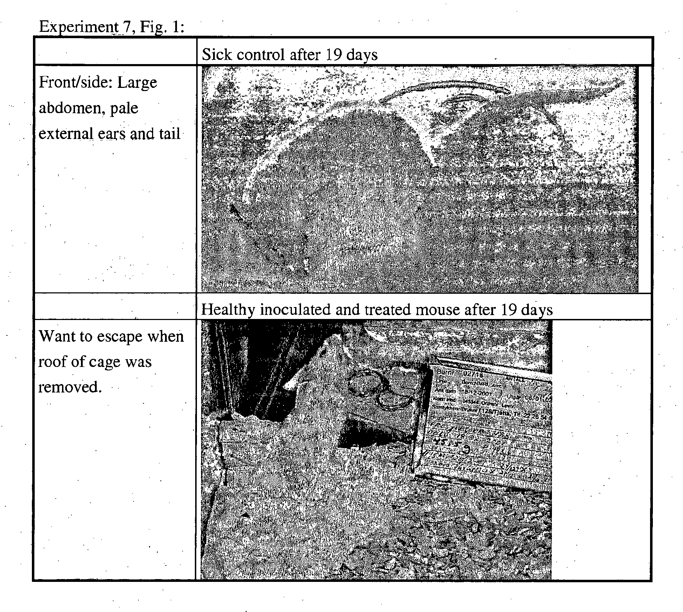

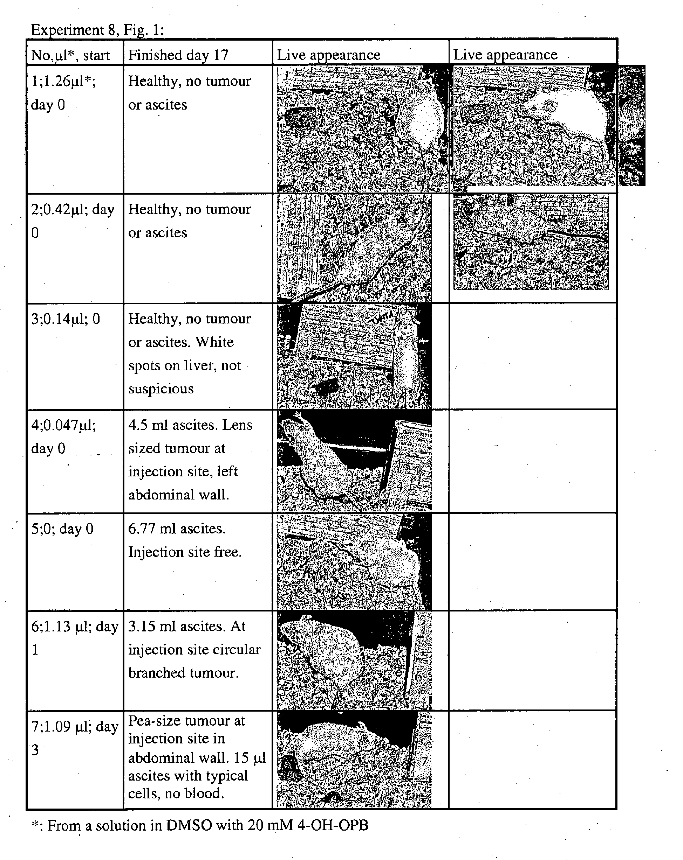

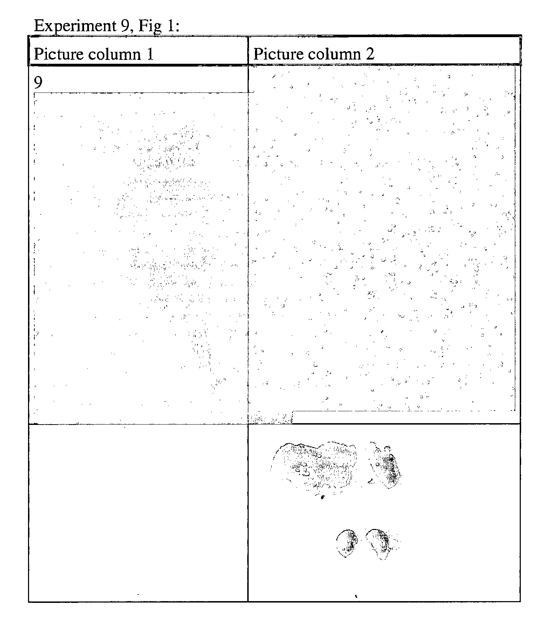

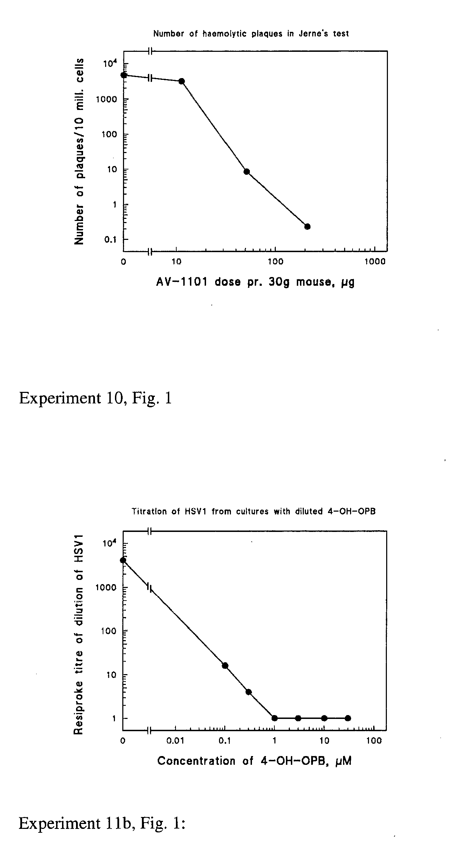

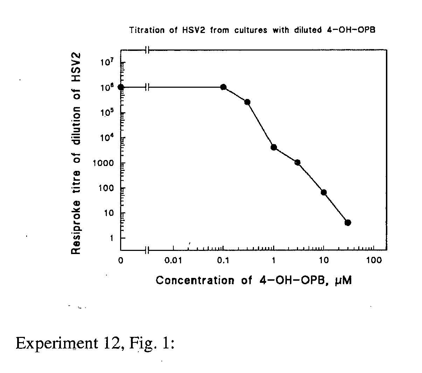

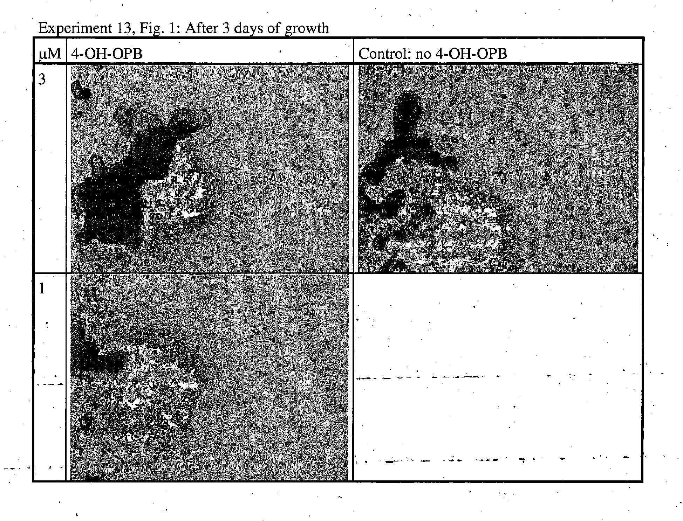

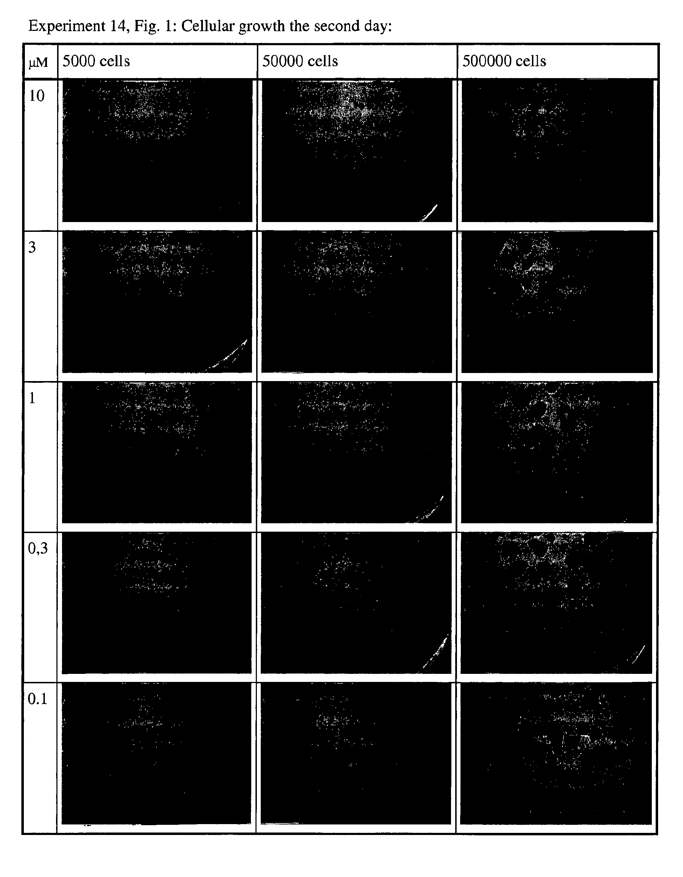

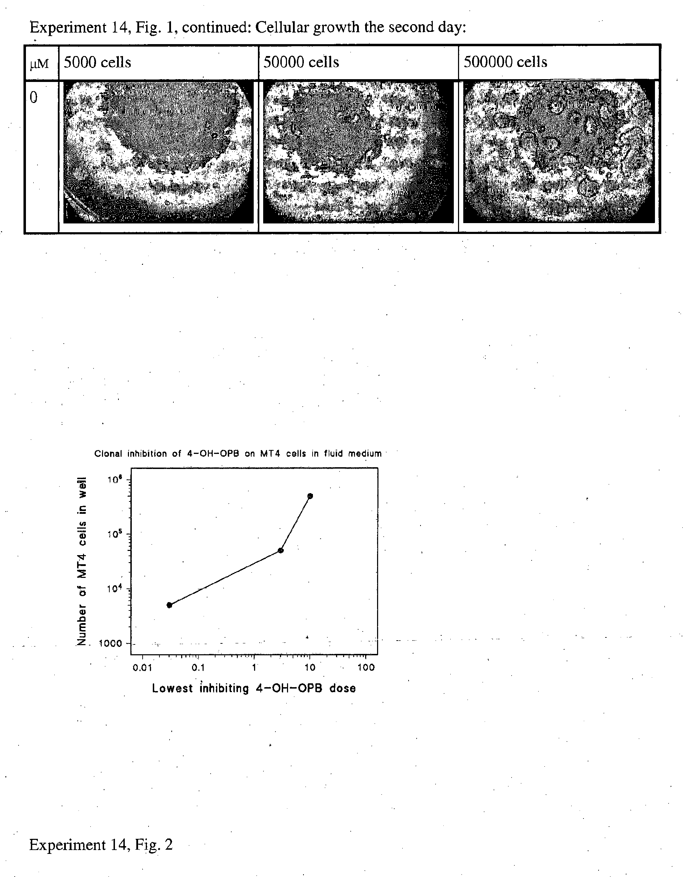

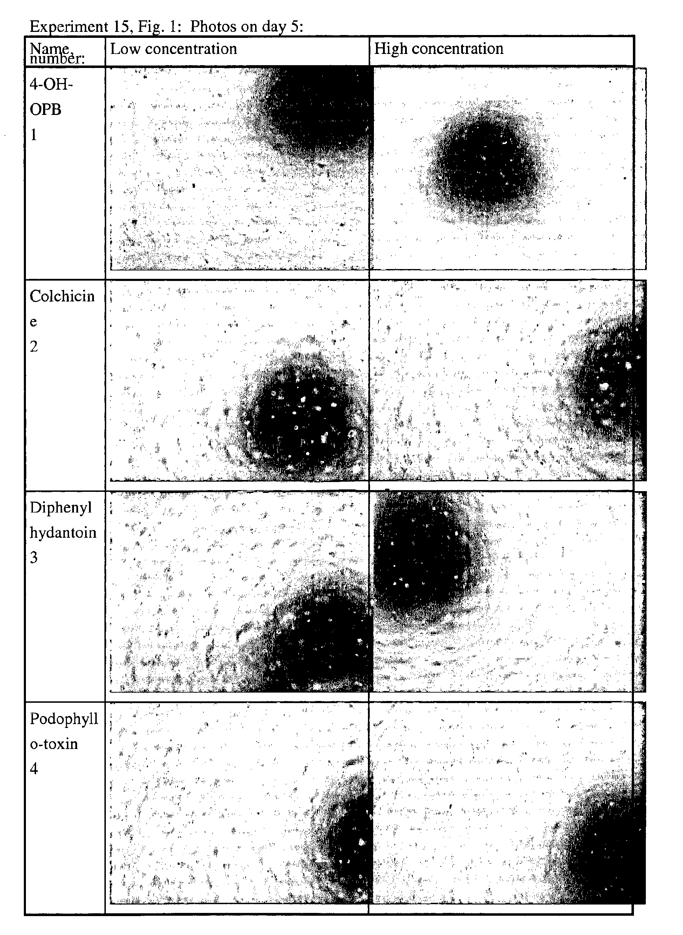

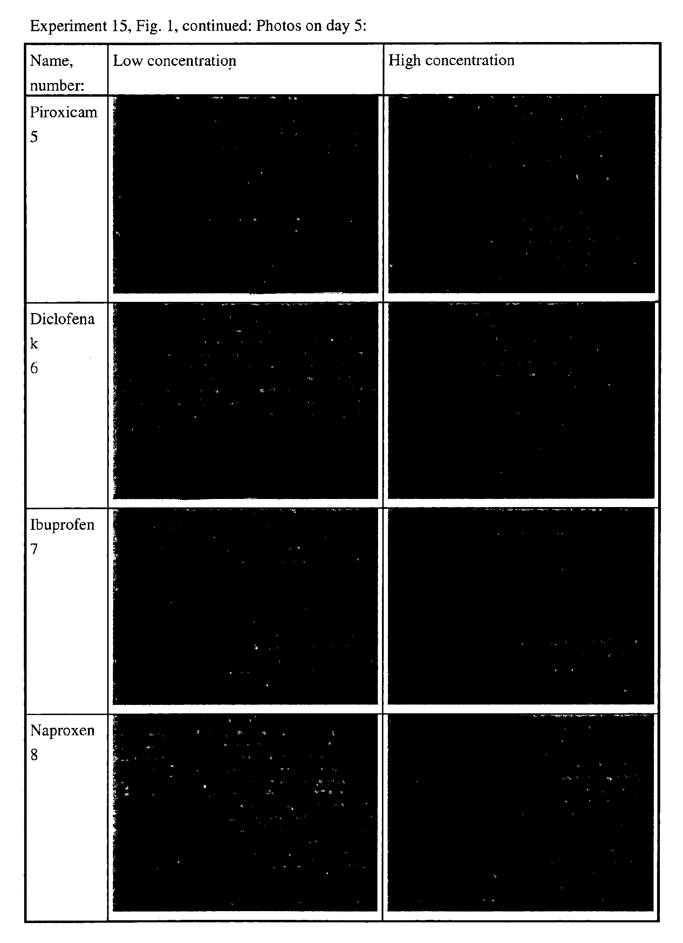

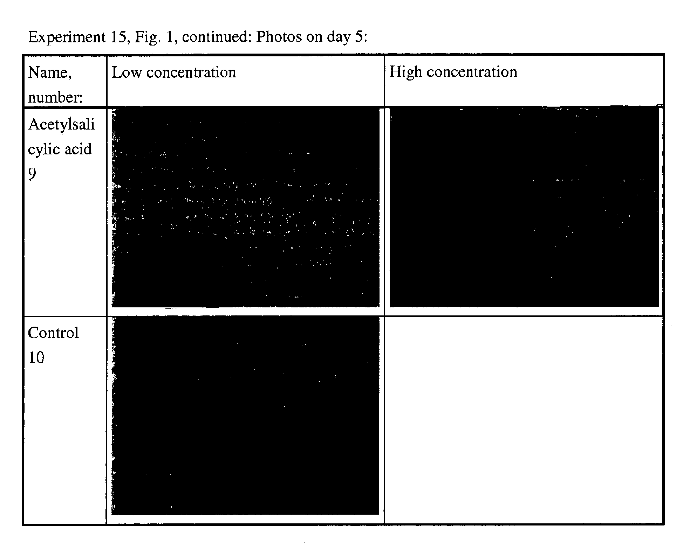

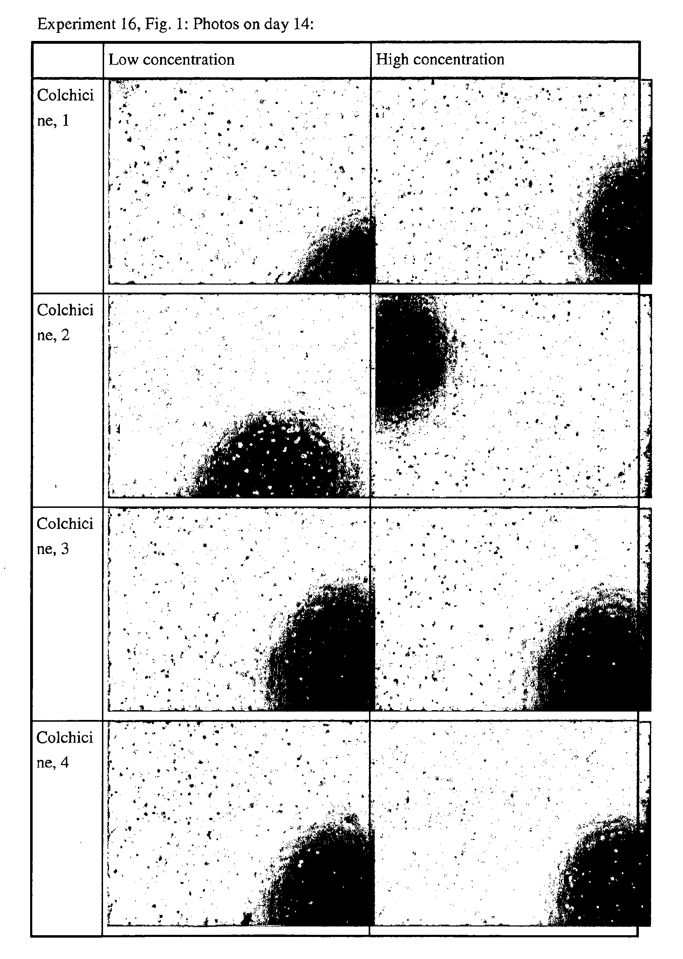

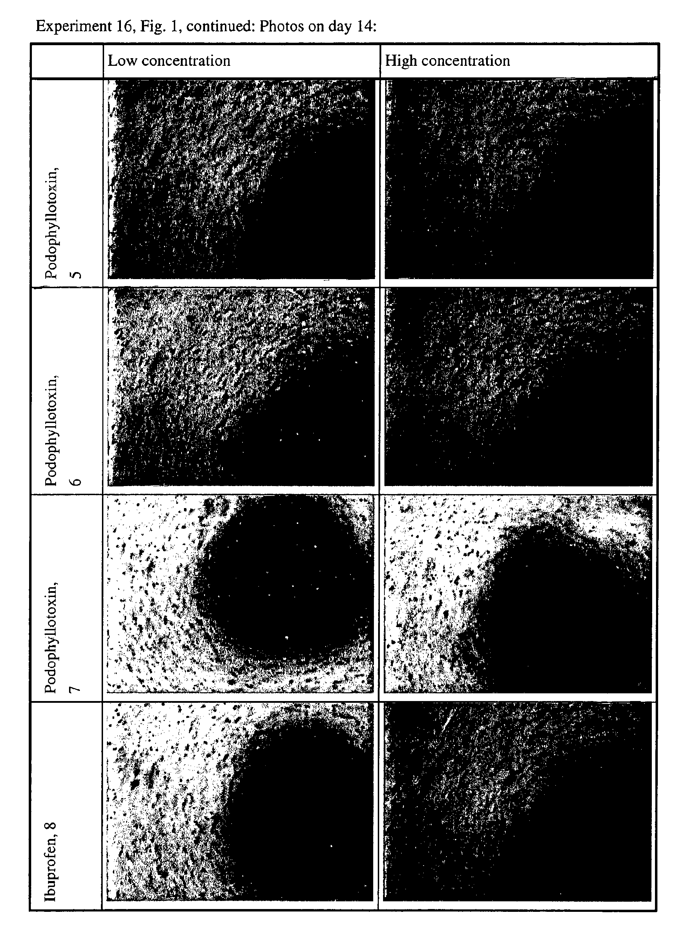

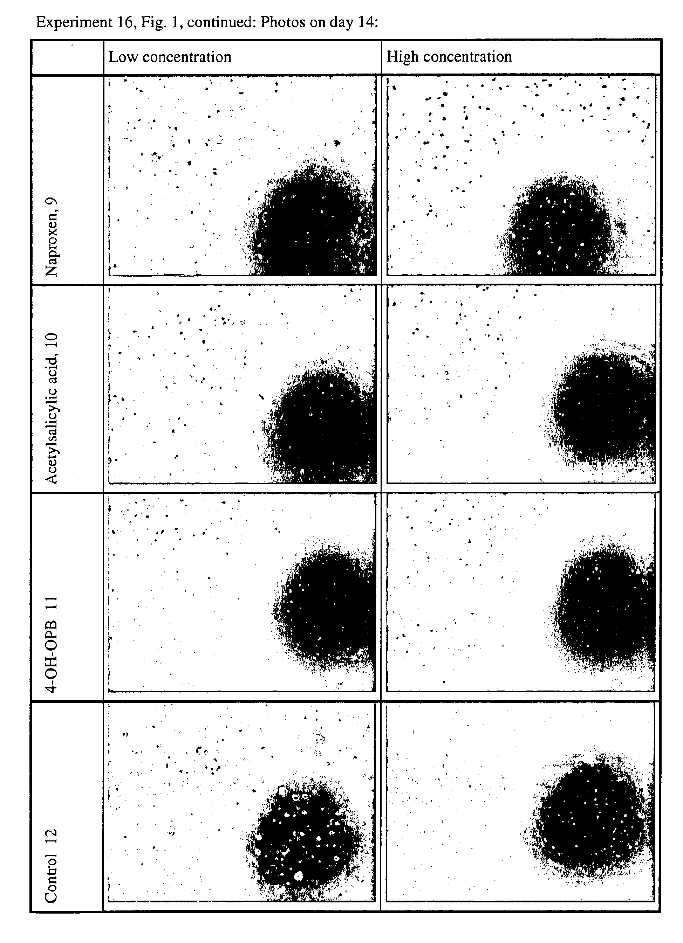

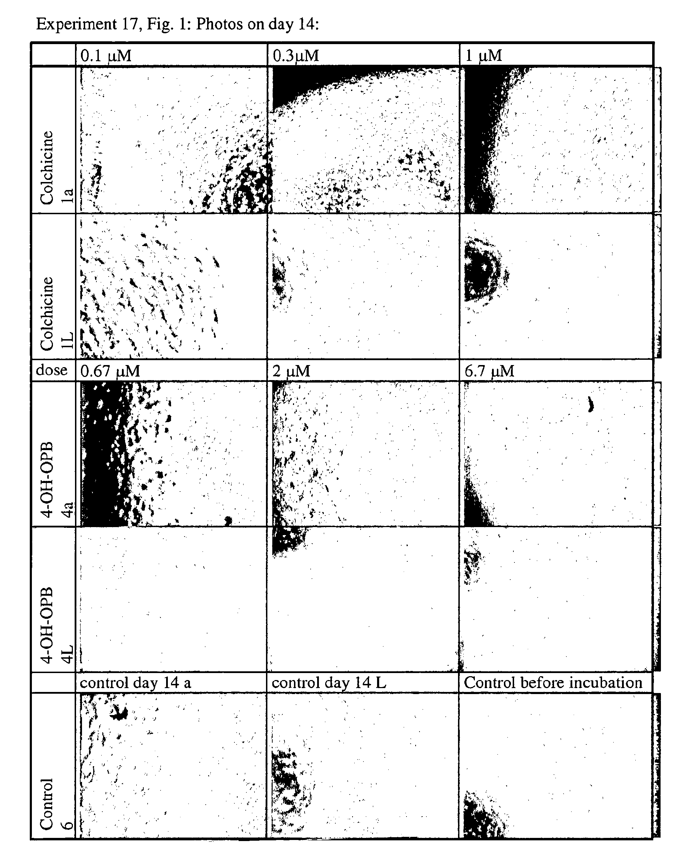

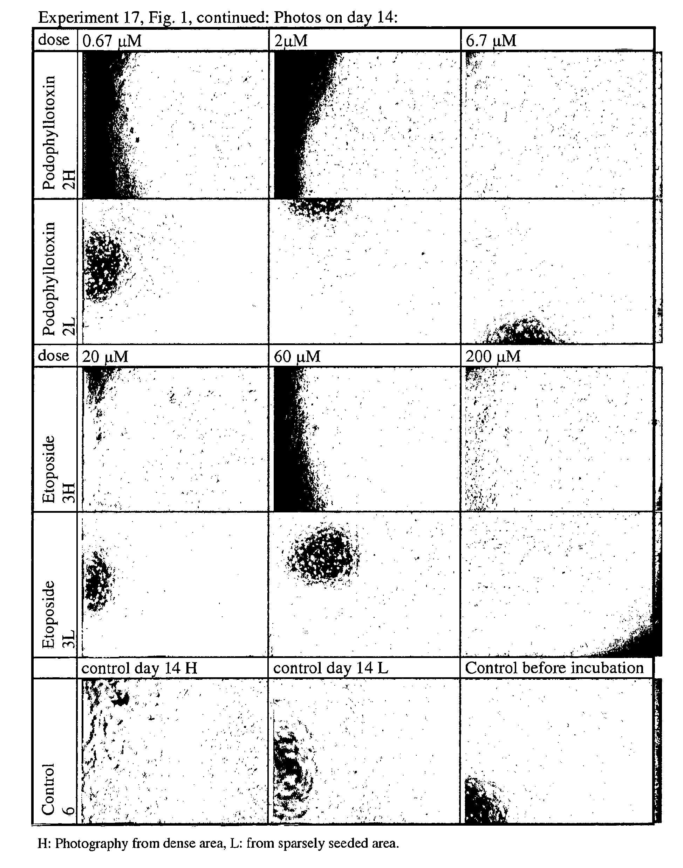

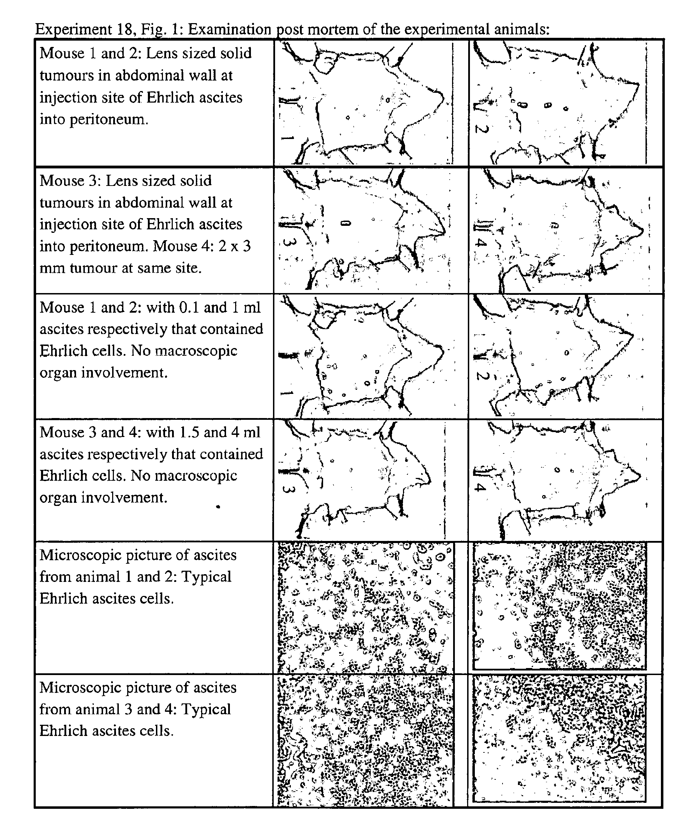

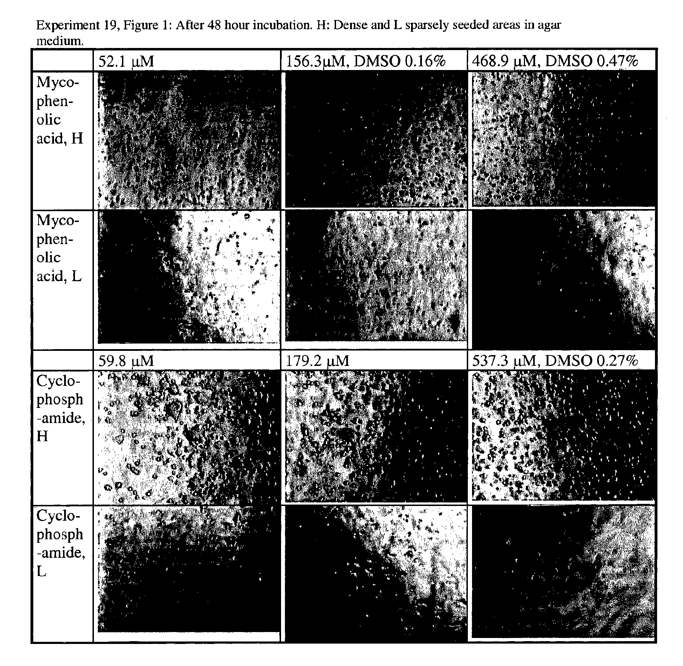

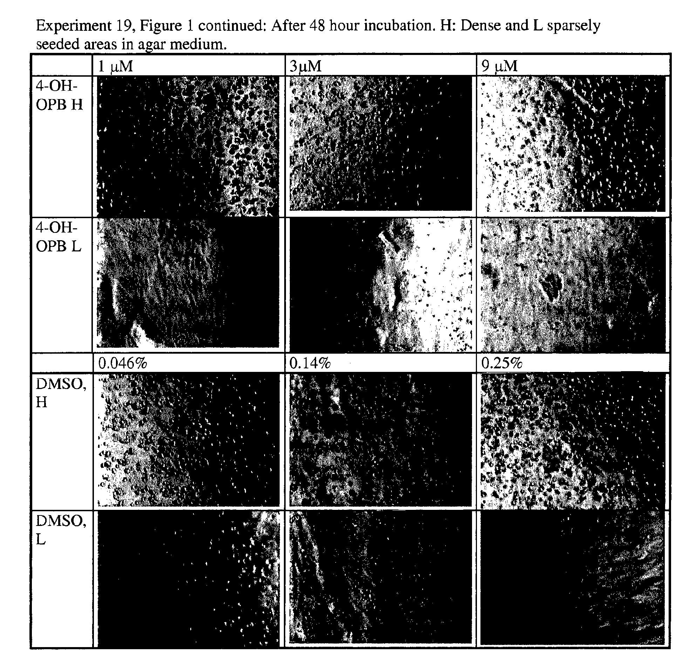

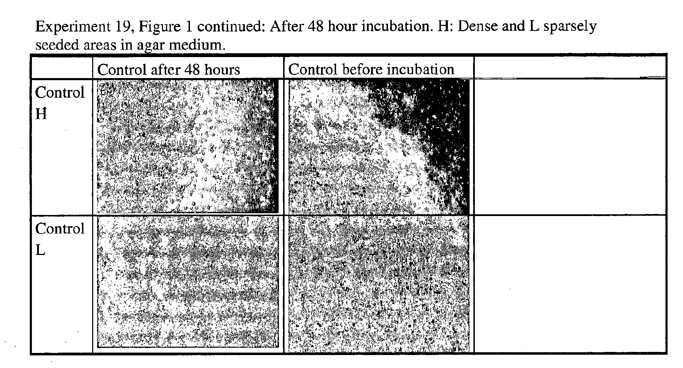

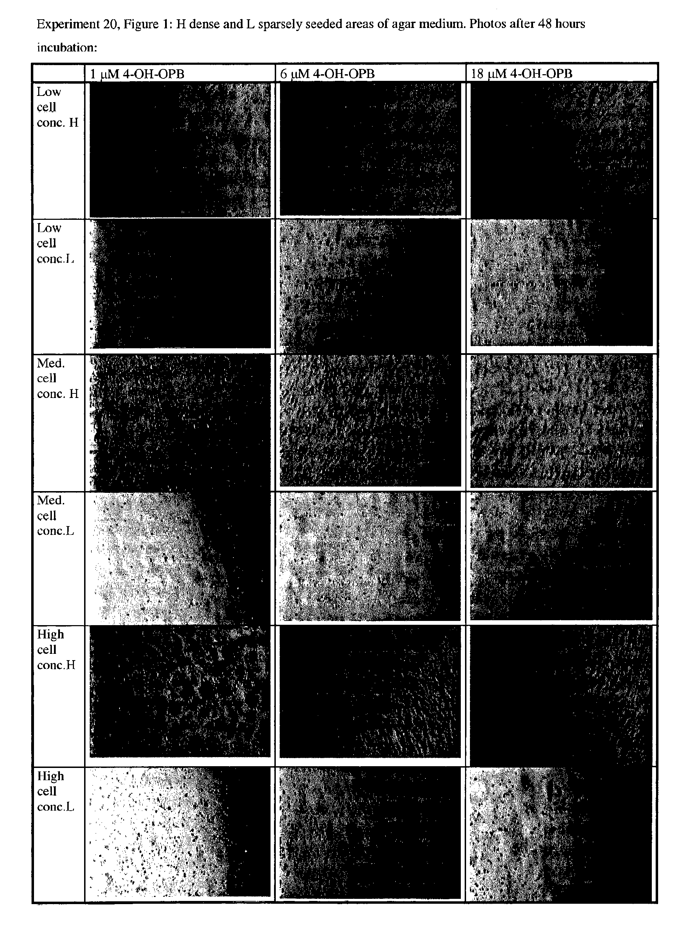

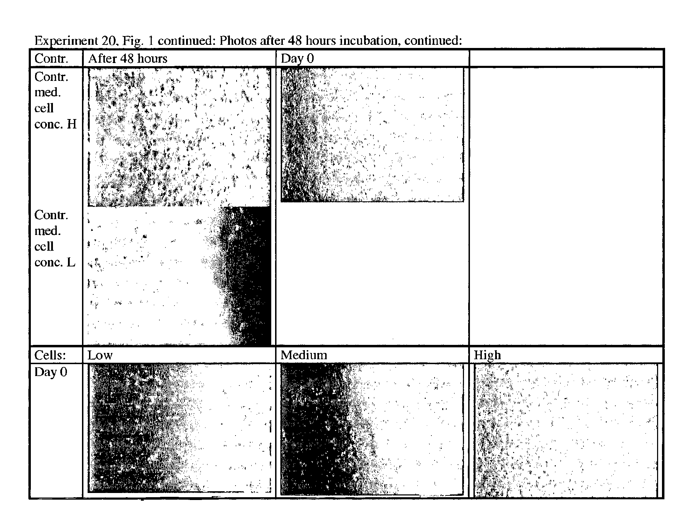

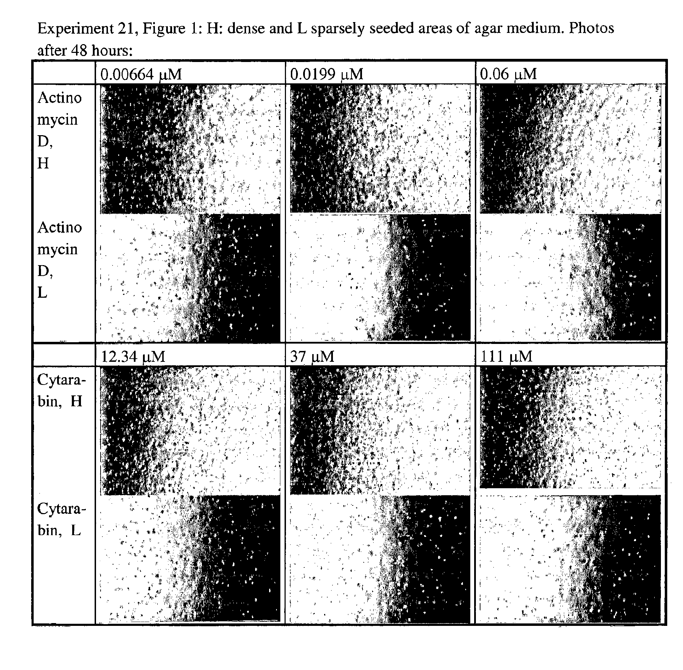

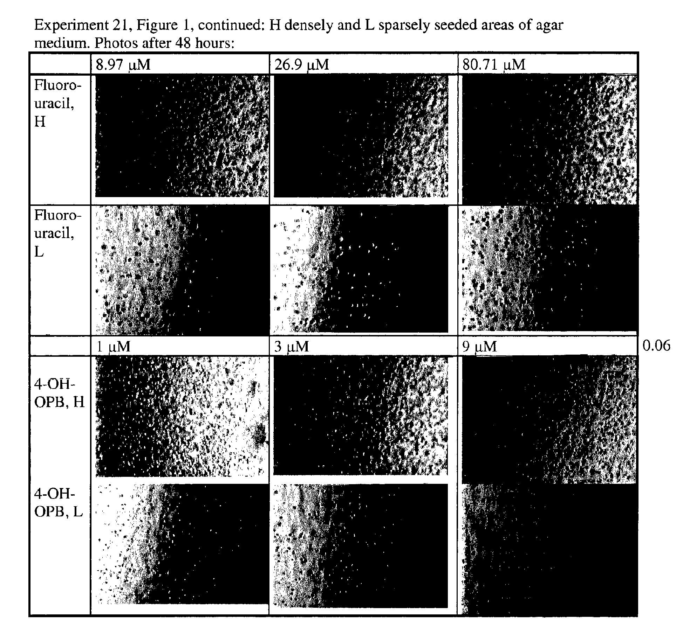

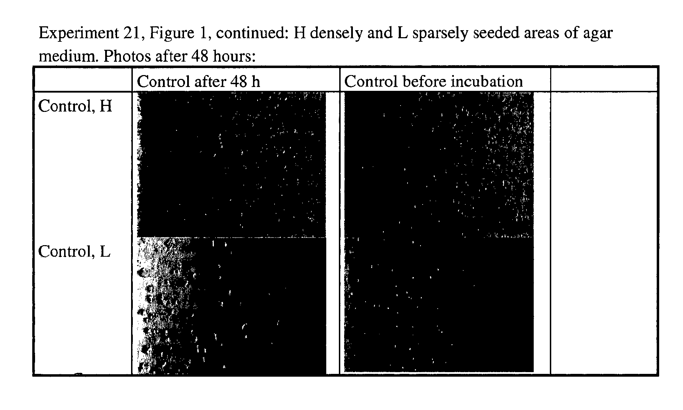

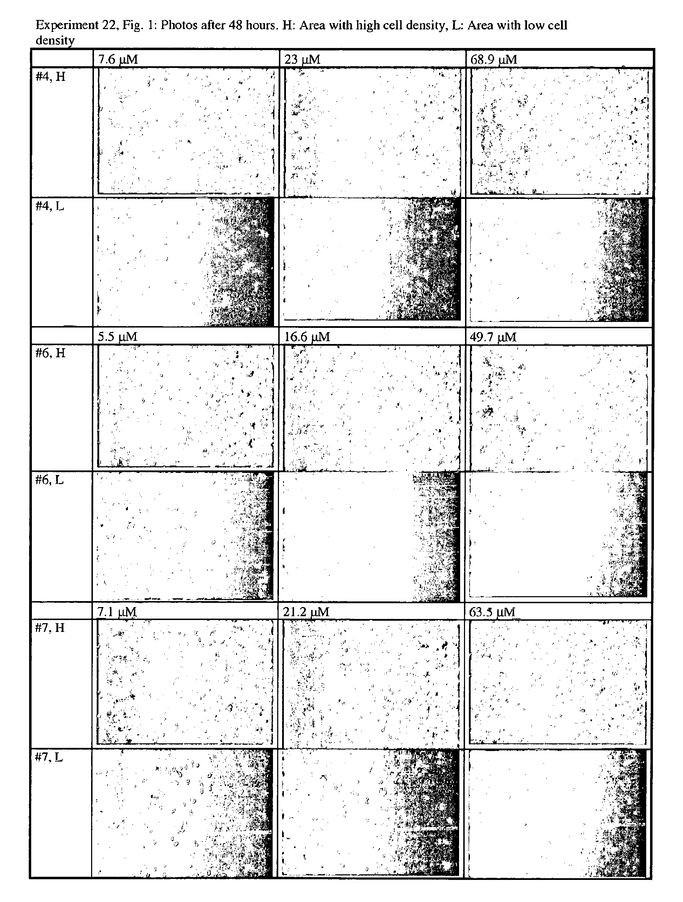

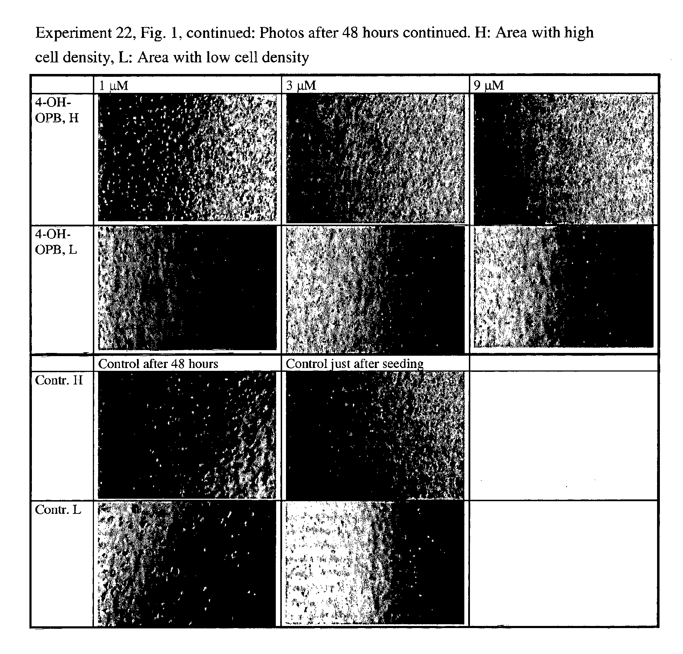

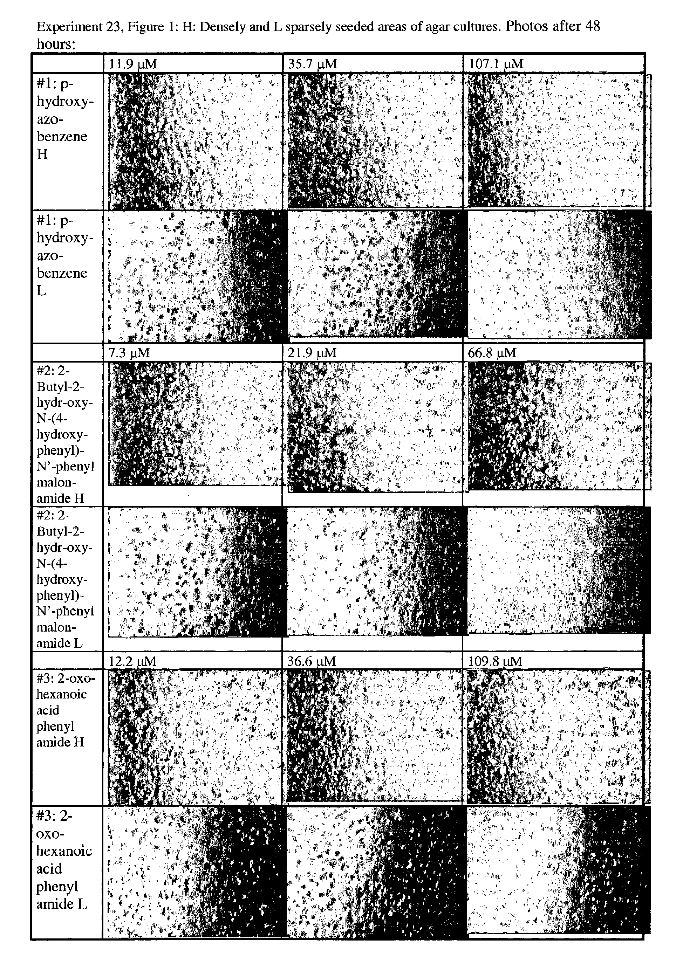

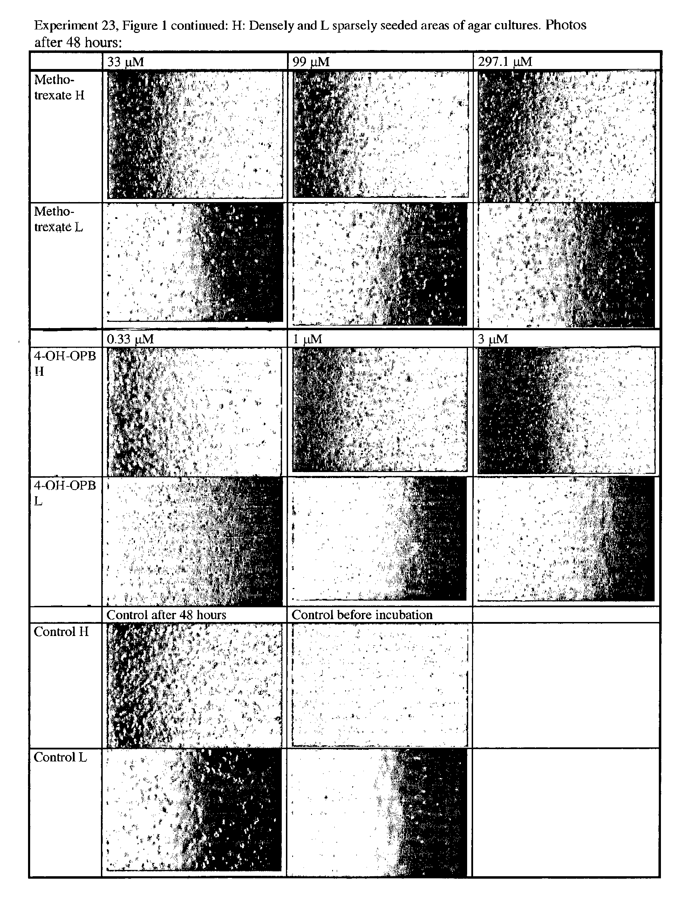

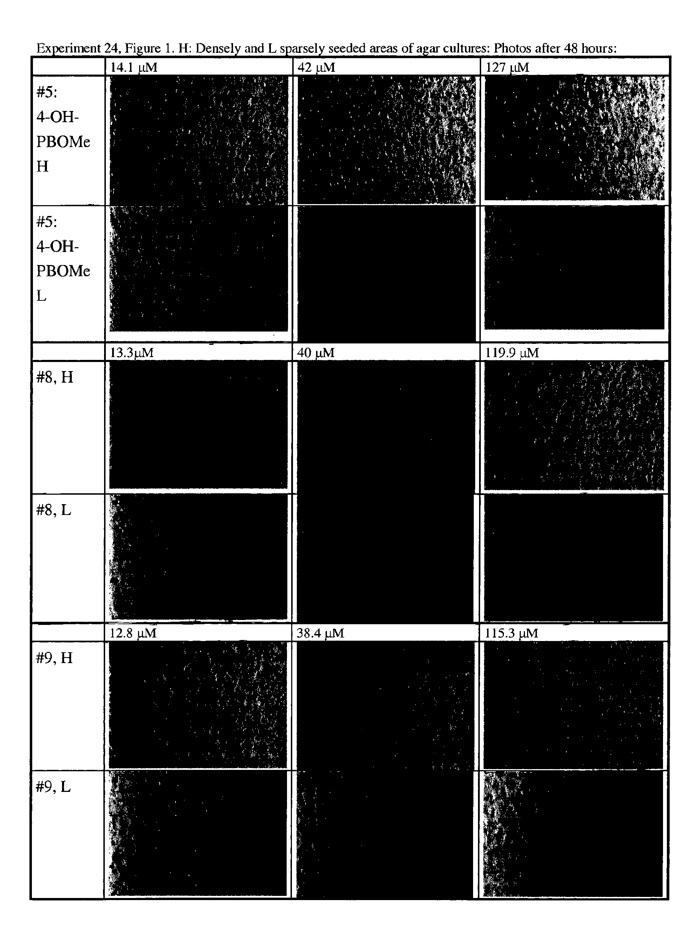

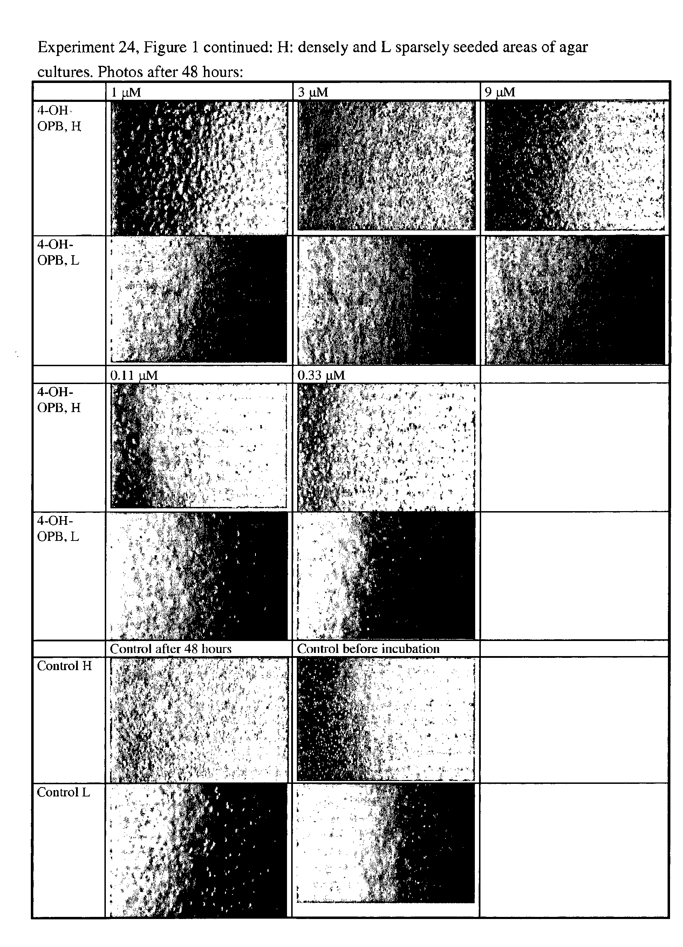

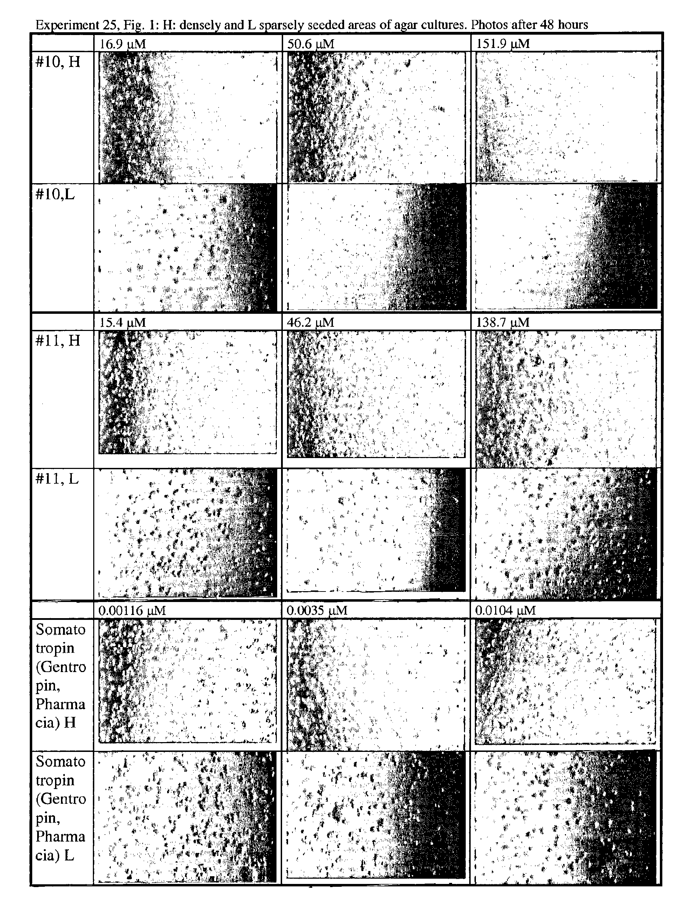

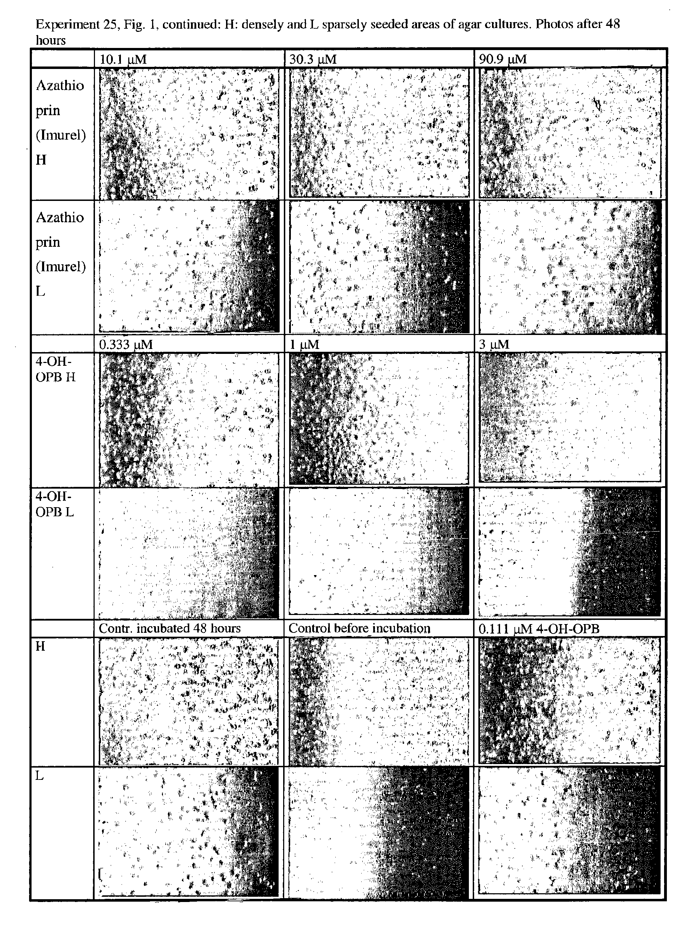

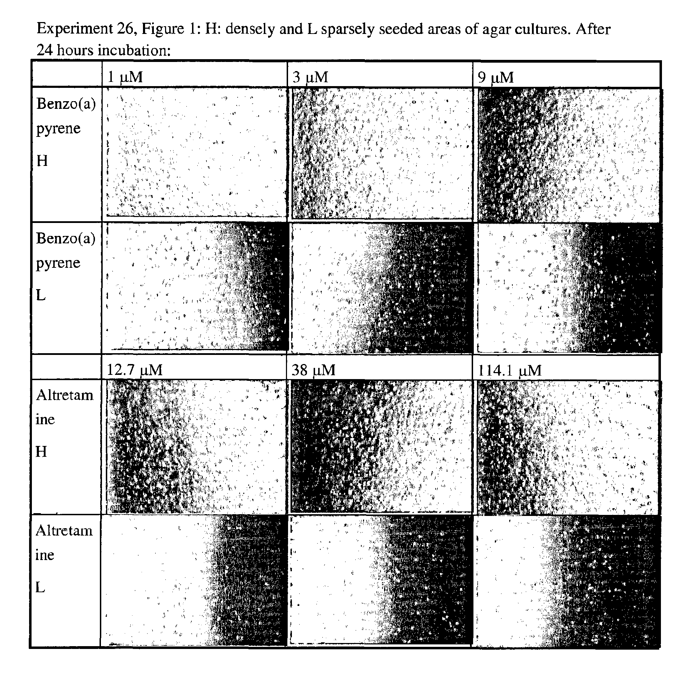

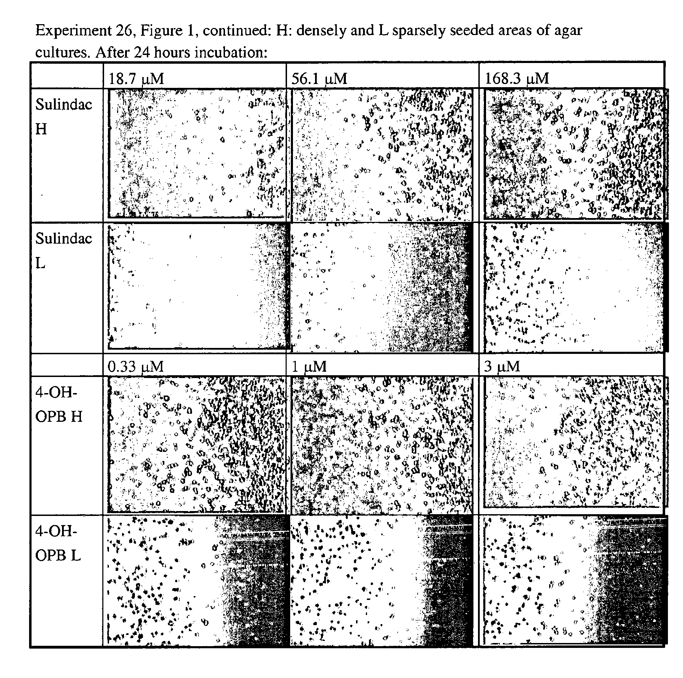

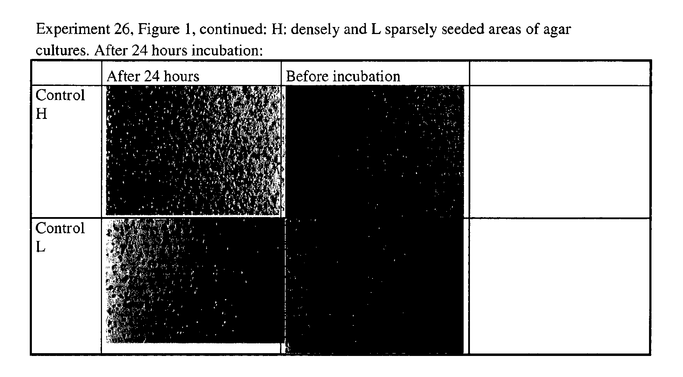

Photos taken after 3 days in the incubator (experiment 2, Fig. 1) showed clearly an inhibition of clonal growth of 4-OH-OPB at a concentration of 500 nM or higher. Other experiments have shown no inhibition with 300 nM. The growth of the untreated control was significant, but small. The reason might be the high agar concentration in this experiment.

### Legend, experiment 3:

### Fig. 1

120000 BHK21/c13 cells were seeded in Falcon plates with 24 wells. Each well had a 0.3 ml bottom and top agar layer ultra low gelling temperature agarose (type IX, Sigma) of 3 and 0.8% respectively in Eagles MEM and RPMI-1640 with 10% foetal bovine serum. Test substances:15% conditioned medium (from BHK21/c13 cultures), the serum extender Mito+ or 0.1 i.u. Insulin Actrapid (Novo)

1, 2 or all 3 of these induced growth. Well with none of these 3, but with serum in Eagles MEM and RPMI showed no growth.

### Legend, experiment 4:

### Fig.1

S100T1, the polyoma virus transformed BHK21/c13 cells, were seeded in 1 ml soft agar medium in Falcon 50 ml centrifuge tubes. This layer contained 1 ml 1.1 % Ultra-low gelling temperature agarose (Sigma, Type IX). On the top of the agar layer 0.5 ml fluid medium was added. It contained conditioned medium from BHK21/C13 cultures, 2.8% foetal calf serum and 0.22% of the serum extender Mito+. Both media contained Eagles MEM, the agar medium also RPMI 1640. Even after 7 days no growth was observed in cultures treated with 3µM 4-OH-OPB. It seemed to cause a perfect clonal inhibition.

### Legends, experiment 5:

### Fig. 1

This figure shows the degree of confluence of polyoma virus transformed BHK21/c13 cells, the S100T1 cell strain, growing on plastic surface that was observed 2-4 days after seeding 10000 cells in appropriate wells of a 96 well tissue culture plate (Nunc). Note that there is about full inhibition of growth when adding 3 µM 4-OH-OPB to the cultures.

### Fig. 2

This figure shows the degree of confluence of polyoma virus transformed BHK21/c13 cells, the S100T1 cell strain, growing on surface that was observed 2-4 days after seeding 3000 cells in appropriate wells of a 96 well tissue culture plate (Nunc). Note that there is about full inhibition of growth when adding 1 µM 4-OH-OPB to the cultures.

### Fig. 3

This figure shows the degree of confluence of BHK21/c13 cells growing on surface. The cultures were observed 2-4 days after seeding 10000 cells in appropriate wells of a 96 well tissue culture plate (Nunc) with 0.1 i.u./ml of Insulin Actrapid (Novo). Note that there is about full inhibition of growth when adding 10 µM 4-OH-OPB to the cultures.

### Fig. 4

This figure shows the degree of confluence of BHKc13 cells growing on surface 2-4 days after seeding 3000 cells in appropriate wells of a 96 well tissue culture plate surface 2-4 days after seeding 3000 cells in appropriate wells of a 96 well tissue culture plate (Nunc). Note that there is about full inhibition of growth when adding 3 µM 4-OH-OPB to the cultures. After 4 days in culture with 100 and 300 nM 4-OH-OPB, these cultures showed a growth inhibition compared to the control.

### Legends, experiment 6:

### Fig.1-4

4-OH-OPB would probably inhibit clonal growth less if added a day after the seeding of cells in agar compared to immediate treatment. There might also be differences between transformed and untransformed cell lines.

Agar cultures were prepared in wells of Falcon 24 well tissue culture plate. Each contained two agar layers of 0.3 ml. The bottom layer contained 1.75% Sigma agarose, type II, A-6877 and the top layer 120000 cells of either BHK21/c13 or its polyoma virus transformed offspring, S100T1, in 0.8% ultra low gelling temperature Sigma agarose type IX, A-5030. The medium contained about equal amounts of Eagle and RPMI 1640 with 10% FBS. Insulin (Actrapid, Novo) was added in a concentration of 0.1 i.u./ml to BHK21/c13 cells only.

If 4-OH-OPB treatment of S100T1 started immediately after seeding, a good clonal inhibition was obtained when the concentration of 4-OH-OPB was above 1 µM. However, a delay of treatment for 24 hours only induced a small growth inhibition when adding 30 µM 4-OH-OPB, a very high dose.

BHK21/c13 showed less inhibition than S100T1 cells when inhibited with the same concentrations of 4-OH-OPB. Photograph after 24 hours showed a small, but significant effect on clonal inhibition from 10 µM or higher when treated immediately on seeding. After 48 hours, photographs of the cultures treated on seeding showed less effect of 10 µM 4-OH-OPB, but the effect still persisted.

However, if treated first after 24 hours, no effect was detected except for transformed cells that showed a small inhibition.

The transformed cell line S100T1 reacted better on clonal inhibition by 4-OH-OPB than the normal parent cell line BHK21/c13.

Cells that had started to form small clones were no longer susceptible.

The only exception of this rule was S100T1 inhibited by 30 µM the day after seeding. At this high concentration of 4-OH-OPB also small colonies of transformed cells showed some inhibition of growth.

### Fig.1

S100T1 cells were given 4-OH-OPB immediately (2^{nd} column) and were compared with cells in the same plate, column 3, that were untreated at that time. Photographs are taken after one day.

### Fig. 2

BHK21/c13 cells were given 4-OH-OPB immediately (2^{nd} column in the table below) and were compared with cells in the same plate, column 3, that were untreated.

### Fig 3

S100T1 cells were given 4-OH-OPB immediately (2^{nd} column) and after 1 day (3^{rd} column). Control without treatment in column 4. Photographs 2 days after seeding:

### Fig. 4

BHK21/c13 cells were given 4-OH-OPB immediately (2^{nd} column) and after 1 day (3^{rd} column). Control without treatment in column 4. Photographs 2 days after seeding:

### Legends, experiment 7:

### Fig.1-3

The malignant Ehrlich mouse ascites tumour was transplanted to two adult male mice (NMRI/Bom). Each of them received 100000 cells intraperitonally. One mouse was treated two times a week with 4-OH-OPB intraperitonally. After 19 days the untreated mouse was moribund (Fig. 1) and both were killed and examined. No tumour was detected in the treated animal. The untreated mouse, however, had about 250 millions of malignant cells in the ascitic fluid and a pea/bean sized solid tumour in the abdominal wall where the needle penetrated during transplantation (Fig. 2). Both subphrenically and connected to the omentum was found fibrinous material with tumour cells and probably infiltration (Fig 3).

### Legends, experiment 8:

### Fig.1

Live appearance of test mice at the end of experiment.

### Fig.2

Mouse #1: No pathology found, neither ascites nor solid tumours. Organs: lungs, heart, liver, spleen, intestines, kidneys, peritoneum, female sexual organs without tumours. Diffuse small light areas were found on liver, not suspicious.
Mouse #2: No pathology found, neither ascites nor solid tumours. Organs: lungs, heart, liver, spleen, intestines, kidneys, peritoneum, female sexual organs without tumours.
Mouse #3: No pathology was found, neither ascites nor solid tumours. Organs: lungs, heart, liver, spleen, intestines, kidneys, peritoneum, female sexual organs showed no tumours. Diffuse light areas observed on liver were somewhat larger than on no. 1, not suspicious

### Fig.3

Mouse #4: Needle is pointing at white tumour at injection site in the abdominal wall. Adherent skin is removed. 4.5 ml ascites with blood. Lens-sized tumour at injection site in left abdominal wall. No affection of organs was observed, but multiple small mm-sized tumours were found retroperitoneally, around tubes and stomach and under diaphragm. Liver and other organs were anaemic.
Mouse #5: Abdomen with ascites after removal of skin. No solid tumour observed in abdominal wall. 6.77 ml ascites was found. Injection site free. No affection of organs, but multiple small mm-sized tumours retroperitonally, around tubes and stomach and under diaphragm. Liver and other organs were anaemic.
Mouse #6: Needle is pointing at tumour in left abdominal wall. 3.15 ml ascites was found. At injection site circular branched tumour. Small, but not so numerous tumours as described for no. 4 and 5 found retroperitonally, in omentum or subphrenically. Spleen was somewhat larger than in the other animals, but the enlargement was diffuse without suspicious tumour masses.
Mouse #7: Needles were pointing at tumour in abdominal wall that was visible after dissection. It was pea-sized and localised at the injection site in the abdominal wall. Only 15 µl acites were found, but it contained typical cells and no blood.

### Legends, experiment 9:

### Fig 1

In column 1 there is a picture of Ehrlich cells from peritoneum of the control mouse #2. In column 2, the ascitic fluid from mouse #1 is under the microscope with the finding of none Ehrlich cells. At the bottom the organs liver, spleen and kidneys are shown. They were adherent and small with signs of toxic affection.

### Fig 2

Mouse #1: The very high dose of 4-OH-OPB caused adherences in peritoneum such as: spleen to stomach, liver to retroperitoneum and diaphragm. The kidneys and liver were small. Ascites or thoracic fluid did not contain Ehrlich cells. The thoracic effusion probably killed the animal.

The two tumours in abdominal wall are clearly seen. The one in lower abdomen coming from the highest number of injected cells (31250) is bigger than the one above coming from 5 times less number of Ehrlich cells (6250). No tumour was found elsewhere in the mouse.

Mouse #2, the control: This was the untreated control, the only one that developed ascites containing typical Ehrlich cells. No solid tumour was found in abdominal wall or elsewhere.

Mouse #3: Tumour in abdominal wall could not be observed externally. Ascites or organ tumours were not found in this mouse.

Mouse #4: This one, however, had slightly observable tumour in the abdominal wall. There was found a fresh haemorrhage in thorax without signs of malignancy after accidental
fracturing thoracic column when killing the animal. No ascites was observed.

### Fig. 3

The figure shows the smallest number of subcutaneously injected Ehrlich cancer cells needed for growth when 4-OH-OPB was injected intraperitonally in different doses to mice. No 4-OH-OPB injected gave no tumour subcutaneously, only ascites. The treated animals did not get ascites with tumour cells, only solid tumours

### Legend for experiment 10:

### Fig.1

This figure shows the number ofhaemolytic plaques obtained from the spleens of mice being immunised 5 days before with sheep red cells. The animals were treated with 4-OH-OPB simultaneously with the injection of red cells. Each animal had been treated with the same dose 4-OH-OPB twice a week for one month until 26 days before the immunisation.

The reason for this treatment the first four weeks was to see if it would influence the growth of transplanted Ehrlich ascites cells. However, these cells failed to grow.

The second biomarker, the sheep red cells, were injected at the same time as 4-OH-OPB (same dose as before).

### Legend, experiment 11b:

### Fig.1

The Herpes titration was read after incubation in four days with different concentrations of 4-OH-OPB as indicated. Maximal inhibition is obtained with 4-OH-OPB doses of 1 µM or more. Total titre reduction is about 3.5 log.

### Legend, experiment 12:

### Fig.1

The figure shows a titration in RK13 cells of HSV2 that was produced by micro well cultures inoculated with 2 µl HSV 2 (Table 1) and treated with 4-OH-OPB two days before the titration. The inoculate was removed the day before titration and the medium was changed. The titration was read after 3 and 4 days (Table 2 and Fig. 1) and was showing a logarithmic titre loss parallel to logarithmic increase of the concentration of 4-OH-OPB that was added to the cultures. Total inhibition was about 5.5 log.

### Legends, experiment 13:

### Fig.1-4

Cells from S100T1, a polyoma virus transformed BHK21/c13 cell line, were seeded in soft agar medium to study clonal inhibition of 4-OH-OPB and Colchicine. Both sparsely and densely seeded areas of the same culture (in 24 well Falcon plates) were studied.

Both compounds inhibited clonal growth in sparsely seeded areas, but not in densely seeded areas of the same culture.

The agar concentration of bottom layer was 1.75% and of top layer with cells 0.8% of the special ultra low gelling temperature agarose (type IX, A5030.

Colchicine and 4-OH-OPB did not inhibit clonal growth in locations with high cell density. But in the same culture was demonstrated full inhibition on clonal growth of sparsely seeded cells. This is probably a good illustration in cell culture of what has been shown for the export of metastatic cells from a region rich in tumour cells in mice. At a certain distance from the main cell mass, single cells were not able to grow when under the influence of a clonal inhibitor like 4-OH-OPB (or Colchicine).

### Legends, experiment 14:

### Fig.1

The lowest dose of 4-OH-OPB that inhibits the growth of clones of MT4 cells in a cell culture with fluid medium was found. The concentration of 4-OH-OPB needed for arresting colony growth was about 2 logs less in MT4 cultures with 5000 cells than in cultures with 500000 cells. The interpretation of this is probably not that each cell needed a certain amount 4-OH-OPB to be bound to postulated receptors of the cell for full clonal inhibition, since in agar cultures seeded with cells in a concentration gradient, the colony inhibition was still different and strongest in sparsely seeded areas.

### Fig. 2

This figure shows the lowest inhibiting dose of 4-OH-OPB that inhibits the growth of MT4 cells in a cell culture with fluid medium. The concentration of 4-OH-OPB needed for arresting colony growth was about 2 logs less in cultures with 5000 cells than in cultures with 500000 cells. The interpretation of this is probably not that each cell needed a certain amount 4-OH-OPB to be bound to postulated receptors of the cell for full clonal inhibition, since in agar cultures seeded with cells in a concentration gradient, the colony inhibition was still different and strongest in sparsely seeded areas.

### Legend, experiment 15:

### Fig. 1-2

Agar cultures were prepared in wells of Falcon 24 well tissue culture plate. Each contained two agar layers of 0.3 ml. The bottom layer contained 1.75% Sigma agarose, type II, A-6877 lot 49H0380 and the top layer 32000 cells in 0.8% ultra low gelling temperature Sigma agarose type IX-A, A-2576 lot 19H0821. The medium contained both Eagle and RPMI 1640 with 10% FBS.

4-OH-OPB was inhibiting clonal growth at the concentrations tested, 2 and 20 µM. In the future lower concentrations should also be tested.

Colchicine did not inhibit clonal growth at concentrations from 0,01-0,1 µM.

Diphenhydantoin did not inhibit clonal growth significantly at concentrations 10-100 µM.

Podophyllotoxin inhibited significantly at 0.3 µM, but there were still some few clones. There was no significant inhibition at 0.03 µM.

Piroxicam did not inhibit clonal growth significantly at concentrations 5-50 µM

Diclofenac showed no significant inhibition at 10-100 µM. However, a probable significant increase in clonal growth was observed at 10 µM.

Ibuprofen showed insignificant inhibition at 100 µM. The inhibition at 1000 µM might not be real since it involved the addition of 30 µl of DMSO to the well. That might be toxic (see next experiment).

Naproxen inhibited significantly at 500 µM, but DMSO toxicity might interfere as mentioned for Ibuprofen.

Acetylsalicylic acid possibly inhibited significantly, but not completely at 100 µM. At 1000 µM the inhibition is complete, but might partially be caused by DMSO as mentioned for Ibuprofen.

### Legend experiment 16:

### Fig.1

Substances with anti-clonal activity are searched for among NSAID's, mitotic inhibitors and common painkillers.

Agar cultures of S100T1 cells, a polyoma virus transformed BHK21/c13 cell line, were prepared in wells of Falcon 24 well tissue culture plate. The wells contained two layers, each of 0.3 ml agar. The medium contained both Eagle and RPMI 1640 with 10% FBS.

Ultra-low gelling temperature agarose (Sigma A-2576, Lot19H0821) of 0.8% was incorporated used in the top layer and the Sigma agarose, type II, A-6877, Lot 49H0380 of 1.75% in the bottom layer.

Before the addition of 4-OH-OPB or the other compounds, the plates were put into the refrigerator for 1 hour for consolidating the top agar layer.

The control did not receive anything unless indicated. The numbers in the left column refers to numbers in table above. In the same table high or low concentrations are indicated.

The results showed that Naproxen and Acetylsalicylic acid probably had a colony inhibiting activity for dosage within a range that might be therapeutic. For Ibuprofen, Colchicine and Podophyllotoxin the same activity possibly existed. But the cell number in each well was low and made it difficult to estimate the possibility of toxicity on cells in densely seeded areas or on the organism.

The control 4-OH-OPB was clearly better than any other.

### Legend experiment 17:

### Fig.1

In the two previous experiments about 40000 cells were seeded in each well of 0.6 ml. In these experiments the initial growth was retarded more than expected compared to other experiments. Therefore, the cell number in the well was increased in this experiment to 144000. This cell number was expected to be more optimal.

The aim of the experiment was to see if the addition of compounds other than 4-OH-OPB inhibited clonal growth of S100T1cells, a polyoma virus transformed cell line derived from BHK21/c13.

Agar cultures of these cells were prepared in Falcon 24 well tissue culture plate. Each well contained two agar layers of 0.3 ml. In the bottom layer 1.75% Sigma agarose, type II, A-6877, Lot 49H0380 was added and in the top layer 120000 cells in medium with 0.8% ultra low gelling temperature agarose type IX-A, Sigma A-2576, Lot19H0821.

The tested growth inhibitors consisted of two groups, the inhibitors that inhibited cell growth in both high and low cell density locations, the Podophyllotoxin group, and the inhibitors that only inhibited clonal growth in low density areas, 4-OH-OPB and Colchicine. Especially for 4-OH-OPB of medium concentration there was good growth of colonies in high-density areas, but no growth in low-density areas.

Colchicine did not inhibit the high-density areas significantly. Only high dose seemed to inhibit the growth of cells in low-density areas, but no complete clonal inhibition could be observed.

Both compounds in the Podophyllotoxin group, Podophyllotoxin and Etoposide, behaved similarly with growth inhibition of both high and low-density areas. But complete inhibition of clonal growth in low-density areas was not observed. Therefore, effective control of metastases would not be expected when treating animal or man.

These findings indicated that Podophyllotoxin or Etoposide might be toxic also to cells in densely populated areas and in the body as is already known.

Testing DMSO resulted in approximately unaffected growth inhibition up to about 1% concentration in soft agar cultures. 4.7% inhibited formation of clones, but not completely.

### Legend, Experiment 18:

### Fig.1

*We wanted to know if suboptimal 4-OH-OPB treatment combined with Etoposide, a podophyllotoxin derivative, in relative high dose would rescue mice transplanted with Ehrlich carcinoma. Also a fairly high Colchicine dose was given to see if it would rescue a transplanted animal. The control animal received a suboptimal 4-OH-OPB treatment. See Table 1, Experiment 18 for details of the treatment. None of the animals, were rescued. The result is in concordance with the results in cell culture that indicated no complete clonal inhibition by Etoposide or Colchicine, see Table 2, Experiment 18, the calendar of treatment and Table 3, Experiment 18, summary of results.*

### Legend experiment 19:

### Fig. 1:

The indicated compounds were added to soft agar cultures of the polyoma virus transformed cell line S100T1 of BHK21/c13 cells. Agar cultures were prepared in wells of a Falcon 24 well tissue culture plate. The wells contained two agar layers of 0.3 ml. The bottom layer contained 2.16% Sigma agarose, type II, A-6877 and the top layer 120000 cells in 0.8% ultra low gelling temperature Sigma agarose type IX, A-5030. The medium contained Eagles MEM and 10% foetal bovine serum (FBS).

The compounds were first solved in DMSO in 20 mM concentration and then diluted in medium. The top layer needed 60-90 minutes in the refrigerator for solidification before incubation at 37 °C in 5% CO₂ atmosphere.

The cell gradient was made by adding all the cells in the last 75 µl of the 300 µl top layer in the periphery of the well.

The resulting growth was photographed using an inverted Olympus microscope CK 40, 4 x objective and Olympus Camedia digital camera C-3040 zoom, see Fig. 1.

### Legend experiment 20

### Fig. 1:

Different cell concentrations will influence the effect of clonal inhibitors. What are the limits?

4-OH-OPB was added in different concentrations to the bottom layer of soft agar cultures of the polyoma virus transformed cell line S100T1 of BHK21/c13 cells that were seeded in 3 different concentrations of 25000, 75000 and 225000 cells/well (low, medium and high cell number). The wells contained two agar layers of 0.3 ml each. Both contained 0.8% ultra low gelling temperature Sigma agarose type IX-A 2576, lot 19H082 with Eagles MEM and 10% foetal bovine serum (FBS).

First 4-OH-OPB was solved in DMSO in a concentration of 20 mM and then diluted in medium and added to the bottom layer before its solidification. Both layers needed 60. 90 minutes in the refrigerator for solidification before incubation at 37 °C in 5% CO₂ atmosphere.

One side of the Falcon 24 well plate was elevated in an oblique position of 12 degrees when solidifying the bottom agar layer in the refrigerator for 60-90 minutes. Next, another 60-90 minutes were spent in the refrigerator to consolidate the soft agar top layer with cells in a horizontal position.

The resulting growth was photographed using an inverted Olympus microscope CK 40, 4 x objective and Olympus Camedia digital camera C-3040 zoom, see Fig.1.

In this experiment of cross-titration of cell number and dose of 4-OH-OPB, there was found a very strong inhibition of growth in low density areas of high and medium concentration of S100T1 cells (375000 or 125000 cells/ml) with a small dose of 4-OH-OPB. However, the smallest number of seeded cells, 42000 cells/ml, did not grow even in dense areas of the cell gradient if treated. Only the cell size might have increased a little.

The control with average cell number and no inhibitor grew well even in sparsely seeded areas and represented the basis for the conclusion that even 1 µM 4-OH-OPB had a significant effect even on wells with large cell numbers, but only in the zone with few cells.

Experiments intended to screen for anti growth effects need to have cell numbers above 42000, probably between 125000 and 375000 cells/ml on order to getting fast results.

In this experiment the conclusion that an anti clonal effect of sparsely seeded cells is present at a concentration of 4-OH-OPB of 1-18 µM seems valid. At the level of 1-6 no significant growth inhibition on denser populated areas of the agar was observed.

### Legend experiment 21:

### Fig. 1:

The polyoma virus transformed BHK21c13 cells, the line S100T1, was seeded in soft agar and treated with cytostatic drugs to see if some inhibited sparsely seeded cells completely (Table 1).

Non of them did, but Actinomycin showed up with results that were similar to 4-OH-OPB.

There was good, but probably no complete inhibition of clonal growth at low toxicity.

**Cytarabine showed incomplete inhibition of clonal growth and significant toxicity.**

The results with Fluorouracil showed relatively moderate inhibition of clonal growth and low toxicity independent of dosage.

### Legend experiment 22

### Fig. 1:

### Legend for Figures number:

These compounds were tested: Number 4, 6 and 7.

The indicated compounds were added to soft agar cultures of the polyoma virus transformed cell line S100T1 of BHK21/c13 cells that were prepared in wells of a Falcon 24 well tissue culture plate. The wells contained two agar layers of 0.3 ml each with 0.8% ultra low gelling temperature agarose (Sigma). The top layer contained 117000 cells and both Eagles MEM and 10% foetal bovine serum (FBS).

The compounds were diluted in bottom medium from a DMSO solution of 20 mM. The agar layers needed 60-90 minutes each in the refrigerator for consolidation, first time when tilting the plate 12 degrees. Consolidating the top layer with cells horizontally resulted in a nice cell density gradient

This time the growth in dense areas of the control 4-OH-OPB was absent in high concentration of the inhibitor and doubtful in medium concentration. The results were accepted since there was still a good difference between the inhibited growth in sparsely seeded areas compared to the growth in densely seeded areas.

The best inhibitor of clonal growth of sparsely seeded cells was #6. #4 showed a smaller effect, and #7 the smallest effect.

### Legend experiment 23

### Fig.1:

The polyoma virus transformed BHK21c13 cells, the line S100T1, was seeded in soft agar and treated with compounds that might be cytostatic to see if some inhibited sparsely seeded cells completely.

The compound #2 (Table 1) and probably also #1, had an effect very similar to 4-OH-OPB, but at at least 66 times higher concentration. They showed an effective clone inhibition of S100T1 cells in soft agar cultures with high concentrations of the compounds. The possibility of using these two analogues in treatment of diseases would very much depend on toxicological factors.

#3 did not show significant growth inhibition at all.

### Legend experiment 24

### Fig.1:

The polyoma virus transformed BHK21c13 cells, S100T1, were seeded in soft agar and treated with the compounds number 5, 8 or 9 (Table 1) to see if some inhibited sparsely seeded cells completely.

The cell containing top agar layer was molded on an oblique bottom layer for production of a cell density gradient. Both contained 0.8% ultra low gelling temperature agarose (Sigma) with Eagles MEM and 10% foetal bovine serum (FBS).

The compounds were solved in DMSO in a concentration of 20 mM and diluted in medium added to the bottom layer before consolidation in the refrigerator. The agar cultures were incubated at 37°C in 5% CO₂ atmosphere.

The resulting growth was photographed using an inverted Olympus microscope CK 40, 4 x objective and Olympus Camedia digital camera C-3040 zoom.

None of the compounds 5, 8 or 9 (Table 2) inhibited sparsely seeded S100T1 cells significantly.

### Legend experiment 25

### Fig.1:

S100T1 cells, polyoma virus transformed BHK21c13 cells, were seeded in soft agar and treated with compounds tested for clonal inhibition or clonal stimulation.

The wells had two agar layers of 0.3 ml each with 0.8% ultra low gelling temperature Sigma agarose type IX-A 2576, lot 19H082, Eagles MEM and 10% foetal bovine serum (FBS).

The compounds were solved in DMSO (20 mM) and diluted in medium and added to the bottom layer. Both layers needed 60-90 minutes each in the refrigerator for consolidation before incubation at 37 °C in 5% CO₂ atmosphere.

The bottom agar layer was consolidated obliquely for getting a cell gradient in the top layer.

Photographs were made using an inverted Olympus microscope CK 40, 4 x objective and Olympus Camedia digital camera C-3040 zoom.

4-OH-OPB analogue #10 inhibited clonal growth of sparsely seeded cells using medium concentration (about 50 µM) without affecting the same cells in densely seeded areas of the same well.

4-OH-OPB analogue #11 did not show any growth inhibition. But high concentration might stimulate growth in densely seeded areas.

This time the control substance 4-OH-OPB (batch 13 from Syntagon, Sweden) showed specific growth inhibition of only sparsely seeded cells in the range of 1-0.3 µM. When using 0.11 µM no growth inhibition was observed. In the well where 3 µM was added, there was a substantial inhibition of the growth in densely seeded areas. But some significantly growing clones occurred. This time the preparation was stored for 24 hours in the refrigerator after being solved in DMSO.

Azathioprin showed no clonal inhibition or stimulation of the cells.

### Legend experiment 26:

### Fig. 1:

The polyoma virus transformed BHK21c13 cells, the line S100T1, was seeded in soft agar and treated with compounds that might be clonal inhibitors or clonal stimulators to observe the effect on sparsely and densely seeded areas of the cell gradient.

The wells contained two agar layers of 0.3 ml each. Both contained 0.8% ultra low gelling temperature agarose type IX-A 2576, lot 19H082 (Sigma) with Eagles MEM and 10% foetal bovine serum (FBS).

The compounds were solved in DMSO in a concentration of 20 mM and then diluted in medium and added to the bottom layer before its consolidation that needed 60-90 minutes in the refrigerator for consolidation in an oblique position. 5% CO₂ was added to the atmosphere when incubated. The top agar layer with cells was consolidated in a horizontal position.

The resulting growth was photographed using an inverted Olympus microscope CK 40, 4 x objective and Olympus Camedia digital camera C-3040 zoom.

The carcinogenic Benzo(a)pyrene in high dose gave increased clonability. This phenomenon is believed to relate to the Carcinogenic effect of the compound.

Sulindac showed both a weak anti-cloning effect on sparsely seeded cells when adding high dose and a clone-inducing effect on the same cell density if the added dose was low. Low dose might also increase growth in densely seeded areas of the same well.

No effect on cell growth was observed when adding Altretamine.

### DEFINITIONS

As used herein the term "clonal inhibitors" or "clonal growth inhibitors" refers to substances that were able to inhibit single cells seeded in agar medium or on a surface or in fluid medium to form colonies.

As used herein the term "non-toxic active inhibitor" denotes a clonal growth inhibitor that shows very little or no toxic effect on cells growing in colonies or close together in culture and therefore probably also *in vivo.*

As used herein the term " primary tumour" is defined as a spontaneously developed tumour in animals or humans or tumours that were the result of application of carcinogens, micro organisms or physical stimulation (e.g. irradiation).

As used herein the term "metastasis" defines a distant spread of a daughter tumour in the body from a primary or metastatic tumour or a transplanted tumour.

As used herein the term "local infiltration" defines the infiltration of tumour cells that partly left the central tumour mass and partly was connected with it. Both penetrated surrounding tissues.

As used herein the term "weak point" refers to a period in the development of a tumour or a metastasis where not all tumours come through. Also in tissue culture, the cloning of a cell, especially normal ones, need special medium with special additions that might stimulate growth. Nevertheless, many cells will not be able to proceed. The number of successes is dependent on the composition of the medium and on the condition of the cell. It is anticipated that some chemical substances might be able or partly able to selectively inhibiting the process during the period of the "weak point".

As used herein the term "test for detecting carcinogens" is defined as the opposite, detection of compounds that are stimulating the cloning process in stead of inhibiting it. Such test results might replace or supplement data obtained by Ames test for carcinogenic activity Therefore, the test intended to find potential anti-cancer drugs might also detect the opposite: cancer-inducing drugs.

As used herein the term "collocation inhibition of clonal growth" is defined as proportional inhibition or neutralisation of the local effect of a clonal growth inhibitor mediated by high cell density in that special location. The cells themselves have the ability to decrease or abrogate the inhibition of cloning if present in high enough density in the particulate site.

### DETAILED DESCRIPTION OF THE INVENTION

The present invention relates to the use of 4-OH-OPB for preparing a pharmaceutical preparation for the treatment of psoriasis or for the treatment or prophylactics of arteriosclerosis or cancer, with the provision that said cancer is not malignancies derived from CD4 lymphocytes and HIV related Kaposis sarcoma.

A method of selection and testing of drugs, potential drugs, food, food additives, toxins, potential toxins, components from physiological or pathological processes, chemical compounds including microbes, the outcome of physical effects (e.g. radioactivity or ultrasound) on the body, parts of the body or on said substances for specific anti-cloning or clonal stimulating effect is described, said method comprises the following:

A clonal test to study the effect on cloning of said substances and;
a collocation inhibition test to study how increase of local cell concentration (increase of cell collocation) may decrease or abrogate the effect of said substances or physical effects on the process of cloning and on toxicity and;
tests for the ability of said substances or physical effects to influence the development of metastases in other ways than on cloning, e.g. on export of metastatic cells from a malignant tumour or location containing tumour cells.

To detect such compounds a method has been established that is able to control the qualities of substances found by screening. The method might have many modifications and one non limited example could be:
- Cells: Polyoma virus transformed baby hamster kidney cells (BHK21/c13), the line T100S1 for studying the effect on cancer cells and the untransformed parent line, BHK21/c13, for studying the effect on carcinogenesis.
- Cell culture: Growth in soft agar medium for developing clones both in sparsely seeded and densely seeded areas in the same culture. The cells should be grown in a top agar layer to avoid growth on the bottom surface. About 125000 cells/ml is needed to assure optimal growth (experiment 20). The normal cell line BHK21/c13 needed assistance of growth factors for development of clones in 0.8% soft agar medium (experiment 3).
- Agar: Ultra low gelling temperature agarose type IX or IXA, A-5020 or A-2576, Sigma, in 0.8% in the medium.
- Cell gradient: By tilting the 24 well Falcon plate with the bottom agar layers (with the compounds) 12 ° when consolidating the agar in refrigerator, a nice cell gradient would develop with the plate in horizontal position when consolidating the top layer with the cells.
- Reading results: After about two days the growth used to be optimal. The resulting growth was photographed using an inverted Olympus microscope CK 40 with 4 x objective and Olympus Camedia digital camera C3-3040 zoom. Both densely seeded and sparsely seeded areas were photographed.

The same principle could be tested in animals using the classical Jeme's test. Shortly described: Mice were immunised with sheep red cells. At the same time subcutaneous injection with a clone inhibitor, e.g. 4-OH-OPB, was performed. After 5 days the spleen was squeezed and spleen-cells counted and mixed with agar medium, sheep red cells and complement. After a short time plaques appeared and were counted and per cent of total number of spleen cells was calculated. The treated mice showed a dose dependent inhibition of growth stimulation of antibody producing cells by 4-OH-OPB treatment (see experiment 10). The treated mice did not show any sign of immunological incompetence. Therefore, it is probable that it is only the reaction against the new immunogen that is affected. This is analogous to what happen in the organism when single tumour cells were injected into the peritoneum and all the other cells in the neighbourhood were unable to abrogate the clonal inhibition of 4-OH-OPB. Only cells of the same specificity seemed to be the «victim» of clonal inhibition even if lots of other B-lymphocytes are present, but with other specificities. The only condition necessary seemed to be that affected cells should be located more than a certain distance from each other.

In addition, the experiment 10 also indicated that 4-OH-OPB could be transported from the subcutaneous injection site to an organ rich in blood and demonstrate an expected effect.

Finally, the experiment 9 showed a block in the export of metastatic cells from subcutaneously injected Ehrlich tumour cells resulting in local tumours in treated animals and only metastatic growth of ascites in untreated animals. High local cell density at the injection site did not abrogate this effect against export of metastatic cells, and cell cultures indicate that only a short distance apart from heavily concentrated cell masses, the clonal inhibition of 4-OH-OPB is fully active (experiment 13). This effect would probably also act against local infiltration of tissues, one of the chief characteristics of malignant growth.

### Thus the test for activity against cancer of a compound consisted of 3 main points:

1. Screening or testing of clonal inhibition
2. Screening or testing of collocation inhibition of clonal inhibition in the same culture.
3. Testing if export of metastatic cells (both local infiltration and metastases) from locations rich in such cells could be blocked.

Many of the reported cell culture experiments and all the animal experiments are directly involved in these tests.

Many other tests are necessary before a new drug could be borne, especially toxicity tests. However, high collocation inhibition would probably indicate low toxicity in the organism.

The three-point test for activity against cancer of a compound has been described over. However, the three-point list of other conditions where these tests may be relevant will be discussed in the follwing:
- Looking for substances having an effect similar to 4-OH-OPB, especially analogues of 4-OH-OPB and testing compounds for anti-carcinogenic properties and possible effect on cardiovascular diseases.
- Testing for carcinogenic or atherogenic properties.
- Testing components in the internal or external milieu such as physiological and pathological components of the body, food, food additives, preserving agents or stuff used for pleasure (e.g. tobacco) or contaminants.

### Testing for carcinogenic or atherogenic properties.

Testing for carcinogenic properties is relevant in many situations and for many compounds or physical effects e.g. like ionising irradiation, ultra sound (probably liberating insulin like growth factor) etc. Since inhibited clonal growth could indicate anti-cancer activity it is logical to assume that increased clonal growth might indicate carcinogenic activity. The compound benzo(a)pyrene, a known carcinogen, gave results that may indicate that this is correct (experiment 26). Also other compounds, Sulindac and Diclofenac, gave results indicating a possible carcinogenic activity, but further studies are needed (see experiments 26 and 15, and Table 2, Detailed description).

Insulin has been shown to stimulate clonal growth of normal, but not transformed cells growing in soft agar culture (Tjøtta E. (1968) Arch. ges. Virusforsh. 25, 363-364), see experiment 3. Hyperinsulinemia is predicting increased risk of coronary atherosclerosis (Tuttle K R. et al. (1999) Am. J. Kidney Dis. 34, 918-925).

Also conditioned medium showed similar properties as insulin in this experiment, but its effect has been known for quite a long time (Tjøtta E., Flikke M. and O. Lahelle (1967) Arch. ges. Virusforsh. 23, 288-291). The significance of this is not fully understood, and it may indicate that factors produced by living cells could stimulate cell growth in a way that might be pathological. Both cancer and arteriosclerosis are diseases that are increasingly frequent in old age and may be caused by a high level of factors analogous to conditioned medium. Therefore, it might be relevant to test for such activity in old people and compare with the level in younger individuals.

Among the available 11 analogous compounds to 4-OH-OPB four have demonstrated similar activity in cell culture (see Table 1, Detailed description).

Many substances from the body, food, drugs or environment might have anti-carcinogenic or anti-clonal properties. In Table 2 of Detailed description are listed preliminary results of testing some NSAID's. Many have been shown having anti-carcinogenic effect before and might also prevent cardiovascular disease. This activity seems to correlate with the effect on clonal growth.

There probably is a balance in normal individuals between clonal inhibitors and stimulators of clonal growth that secure health. The described method might be helpful in discovering such a balance. We have started with two hormones insulin and growth hormone, but others should follow. In special individuals, e.g. Down's syndrome, there is less arteriosclerosis than expected. Therefore, search using the methods presented here might discover the reason. There is no good animal model for studying development of arteriosclerotic disease. The described in vitro tests might be a good beginning. Screening compounds, body components, food, drugs or pollutants for possible effects would probably need a normal cell line, e.g. BHK21/c13. Since testing for possible stimulation of these cells in soft agar culture seems necessary, other stimulants should be used in minimal amounts in order to increase the sensitivity of the test.

Many cytotoxic agents have been used for treatment of malignant disease and some have been tested here.

Testing of food, food additives, preserving agents or stuff used for pleasure (e.g. tobacco) and pollutants has not jet been undertaken, but is probably important since such agents from the external milieu has been considered being the cause of many diseases. A more precise knowledge would make prevention easier, and be important for informing the "man in the street".

**Table 1, Detailed description:**

| Analogues of 4-OH-OPB (see experiment 25, Table 3). Positive reactions in cursive. | | | | |
|---|---|---|---|---|
| No | | Low | Medium | High |
| #1 H | p-hydroxy-azobenzene | +++ | +++ | +(+) |
| #1 L | p-hydroxy-azobenzene | ++ | ++ | +- |
| #2 H | 2-Butyl-2-hydroxy-N-(4-hydroxy-phenyl)-N'-phenyl malonamide | ++(+) | ++ | ++ |
| #2 L | 2-Butyl-2-hydroxy-N-(4-hydroxy-phenyl)-N'-phenyl malonamide | ++ | ++ | - |
| #3 H | 2-oxo-hexanoic acid phenylamide | ++(+) | ++(+) | ++ |
| #3 L | 2-oxo-hexanoic acid phenylamide | ++(+) | ++(+) | ++ |
| #4 H | 2-Butyl-N-(4-hydroxy-phenyl)-N'-phenylmalonamide | ++ | ++ | ++ |
| #4 L | 2-Butyl-N-(4-hydroxy-phenyl)-N'-phenylmalonamide | ++ | ++ | ((+)) |
| #5 H | 4-OH-PBOMe | ++ | +(+) | + |
| #5 L | 4-OH-PBOMe | ++(+) | ++(+) | + |
| #6 H | 1,2₋diphenyl-4-hydroxy-4-[2-(phenylsulfinyl)ethyl]-3,5-pyrazolidinedione. | ++ | ++ | ++ |
| #6 L | *1,2-diphenyl-4-hydroxy-4-[2-(phenylsulfinyl)ethyl]-3,5- pyrazolidinedione* | ++ | ((+)) | - |
| #7 H | *4-(1-butyl)-4-hydroxy-1-(4-methoxyphenyl)-2-phenyl-3,5-pyrazolidinedione* | ++ | ++ | ++ |
| #7 L | 4-(1-butyl)-4-hydroxy-1-(4-methoxyphenyl)-2-phenyl-3,5-pyrazolidinedione | ++ | ++ | (+) |
| #8 H | 1,2-diphenyl-4-hydroxy-4-(4-methylphenyl)-3,5-pyrazolidinedione | +(+) | ++ | ++ |
| #8 L | 1,2-diphenyl-4-hydroxy-4-(4-methylphenyl)-3,5-pyrazolidinedione | +(+) | +(+) | + |
| #9 H | 4-benzyl-1,2-diphenyl-4-hydroxy-3,5-pyrazolidinedione | ++(+) | +++ | ++ |
| #9 L | 4-benzyl-1,2-diphenyl-4-hydroxy-3,5-pyrazolidinedione | +(+) | + | + |
| #10 H | *4-hydroxy-4-aldehyd-1,2-diphenyl-pyrazolidinedione* | ++ | ++ | ((+)) |
| #10 L | *4-hydroxy-4-aldehyd-1,2-diphenyl-pyrazolidinedione* | ++ | - | - |
| #11 H | 4-butyl-4-hydoxy-1,2-diphenyl-3,5-pyrazolidnedione | ++ | ++ | ++(+) |
| #11 L | 4-butyl-4-hydoxy-1,2-diphenyl-3,5-pyrazolidnedione | ++ | ++ | ++ |

| | | | | |
|---|---|---|---|---|
| Increasing clonal growth: ((+)), (+), (+, +, ++, ++(+), +++, no clonal growth: +-, - H: high density area of cell gradient, L: low density area of cell gradient | | | | |

*Conclusion: 4 of the 4-OH-OPB analogues, #1, #2, #6 and # 10 showed complete inhibition of growth in sparsely seeded areas of agar culture of S100T1 cells when growth in densely seeded areas of the same culture showed significant growth.*

**Table 2, Detailed description, showing preliminary results when testing effect on cell cloning in soft agar: See experiments 13-18,21-26**

| No | | Low | Medium | High | |
|---|---|---|---|---|---|
| 1H* | Acetyl salicylic acid | | | | |
| 1L | Ac. salicyl. acid E15,16 | (+) | | ((+)) | Possible inhibition of clonal growth |
| 2H# | Actino-mycin D | ++ | ++ | + | Good, but not complete inhibition |
| 2L | Actino-mycin D E21 | + | (+) | ((+)) | of clonal growth at low toxicity |
| 3H# | Altretamine | ++ | ++ | ++ | No effect |
| 3L | Altretamine E26 | ++ | ++ | ++ | |
| 4H# | Azathioprin | ++ | ++ | ++ | No effect |
| 4L | Azathioprin E25 | ++ | ++ | ++ | |
| 5H | Benzo(a)pyrene | ++ | ++ | ++ | Increased clonal growth at low density |
| 5L | Benzo(a)pyrene E26 | ++ | ++ | +++ | and high concentration |
| 6H* | Colchicine | +++ | +++ | +++ | Growth in high density areas |
| 6L | Colchicine E13 | - | - | - | No growth in low density areas |
| 7H# | Cyclophosphamide | ++(+) | ++(+) | ++ | When unactivated growth increased in |
| 7L | Cyclophosphamide E19 | ++ | ++ | ++ | densely seeded areas up to medium conc. |
| 8H# | Cytarabin | ++ | ++ | (+) | Incomplete inhibition of clonal growth |
| 8L | Cytarabin E21 | + | + | (+) | and significant toxicity |
| 9H¤ | Diclofenac | | | | |
| 9L | Diclofenac E 15 | ++(+) | | ++ | A possible increase of clonal growth |
| 10H# | Etoposide | + | + | (+) | Reduced growth both in densely and |
| 10L | Etopoiside E17 | (+) | (+) | (+) | sparsely seeded areas |
| 11H# | Fluorouracil | ++ | ++ | ++ | Rel. moderate inhibition of clonal growtl |
| 11L | Fluorouracil E21 | + | + | + | and low toxicity independent of dosage |
| 12H* | Ibuprofen | | | | |
| 12L | Ibuprofen E 15,16 | (+) | | (+)) | Clonal inhibition possible |
| 13H§ | Mycophenolic acid | (+) | (+) | (+) | Less growth in both high and low |
| 13L | Mycophenolic acid E19 | (+) | (+) | (+) | concentration and cell density |
| 14H* | Naproxen | | | | |
| 14L | Naproxen E15,16 | (+ | | (+) | Clonal inhibition possible |
| 15H* | Piroxicam | | | | |
| 15L | Piroxicam E15 | ++ | | + | Clonal inhibition possible |
| 16H# | Podophyllotoxin | (+ | (+ | (+) | A weak clonal inhibition and |
| 16L | Podophyllotoxin E 15-17 | + | + | (+) | cytotoxic |
| 17H | Somatotropin | ++(+) | ++(+) | ++ | Growth increase at both cell densities at |
| 17L | Somatotropin E25 | ++(+) | ++(+) | ++ | low and medium concentration |
| 18H¤ | Sulindac | ++(+) | ++ | ++ | Possible clonal stimulation at low |
| 18L | Sulindac E26 | +++ | ++ | + | concentration |

| | | | | | |
|---|---|---|---|---|---|
| Increasing clonal growth: ((+)), (+), (+, +, ++, ++(+), +++, no clonal growth: +-, - H: high density area of cell gradient, L: low density area of cell gradient *: Mostly NSAID's, many with anti-carcinogenic effect and with effect on cardiovascular disease. #: These are cytotoxic agents and many are used in treating malignant disease. ¤: These are in use as NSAID's, but has not shown any cancer preventing effect. § : Immune suppressant | | | | | |

A well known cause of increased incidence of malignancies is exposure to x-rays or radioactivity. The inventor has observed that liberation of growth factor(s) that stimulate(s) clonability from cells being frozen and thawed or exposed to ultra sound. Therefore, physical effects on cells may also influence the level of clonability. Since decrease of clonability is correlated to anti-cancer effect, the opposite, compounds stimulating clonal growth might stimulate carcinogenesis or development of metastases.

Clonal inhibitors are of interest in treatment or prophylaxis of several diseases or disorders. Inherited or phenotypic abnormalities might be detected before the development of disease (e.g. cancer). These substances or principles changing clonal growth might be of interest for reproductive or other physiology and embryology. Embryology and reproductive physiology since the key processes are development of new clones , and normal physiology since changes in old age seems to create a better milieu for abnormal clonal growth After selecting compounds in culture, there is need for studying the toxic effects both in cultures, animals or man and to test the inhibiting potential on tumours and the effect on tumour cell spread in the body

The higher the "detoxifying" or "inactivating" collocation effect, the less toxic the substance is supposed to be for the body. Such substances having low toxicity could be expected to be used over long time periods preventing recurrences or metastatic growth, but not to inhibit tumours larger than a certain size.

Data from the testing of clonal inhibition, toxicity, inhibition of the anti-clonal growth effect by local collocation of tumour cells and the influence on metastatic cellular spread (see later) would be important when ranking substances tested. If, in addition, these results were compared with those previously examined and better known candidates, it might be possible to put up a priority list of candidates that should be examined further.

When testing candidate drugs for the treatment of a certain patient, the data from the clonal test together with the collocation inhibition test and, if possible, the effect on the development of metastases will probably indicate the expected clinical effect more precisely than clonal inhibition alone. These data might also indicate the optimal clinical stage for using a particular drug and the optimal drug for a particular patient.

Evaluation of the ability to reduce the export of metastatic cells also is vital for clinical use. An example of such test has been described using mice and Ehrlich carcinoma (experiment number 9). The indicated treatment is expected to reduce or eliminate the chance of metastatic spread during and after necessary conventional treatment, mainly surgery, irradiation or cytostatics. However, the growth is not expected to decrease if tumours were over a certain size. It is the inventor's opinion, however, that such tumours might behave more like benign tumours since treatment might stop secondary metastatic spread (experiment 9) and local infiltration (see experiment 13).

The animal experiments indicate that other cells in the animal with other phenotypes do not act as collocation inhibitors of the cloning inhibition of Ehrlich carcinoma cells. One of the test substances; 4-OH-OPB, a substance previously known to treat viral infections, seems to be fully active on tumour cells in the peritoneum of mice even when the cell mass of organs close to peritoneum far exceeds the cell mass of the injected tumour cells (experiments 7-8). Further, the whole immune system seems not affected when treated with 4-OH-OPB, only the reaction against new intruders (experiment 10).

Further, Ehrlich ascites tumour in mice, is used as a good example of single tumour cells capable of developing malignant colonies after what is supposed to be cloning-like processes when seeded in peritoneum. When treated at the same time as being transplanted, before having chance of being cloned, these cells were inhibited easily and cannot be traced at the time when untreated control mice die with extensive involvement (experiment 7-8).

However, postponing treatment one day or more made the disease resistant for even several times higher doses than those that were able to cure the malignancy when treated immediately during transplantation (experiment number 8).

But the higher the dose of intraperitoneally injected 4-OH-OPB, the smaller the necessary number is of subcutaneously injected Ehrlich tumour cells that might induce tumour at the site of injection.

An inhibiting effect on a supposed metastatic migration of Ehrlich tumour cells by using 4-OH-OPB would explain these observations since untreated control developed ascites only, and no local tumours at the subcutaneous injection sites.

Therefore, there is at least two distinct effects of 4-OH-OPB, one is anti-clonal growth, the other is an anti-metastatic effect that seems to inhibit export of metastatic cells from the subcutaneously transplanted cell mass. Other collocated tumour cells inhibit the first of these, not the other. The inventor has shown that 4-OH-OPB inhibits growth and migration of cells coming from an area containing many transplanted tumour cells.

It has not previously been shown that the detected substance 4-OH-OPB has the ability to act as a selective inhibitor of clonal growth and an inhibitor of the export of metastatic cells. In the patent application No PCT/GB00/02513 of by A-Viral ASA it is only expected that this compound (AV-1101) would inhibit or cure malignancies derived from CD4 lymphocytes and HIV related tumour Kaposis sarcoma.. Therefore, such malignancies are not included in this application.

The explanation of the experiments in mice are in accordance with experiments in cell cultures where even small colonies are much more resistant against treatment than single cells (experiment 6). It is assumed that during delay of 24 hours or more before starting treatment after transplantation of Ehrlich ascites in mice, some cells might have had the chance to start growing. This might have created places with new cells or colonies where collocation is able to suspend or significantly decrease the effect of the inhibitor of clonal growth.

When injecting Ehrlich cells subcutaneously, the injected cells probably quickly started "travelling" to peritoneum. This migration seemed to be so effective that only peritoneum contained tumour cells after subcutaneously injecting such cells in the untreated control.

However, when treated with intra-peritoneal injections of the clonal inhibitor 4-OH-OPB, no spread of tumour cells from these subcutaneous injection sites were found in peritoneum.

Further, when testing inhibitors of tumours from individual patients, the results of clonal growth should be interpreted correctly and treatment instituted during and after removing or inactivating detectable tumours or metastases. There is hope of getting control of remaining single cell metastases after starting such treatment. Used in this way it is expected to be life saving in a number of patients. There is also a possibility that such treatment of inoperable tumours might inhibit or stop development of metastases.

Regarding spontaneous or induced tumours, prophylactics might be relevant when there is great risk of contracting the disease, e.g. familiar breast cancer or colon cancer.

A useful possibility for studying spontaneous tumours may be tests using animals developing tumours spontaneously (e.g. Dr. Reidar Eker's kidney tumour in rats). Also prevention or treatment of cancer in old cats or dogs would be interesting since it should be possible to obtain a sufficient number. However, candidate drugs for prophylactics of spontaneous tumours should preferably first be selected by using cultured normal cells being stimulated to be able to form colonies in soft agar (e.g. experiment number 6).

An evaluating system for anti-cancer drugs mentioned before comprises:
1. Screening or testing of clonal inhibition
2. Screening or testing of collocation inhibition of clonal inhibition in the same culture.
3. Testing if export of metastatic cells (both local infiltration and metastases) from locations rich in such cells could be blocked.

More specific:

A method of selection and testing of drugs, potential drugs, food, food additives, toxins, potential toxins, components from physiological or pathological processes, chemical compounds including microbes, the outcome of physical effects (e.g. radioactivity or ultrasound) on the body paints of the body or on the effect of already mentioned substances for specific anti-cloning or clonal stimulating effects that is comprising:
a) A clonal test to study the effect on cloning of said substances;
b) A collocation inhibition test to study how increase of local cell concentration (increase of cell collocation) may decrease or otherwise alter the effect of said substances or physical effects on the process of cloning. It is expected that high clonal inhibition without toxic effect on densely seeded areas indicates low toxicity in the body and;
c) Tests for influencing the development on metastases in other ways than cloning, e.g. by said substances or principles on export on metastatic cells from a malignant tumour or location containing tumour cells.

The present invention relates to the use of 4-OH-OPB for preparing a pharmaceutical preparation for the treatment of psoriasis or for the treatment or prophylactics of arteriosclerosis or cancer, with the provision that said cancer is not malignancies derived from CD4 lymphocytes and HIV related Kaposis sarcoma.

In the following each step of the method will be explained in greater detail.

### The clonal test

The clonal test comprises:
1. seeding cells in soft agar optionally containing growth factor(s);
2. handling or incubation of special gels (e.g. ultra low gelling temperature agarose), including the production of cell density gradients.
3. incubating in suitable temperature and atmosphere and;
4. regular monitoring of the cells and development of clones.

The clonal test can be directed in fluid medium or in soft agar medium. Usually only transformed cells will form clones in soft agar. In 0.8% soft agar (Sigma Agarose Type IX or Type LXA) as top agar layer with Eagles MEM or RPMI 1640 and 10 % foetal bovine serum only S100T1 (polyoma virus transformed BHK21/c13 cell line) was able to form colonies, not the parental strain. Normal cell lines needed stimulation by growth factors (experiment number 3).

Baby hamster kidney cells (BHK21/c13) was used that were transformed (S100T1) by a small plaque strain of polyoma virus. The virus and BHK21/c13 cells were both obtained from Macpherson's laboratory in Glasgow in 1964. Our findings (experiment number 3) are in agreement with the report of Macpherson and Montagnier that only transformed cells could form colonies in soft agar.

However, many other cells probably are suitable in this test, and we need to mention some: malignant cells, normal cells, cell lines, transformed cells and cells from patient tumours or cells from the immune system (e.g. the spleen) being clonally selected after immunization (experiment number 10). The latter can also be detected or quantified using e.g. Jerne's classical test.

Other cloning techniques, e.g. cloning in fluid medium using micro-well plates are as well possible for the detection of clonal inhibitors.

Addition of other growth factors than insulin may be relevant, especially for normal cells growing in agar. Experimental testing of some examples has been described later (experiment 3 and 6). Normal cells might be relevant for finding substances that were able to inhibit carcinogenesis since malignant tumours are expected to develop from more or less normal cells in the body.

The results of the experiments number 3 and 6 show clearly that it is possible to study the clonal growth and inhibition of clonal growth in normal cells (BHK21/c13), after providing growth enhancing substances to said cells. Some of these are listed here:

Conditioned medium stimulates the growth of clones both from normal and transformed cells (Tjøtta E., Flikke M. and O. Lahelle (1967) Arch. ges. Virusforsh. 23,288-291). The effect can be greatly improved by concentration and could be essential when using tumour cells from patients.

Insulin is a very important factor that stimulates the growth of clones of normal, but probably not of transformed or tumour cells (E. Tjøtta, Arch. Ges. Virusforsch. 25, 363-364, 1968).

Factors in serum are potent growth stimulators of all cells, but serum alone is not enough for cloning of normal BHK21/c13-cells in agar.

Also insulin-like growth factor would probably stimulate cloning in normal cells, not the polyomavirus transformed and cytokines may be active.

Serum extenders e.g. Mito+ is found to allow growth of normal cells together with small amounts of serum. A combination of these factors is also active.

In experiment number 4 an 6 the compound 4-OH-OPB is tested for clonal inhibition of BHK21/c13 cells optionally transformed by polyomavirus growing in agar. The result showed clearly that no growth or inhibited growth was observed in cultures that were treated with 4-OH-OPB.

Finally the other aspect of the test, where cloning is stimulated in stead, should be performed similarly. Based on the theory that cloning is essential for development of cancer or its metastases, an increase of clonability by chemical compounds, physical effects, infectious agents etc. would indicate a possible carcinogenic effect and stimulation of the development of metastases and possibly influence on other pathological conditions where cloning might be part of the aetiology (e.g. atheromatosis).

### Collocation inhibition test

The collocation inhibition test comprises;
a) transplantation of dispersed tumour cells to animals, e.g. Ehrlich ascites to mice, or seeding of experimental cell cultures with the cells in the way previously described;
b) supplying the test with substances previously described and;
c) monitoring the tumour cells in the animal or the cells in experimental cell cultures for abrogation of inhibited clonal growth in areas or locations with many cells.

After selecting the compounds in tissue culture, there is need for studying the toxic effects in cell culture, animals or man and to test the inhibiting potential on tumours in animals.

A good example of a tumour suitable for studying tumour cells being cloned *in vivo* is Ehrlich ascites tumour in mice. Tumour cells harvested from ascitic mouse were mainly solitary. Transplanted to a new animal these cells will be distributed over large surfaces in the peritoneum and by the time of transplantation the cells do not have chance to form aggregates that might resist clonal inhibition due to collocation. However, when postponing the treatment for one day, clonal inhibition is not easy to obtain (experiment number 8). This is supposed to be caused by local increase in cell density or collocation caused by growth or aggregation or both. Also cell culture experiments indicate a decreased growth inhibition after having been growing before treatment or having been seeded more densely (experiment number 5 and 6).

### Test for influencing the development of metastases

The test for influencing the development of metastases comprises:
a) injection of different numbers of tumour cells in different locations of animals for testing the capability to generate metastases or local tumours;
b) supplying the test substance previously described and;
c) monitoring the ability of said test substances to reduce the migration of tumour cells and induce development of local tumours at the injection sites in animals..

Development of metastases depends on cells that can migrate from a primary tumour or primary transplantation site and grow at the new more preferred location. There are separate assays testing the effect of candidate drugs etc. on each of these stages:

This assay involves different numbers of Ehrlich carcinoma cells injected in six subcutaneous locations of mice that were treated by intraperitoneal injections of the compound tested, e.g. 4-OH-OPB (experiment number 9). The treatment was effective in stopping migration of tumour cells to peritoneum. The cells were probably arrested in the original subcutaneous location where the local cell concentration consequently became higher then that in untreated controls. When operating with different cell numbers in the different injection sites, the number of growing tumours indicated which cell number that is necessary for the development of tumours in treated or untreated animals.

The next test is the assay detecting the effect on clonal inhibition performed either in cell culture or in mice or in other animals if necessary.

The first test is preferably using tumour cells capable of generating metastases (e.g. Ehrlich carcinoma, experiment number 9) the other could also be using the same transplantable ascites tumour injected intraperitonally in mice treated with the compound under test, (e.g. the 4-OH-OPB, experiment number 7 and 8). In cell culture, the polyoma virus transformed BHK21c13 cell line S100T1 has been used as example (experiment number 2,4-6).

Each detected new compound having an effect on clonability in cell culture needs to be evaluated for the ability of collocated cells to inhibit or modify this effect. Collocation of cells seems not to affect the effect on the export of metastatic cells, only the effect on clonal growth.

Further, when testing individual patients for inhibitors of their own tumour cells, the results of clonal growth should be interpreted correctly and treatment instituted after or during removing or inactivating tumours or metastases that were too great for cytotoxic treatment. There is hope of getting control of single cell metastases after starting the treatment. Used as indicated it is expected that this effect will be life saving in a number of patients.

Next, even with greater tumours that might be inoperable, the indicated treatment might reduce or stop the export of metastases since collocation do not seem to interfere with the effect on export of metastatic cells.

It has not previously been shown that the detected substance 4-OH-OPB has the ability to act as a selective clonal inhibitor and inhibitor of the export of metastatic cells.

The present invention comprises the use of the substance 4-OH-OPB that has been shown to stop growth of Ehrlich carcinoma cells transplanted to peritoneum of mice if the mice were treated immediately. Export of Ehrlich cells from the subcutaneous injection site is probably also stopped. The subcutaneous collocation of Ehrlich cells, however, had to be higher than a certain minimum in order to support local tumour growth in treated animals. To sustain the local collocation of relatively few injected tumour cells at a level where local growth was possible, quite high doses of 4-OH-OPB was needed. If the injected number of tumour cells was relatively low, even higher doses of 4-OH-OPB were necessary for the induction of local growth. That means that both cell number and concentration of 4-OH-OPB in tissues acts in the same way. The inventor believes that 4-OH-OPB increases local concentration of tumour cells because they no longer migrate or migrate less than before. Only if the local tumour cell concentration becomes higher than a certain minimum local tumour growth could start.

### The compounds that inhibit clonal growth

The substances obtained by the described method can be used for pharmaceutical preparations for the treatment or prophylactics of cancer, arteriosclerosis, autoimmunity, and rejection of transplants or pathological processes related to the growth that might be initiated by radioactivity or other physical effects.

Regarding spontaneous or primary tumours, prophylactic measures might be relevant when there is great risk of contracting the disease, e.g. familiar cancer mamma or cancer of the colon.

Compounds that inhibit clonal growth should be tested to exclude a too strong inhibition of the immune system. The immune system is very much dependent on its ability to produce clones that were effective against new infections. The use of compounds inhibiting the growth of malignant clones might be important for prophylactics of cancer and inhibiting the development of metastases, but might also influence physiological processes negatively, e.g. natural cloning processes in the immune system and organogenesis in the foetus.

In order to examine the degree of diminishing cloning in the immune system, Jerne's test in mice may be suitable (Experiment 10). Sheep erythrocytes are injected into mice. A few days later cells from the spleen of the animals are studied. In experiment 10 the smallest dose was about 3.8 µM if 4-OH-OPB was solved in the extracellular fluid of the mouse. The smallest effective dose in experiment 8 was 0.37 µM in extracellular fluid and 4.7 µM in peritoneum. But there are indications that normal cells might tolerate higher doses than malignant cells. *Therefore, it is thought that effective tumour inhibiting doses of 4-OH-OPB probably will affect the clonability of antibody producing cells in the spleen under or shortly after primary immunisation moderately, about 2 logs.*

Clonal inhibitors, however, might be dangerous for foetal development since the development of organs is based on clones.

The compound, 4-OH-OPB, that is not a classical cytostatic, was found by the described method dealing with detection of possible drugs suitable for cancer treatment or treatment of other diseases where cloning is a central part ofpathogenesis (e.g. arteriosclerosis). This compound might become a drug not previously being shown possessing said effect. 4-OH-OPB is probably less toxic than any cytostatic drug, but nevertheless capable of arresting tumour progression when the malignant process is in its most vulnerable period i.e. the process of cloning.

Since the process of cloning is considered more difficult for the cell to go through than regular cell growth assisted by collocated cells, it is assumed that in the body the initial cloning of a primary tumour and even the start of metastases or local infiltration from single or few cells is easier to inhibit than already cloned bigger tumours. However, export of cells from "bigger" tumours or a location rich in tumour cells might be inhibited by treatment. This effect is thought to induce a more benign behaviour of a malignant tumour and possibly a full change from malignant to benign growth.

### Discussion

It is important to verify the anti-tumour effect in animals of compounds discovered by the method indicated above. Tumour cells could be injected into peritoneum and the compound that is tested could be injected in the same place. Tumour cells might be injected in lymphatic vessels in order to study the development of tumours in lymph nodes or injected in other cavities or tissues to study the growth in such places. Also, haematogenous dissemination of tumour cells might be studied using intravenous or intra-arterial injections of transplantable tumour cells.

Results obtained by using transformed or tumour cells are supposed to show possible effects on the development of tumour metastases better than using normal cells.

Cloning of normal cells in soft agar needs growth factors added to the medium. Suppressers of this system would probably yield results relevant for the development of primary cancer or arteriosclerosis.

The growth factors used in the experiments are examples of many other relevant substances.

It is important to know the distribution, elimination and transport of the tested candidate drugs in the body. When injected in the same compartment as the tumour (e.g. peritoneum) no transport through the circulation is necessary. But injecting the compound in other sites than where the transplantable tumour is inoculated will provide data indicating how effective the transport through the circulation might be.

Compounds inhibiting insulin or insulin-like growth factor might be more effective against primary tumours or atheromatosis since these factors are not expected to stimulate transformed cells (E. Tjøtta, Arch. Ges. Virusforsch. 25, 363-364, 1968). However, serum and conditioned medium in combination stimulated both transformed and normal cells and inhibitors of these factors might retard both primary and metastatic tumour cell growths.

It was assumed that among mitotic inhibitors like OH-urea, colchicine or podophylline or derivatives there would be found some with activity. Also compounds with analogous chemical structure to 4-OH-OPB were thought having a chance to possess activity. Further NSAID's that has been found to decrease the incidence of malignant tumours or vitamins and minerals, especially those with anti-oxidative effects with possible similar effect on tumour incidence, represent very interesting groups. These groups have provided candidates with activity shown in Table 1-2 in Detailed description.

If cloning is an important part of the development of arteriosclerosis, as many think, prophylactic treatment is expected to be effective. Plaques larger than a certain size are not expected to react because of inhibition of anti-clonal effect by collocated cells. If HSV is participating, 4-OH-OPB with an additional anti-viral effect (Experiment number 11a,b and 12) could still be effective on greater plaques, but this is speculation that will need verification.

Also benign tumours elsewhere, e.g. in the skin (nevi, warts, café au lait spots, senile warts etc) might be sensitive. Diseases having increased mitotic activity like psoriasis are expected to benefit.

Sera from Down's syndrome will be tested to see if growth stimulation of clones might be different from normal people. If reduced or altered clonal stimulation can be shown in such sera, it may explain why these people have less incidence of arteriosclerosis and higher incidence of Alzheimer's disease and infections.

In the literature the compound 1-1-dimethylhydrazine that enhances development of plaques in Jerne's test (see experiment number 10) was reported having carcinogenic activity. Therefore, it is probable that also other compounds with similar effect in Jerne's test or other clonal tests can have a cancer promoting effect.

Some 4-hydroxy-3,5-dioxo-pyrazolidines are described in the literature for treatment of HIV infections and some other viral infections, tropic spastic paraparesis, autoimmune diseases, transplantation rejection and special tumours as Sezary syndrome, mycosis fungoides, T-cell lymphoma, and Kaposis sarcoma. The example used in the described experiments is 4-OH-OPB, which is 4-butyl-4-hydroxy-2(p-hydroxyphenyl)-1-phenyl-3,5-pyrazolidinedione with the general formula: 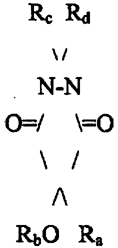 where Ra - Rd might be defined as in Table 1, Discussion, on next page:

The immunosuppressive and antineoplastic drugs available might have inhibiting effects on clonability of tumour cells, and possibly also inhibit metastatic migration.

However, collocated cells might partly or fully neutralise the effect on clonability if the substances have low toxicity. This knowledge is probably very important when treating patients, especially those with malignant diseases. Since most of the cytotoxic drugs used against cancer also is expected to have a smaller or greater effect against clonal growth, there are possibilities of finding suitable candidates among them that could be used in reduced, less toxic concentrations over long time periods giving effects as described for clonal inhibitors.

Therefore, the substances exemplified in the following might be potential candidates and is currently tested by the method of the invention in the inventor's laboratory.

**Table 1, Discussion:**

| Rₐ | R_{b} | R_{c} | R_{d} |
|---|---|---|---|
| H | H | H | C₆H₅ |
| H | H | C₆H₅ | C₆H₅ |
| CH₃ | H | H | C₆H₅ |
| CH₃ | H | H | -CH₂-C6H₅ |
| CH₃ | H | H | P-CH₃0-C6H₄ |
| CH₃ | H | H | p-Cl-C6H₄ |
| C₂H₅ | H | H | C₆H₅ |
| C₂H₅ | H | C₆H₅ | C₆H₅ |
| C₂H₅ | H | H | N-methyl-piperidin-4-yl |
| iC₃H7 | H | H | C₆H₅ |
| nC₃H7 | H | H | C₆H₅ |
| nC₃H7 | H | C₆H₅ | C₆H₅ |
| C₃H7 | H | H | 5-phenyl-triazol-1-yl |
| C₄H9 | H | H | C₆H₅ |
| c₄H9 | H | C₆H₅ | C₆H₅ |
| C₄H9 | H | o OH-C₆H₄ | o OH-C₆H₄ |
| C₄H9 | OH | m OH-C₆H₄ | m OH-C₆H₄ |
| C₄H9 | OH | p OH-C₆H₄ | p OH-C₆H₄ |
| C₄H9 | H | H | N-methyl-piperidin-4-yl |
| C₅H₁₁ | H | H | C₆H₅ |
| C₅H₁₁ | H | C₆H₅ | C₆H₅ |
| C₅H₁₁ | H | H | 5-phenyl-triazol-1-yl |
| Cyclohexyl | H | H | C₆H₅ |
| Phenyl | H | H | C₆H₅ |
| Phenyl | H | C₆H₅ | C₆H₅ |
| Benzyl | H | H | C₆H₅ |
| Benzyl | H | C₆H₅ | C₆H₅ |
| CH₃CO(CH₂)₂ | H | C₆H₅ | C₆H₅ |
| (CH₃)₂C=CH- | H | C₆H₅ | C₆H₅ |
| (CH₂)₂C=CHCH₂- | H | C₆H₅ | C₆H₅ |
| C₆H₅SOCH₂CH₂- | H | C₆H₅ | C₆H₅ |
| Pyrrolidin-1-yl | H | C₆H₅ | C₆H₅ |
| Piperidin-1-yl | H | C₆H₅ | C₆H₅ |
| Morpholip-4-yl | H | C₆H₅ | C₆H₅ |

### Immunosuppressive drugs

### Selective immunosuppressive drugs

| | |
|---|---|
| Cyklosporin | Sandimmun «Novartis» |
| | Sandimmun Neoral «Novartis» |
| Muromonab-CD3 | Orthoclone OKT3 «Janssen-Cilag» |
| Tacrolimus | Prograf «Fujisawa» |
| Mycophenolic acid | CellCept «Roche» |
| Basiliximab | Simulect «Novartis» |
| Etanercept | Enbrel "Wyeth-Lederle" |
| Infliximab | Remicade "Schering-Plough" |
| Leflunomid | Arava «Aventis Pharma» |

### Other immunosuppressive drugs

| | |
|---|---|
| Azatioprin | Imurel «GlaxoSmithWellcome» |

### Antineoplastic drugs

### Alkylating substances

### Mustard gas analogues

| | |
|---|---|
| Cyklofosfamide | Sendoxan «Asta Medica» |
| Chlorambucil | Leukeran "GlaxoSmithKline" |
| Melfalan | Alkeran «GlaxoSmithKline» |
| Ifosfamide | Holoxan «Asta Medica» |

### Alkylsulfonates

| | |
|---|---|
| Treosulfan | Treosulfan «LEO» |

### Etylenimines

| | |
|---|---|
| Tiotepa | Thiotepa «Wyeth Lederle» |

### Nitroso-urea compounds

| | |
|---|---|
| Lomustine | Lomustine «medac» |

### Other alkylating substances

| | |
|---|---|
| Temozolomide | Temodal «Schering-Plough» |

### Antimetabolites

### Folic acid analogues

| | |
|---|---|
| Metotrexate | Emthexat «Nycomed Pharma» |
| | Methotrexate «Wyeth Lederle» |
| Raltitrexed | Tomudex «AstraZeneca» |

### Purine analogues

| | |
|---|---|
| Merkaptopurine | Puri-Nethol «GlaxoSmithKline» |
| Cladribine | Leustatin «Janssen-Cilag» |
| Fludarabin | Fludara «Schering AG» |

### Pyrimidine analogues

| | |
|---|---|
| Cytarabine | Cytarabin «Pharmacia» |
| | Cytosar «Pharmacia» |
| Fluorouracil | Fluorouracil «Faulding» |
| | Flurablastin,"Pharmacia» |
| Gemcitabine | Gemzar «Lilly» |

### Plant alkaloids etc.

### Vinca alkaloids and analogues

| | |
|---|---|
| Vinblastine | Velbe «Lilly» |
| Vinkristine | Oncovin «Lilly» |
| Vincristine «Pharmacia» | |
| Vinorelbine | Navelbine «Pierre Fabre Pharma Norden» |

### Podophyllotoxin derivates

| | |
|---|---|
| Etoposide | Eposin «Nycomed Pharma» |
| Etopofos «Bristol-Myers Squibb» | |
| Vepesid «Bristol-Myers Squibb» | |

### Taxanes

| | |
|---|---|
| Paklitaxel | Taxol «Bristol-Myers Squibb» |
| Docetaxel | Taxotere «Aventis Pharm» |

### Cytotoksic antibiotics and similar substances

### Aktinomycines

| | |
|---|---|
| Daktinomycine | Cosmegen «MSD» |

### Antracyclines and similar substances

| | |
|---|---|
| Doksorubicine | Adriamycin «Pharmacia» |
| | Caelyx «Schering-Plough» |
| | Doxorubicin «Nycomed Parma» |
| Epirubicine | Farmorubicin «Pharmacia» |
| Mitoksantrone | Novantrone «Wyeth Lederle» |

### Other cytotoxic antibiotics

| | |
|---|---|
| Bleomycin | Bleomycin «Asta Medica» |
| Plikamycin | Mithracin «Pfizer» |
| Mitomycin | Mutamycin «Bristol-Myers Squibb» |

### Other anti-neoplastic drugs

### Compounds containing Platina

| | |
|---|---|
| Cisplatin | Cisplatin «Nycomed Pharma» |
| | Plastin «Pharmacia» |
| Carboplatine | Carboplatin «Faulding» |
| | Carboplatin «Pharmacia» |
| | Carbosin «Nycomed Pharma» |
| | Paraplatin «Bristol-Myers Squibb» |

### Other antineoplastic drugs

| | |
|---|---|
| Altretamine | Hexalen «MedImmume» |
| Estramustine | Estracyt «Pharmacia» |
| Topotecane | Hycamtin «GlaxoSmithKline» |
| Irinotecane | Campto «Aventis Pharma» |
| Verteporfine | Visudyne «Novartis» |

The non-steroid anti-inflammatory or anti-rheumatic drugs have in several instances been found to protect against the development of malignant tumours.

The inventor expects that in many instances members of the group that possess such activity will also show clonal inhibition with collocation inhibition since the toxicity usually is low.

When testing, it would be natural to include test for inhibition of metastatic migration. Such knowledge would probably be very important when treating patients, especially those with malignant diseases.

### Anti-inflammatory and anti-rheumatic drugs except steroids

### Derivatives of acetic acid and similar substances

| | |
|---|---|
| Indometacin | Confortid «Alpharma» |
| | Indocid «MSD» |
| Sulindak | Clinoril «MSD» |
| Diklofenak | Cataflam «Novartis» |
| | Diclofenac «ratiopharm» |
| | Modifenac «Alpharma» |
| | Voltaren «Novartis» |
| Ketorolac | Toradol «Roche» |
| Aceklofenak | Barcan «UCB» |
| Diklofenak, in combinations | Arthrotec «Pharmacia» (+ misoprostol) |

### Oxicames

| | |
|---|---|
| Piroksikam | Brexidol «Nycomed Pharma» |
| | Felden «Pfizer» |
| | Pirox «Alpharma» |
| | Piroxicam «NM Pharma» |
| | Tetram «Nycomed Pharma» |
| Meloxikam | Mobic «Boehringer Ingelheim» |

### Derivates of Propionic acid

| | |
|---|---|
| Ibuprofene | Brufen «Abbott» |
| | Brufen Retard «Abbott» |
| | Ibumetin «Nycomed Pharma» |
| | Ibuprofen «ratiopharma» |
| | Ibux «Weifa» |
| Narproxene | Alpoxen «Alpharma» |
| | Ledox «Weifa» |
| | Napren «Nycomed Pharma" |
| | Napren-E «Nycomed Pharma» |
| | Naprosyn «Roche» |
| | Naprosyn Entero «Roche») |
| | Naproxen "NM Pharma" |
| | Naproxen-E «NM Pharma» |
| Ketoprofene | Ketoprofen NM Pharma |
| | Orudis «Aventis Pharma» |
| Coxibes | |
| Celecoxib | Celebra «Pfizer, Pharmacia» |
| Rofecoxib | Vioxx «MSD» tabl. |

### Other anti-inflammatory and anti-rheumatic drugs, none- steroids

| | |
|---|---|
| Nabumetone | Relifex «SB» |
| Butazones | Phenylbutazone |
| | Oxyphenylbutazone |

### Derived from salicylic acid

| | |
|---|---|
| Pyrazolones | Many |
| Anilides | Many |

### Experimental number, problem, result, pages: T=text, L=legends, F=figures:

| No | About | Result |
|---|---|---|
| 1 | Polyoma transformed and normal parental BHK21/c13 cells seeded in 0.8%-1.2% agars | 0.8% agars supported growth of transformed cells. None supported the parental line. |
| 2 | 500-3000nM 4-OH-OPB to polyoma transformed BHK21/c13 cells | Even 500 nM stopped clonal growth |
| 3 | Stimulation of BHK21/c13 by conditioned medium, insulin and Mito+ in 0.8% agars | All of these stimulated clonal growth in agar. |
| 4 | 3 µM 4-OH-OPB to polyoma transformed BHK21/c13 cells | Completely stopped clonal growth in agar |
| 5 | Trying to evaluate the effect of 4-OH-OPB on normal contra transformed cells; crowded contra sparsely seeded surface cultures, toxic effects and therapeutic window. | Transformed and sparsely seeded cells are more susceptible. Toxic effect on especially crowded normal cells (the body) is especially low. Therapeutic window seems to be wide. |
| 6 | Analogous to the experiment no 4 but higher cell concentrations were seeded in agar in stead of on surface. | Basically the same findings as in experiment no 4. An extra observation: after the single cells formed colonies, they were not susceptible any more. One exception: transformed cells attacked by large doses of 4-OH-OPB. |
| 7 | Two adult male mice (NMRI/Bom) were transplanted with 100000 Ehrlich ascites cells intraperitonally | One got 2 weekly i.p. injections with 1.6 µl 20 mM 4-OH-OPB in 1 ml RPMI and showed no sign of tumour after 19 days when the untreated control was moribund with ascites and solid tumour in abdominal wall. |
| 8 | Testing the smallest inhibiting dose of 4-OH-OPB in mice with i.p. transplanted Ehrlich ascites tested in 4 mice and 1 control. The initiation time of treatment also evaluated in 2 mice + control. | The smallest intraperitoneal inhibiting dose was 4.8 µM. |
| | | The treatment had to start immediately for complete rescue. |
| 9 | Testing effect of i.p. injection of 4-OH-OPB on Ehrlich transplanted subcutaneously | Found inhibition on metastatic migration without inhibition by collocated cells. |
| | | |

| No | About | Results |
|---|---|---|
| 10 | Testing the effect of 4-OH-OPB on clonability of cells producing lytic anti-sheep red cells in the spleen. | Moderate to relatively high doses of 4-OH-OPB significantly reduced the number of antibody producing cells after immunisation. |
| 11a, 11b | Testing the effect of 4-OH-OPB on the replication of HSV1 in RK13-cells. | There was a significant inhibition for doses of 4-OH-OPB that were between 1 and 10.5 µM. |
| 12 | Testing the effect of 4-OH-OPB on the replication of HSV2 in RK13-cells. | There was a significant inhibition for doses of 4-OH-OPB that were between 1 and 10.5 µM. |
| 13 | Testing the effect of 4-OH-OPB and Colchicine on a density gradient of BHK21/c13 S100T1 cells in soft agar medium | The addition of Colchicine and 4-OH-OPB is only effective in stopping growth in areas of sparsely seeded cells. In the same picture both inhibition and collocation inhibition is demonstrated, showing that the effect is not dependent on the concentration of the added inhibitor, but on the local cell density. This phenomenon is probably related to the shown inhibition of the export of metastatic cells from a location rich in tumour cells and may be a good screening test for the effect of unknown test substances on the development of metastases. |
| | | |

| No | About | Results |
|---|---|---|
| 14 | The effect of cell density on clonal growth of MT4 cells inhibited by 4-OR-OPB | MT-4 cell cultures were seeded with 5, 50, 500, 5000, 50000 or 500000 cells. The growth was inhibited by 0.1-10 µM 4-OH-OPB and photographed the second day. Only wells seeded with 5000 cells or more showed growth. The inhibitory effect of 4-OH-OPB was much more pronounced on sparsely seeded cells than on cultures containing many cells. |
| 15 | Screening NSAIDs or potential drugs for anti-clonal effect using polyoma virus transformed BHK21/c13 cells, the S100T1 growing in agar medium. | Ibuprofen, Naproxen and Acetyl salicylic acid showed possible clonal inhibition at low concentration that might indicate anti-cancer activity. |
| | | Diclofenak, however, showed the opposite: increased clonal growth at low concentration indicating a possible carcinogenic activity. Podophyllotoxin probably inhibited, but only when using high dose. Piroxicam inhibited less, but similarly |
| 16 | Testing drugs or potential drugs for anti-clonal effect using polyoma virus transformed BHK21/c13 cells, the line S100T1 growing in soft agar medium. | 4-OH-OPB inhibited clones without escaping colonies. Colchicine probably inhibited significantly, but less if stored frozen for 8 days. More than 0.065 µM Podophyllotoxin inhibited clones significantly. |
| | | Ibuprofen and Naproxen possibly inhibited and Acetyl salicylic acid probably inhibited cloning in concentrations thought to be equivalent to 0.8, 0.5 and 1 grams respectively (body weight 70 kg). But this inhibition was not as effective as low dose 4-OH-OPB. |
| | | Results with high concentrations of Ibuprofen and Acetyl salicylic acid could not be accepted since the concentration of the solvent DMSO became too high. |
| | | |

| Experiment no | About | Results |
|---|---|---|
| 17 | Verifying observations of clone inhibition and testing Etoposide, a derivate of Podophyllotoxin, in the same system. | None of the tested inhibitors (Colchicine, Podophyllotoxin, or Etoposide) did inhibit clonal cell growth as effectively as 4-OH-OPB. All three seemed to allow the growth of a few small clones in sparsely seeded locations of the agar. Etoposide seemed to induce the best inhibition of cells in crowded areas. Therefore, this might be the best candidate for combination with 4-OH-OPB during short periods of treatment. Colchicine is known to be tolerated during longer periods, an advantage if needed in treatment. |
| | | Long time toxicity of Podophyllotoxin and Etoposide is known to be high and is in agreement with their significant inhibition of growth in crowded areas. |
| 18 | Mice transplanted with Ehrlich ascites tumour was treated with suboptimal dose of 4-OH-OPB alone or in combination with Etoposide the day after transplantation. Colchicine was tried in high dose along with the transplanted cells | The mice receiving suboptimal 4-OH-OPB alone or in combination with Etoposide were not rescued. Colchicine alone in high dose did not rescue the animal either. The conclusion is that up to now only 4-OH-OPB has been shown to rescue mice from transplanted Ehrlich tumour. |
| 19 | Mycophenolic acid and Cyclophosphamide were tested in soft agar cell culture studying their effect on clonal growth | No inhibition of clonal cell growth was obtained, but cyclophosphamide stimulated growth both in high and low density areas. Since the drug must be activated in liver, the outcome in the body will probably be different. However, if the activation is impaired, the effect might harm the patient. |
| | | |

| Experiment no | About | Results |
|---|---|---|
| 20 | Cell number and 4-OH-OPB was cross titrated | Low cell numbers, 42000 cells/ml gave no growth. 125000 - 375000 cells/ml gave no growth in treated wells where cells were sparsely seeded. However, in densely seeded areas of the agar culture there was significant growth, especially when the inhibitor was given in low dose. The ability not to grow if only few similar cells are collocated probably protects against cancer. 4-OH-OPB seems to be able to assist the organism in such protection. |
| 21 | Testing Actinomycin, Cytarabin and Fluorouracil for ability to inhibit clonal growth in culture. | There was signs of good, but not complete inhibition of Actinomycin. Cytarabin showed incomplete inhibition and signs of toxicity. Fluorouracil showed moderate inhibition of clonal growth and low toxicity independent of dosage. |
| 22 | Number 4,6 and 7 among 11 analogues of 4-OH-OPB were tested for clonal inhibition in cell culture. | Only #6 showed clonal inhibition at a concentration that seemed non-toxic to cells in crowded areas. |
| 23 | Number 1-3 among 11 analogues of 4-OH-OPB were tested for clonal inhibition in cell culture. | # 1 and #2 showed clonal inhibition at a concentration that seemed non-toxic to cells in crowded areas. |
| 24 | Number 5, 8 and 9 among 11 analogues of 4-OH-OPB were tested for clonal inhibition in cell culture. | None showed clonal inhibition at a concentration that seemed non-toxic to cells in crowded areas. |
| | | |

| Experiment no | About | Results |
|---|---|---|
| 25 | Number 10 and 11 among 11 analogues of 4-OH-OPB were tested for clonal inhibition in cell culture. | # 10 showed clonal inhibition at a concentration that seemed non-toxic to cells in crowded areas. |
| 26 | Benzo(a)pyrene, Altretamine and Sulindac were tested for clonal inhibition in cell culture. | Benzo(a)pyrene and Sulindac did increase the clonal growth. For Sulindac increase of dose abrogated this effect. It is considered logic that Benzo(a)pyrene, a known carcinogen, is demonstrating an effect opposite the effect of 4-OH-OPB since 4-OH-OPB can stop the growth of the transplantable Ehrlich carcinoma in mice. Altretamine had no effect. |

### Experiment number 1

### The consentration of soft agar that supports growth of the normal cell line BHK21/c13

The agar concentration that supported growth of normal and polyoma virus transformed baby hamster kidney cells has been tested. It was important to find the agar concentration that supported the growth of polyoma virus transformed baby hamster kidney cells, but not the normal parental cell line (BHK21/c13).

There is no significant growth of the normal BHK21/c13 cells in agar 5 days after seeding 600000 cells in 0.3 ml soft top agarose layer (Sigma type IX ultra-low gelling temperature) in wells of Falcon 24 wells tissue culture plate. The bottom layer consisted of 0.6 ml of 1.65% of the same agarose. The polyoma virus transformed BHK21/c13, however, showed clonal growth in 0.8% agarose, not in 1.2% (Fig. 1). Growth in soft agar is in accordance with the findings of MacPherson I and Montagnier L (1964, Virology 23, 291-294).

### Experiment number 2:

### Dose response of 4-OH-OPB as inhibitor of clonal growth of BHK21/c13 polyoma virus transformed clone S100T1 in agar culture.

About 400000 cells of the BHK21/c13 polyoma virus transformed clone S100T1 were seeded in 50 ml Falcon centrifuge tube with 1 ml medium and 1.5% Sigma type IX, ultra-low gelling temperature agarose and RPMI 1640 with 2.8% FBS and 0.22% Mito+

No top medium was added.

4-OH-OPB: Final concentrations were from 500 to 3000 nM.

After seeding and mixing, the tubes were placed in the refrigerator for about 1 hour to consolidate the ultra low melting point agarose.

Photos were taken after 3 days in the incubator (experiment 2, Fig. 1).

**Experiment 2, Table 1: Results after 3 days:**

| | No 4-OH-OPB | 500 nM 4-OH-OPB | 1000 nM 4-OH-OPB | 1500 nM 4-OH-OPB | 2000 nM 4-OH-OPB | 3000 nM 4-OH-OPB |
|---|---|---|---|---|---|---|
| 0-control: | No growth | | | | | |
| Growth: | Significant growth | Growth inhibited | Growth inhibited | Growth inhibited | Growth inhibited | Growth inhibited |

### Conclusion:

*This experiment showed clearly an inhibition of clonal growth when adding 4-OH-OPB at concentrations of 500 nM or higher. Earlier 300 nMshowed no inhibition. The growth of the untreated control was significant, but small. The reason might be the relative high agar concentration in this experiment.*

### Experiments number 3:

### Growth of the normal baby hamster kidney cells, BHK21/c13, in agar medium with conditioned medium, insulin or the serum extender Mito+.

This experiment used BHK21/c13 cells grown in the Falcon plates with 24 wells. Each well received 120000 cells seeded in a top layer of 0.3 ml with 0.8% ultra low gelling temperature agarose (type IX, Sigma). This layer was placed upon a 0.3 ml bottom layer with 3% of the same agarose. The medium contained Eagles MEM and RPMI 1640 with 10% foetal bovine serum. The medium was supplemented with either: 15% conditioned medium (from BHK21/c13 cultures), the serum extender Mito+ or 0.1 i.u. Insulin Actrapid (Novo) or with two or all three of these in combination.

**Experiment 3, Table 1: Clonal growth of normal baby hamster kidney cell line, BHK21/c13:**

| | As described in left column | Control without insulin ↓ |
|---|---|---|
| Insulin 0.1 iu/ml + conditioned medium | ++ | ++ |
| Insulin 0.1 iu/ml, Mito+, and conditioned medium | ++ | ++ |
| Insulin 0.1 iu/ml | ++ | +- |
| Insulin 0.1 iu/ml and Mito+ | ++ | ++ |

### Results and conclusion:

*There is observed growth in all wells containing 1, 2 or 3 of the tested growth promoters. Only the well with nothing except serum in Eagles MEM showed no growth (Table 1,* *Fig. 1**). That indicates that normal BHK21*/*C13-cells will not grow in agar if there is not any extra growth stimulantpresent. That an analogue soft agar medium did not support growth of normal cells was first reported by* MacPherson I and Montagnier L (1964) Virology 23, 291-294*). The effect of conditioned medium and insulin has also been reported earlier (*Tjøtta E., Flikke M. and O. Lahelle (1967) Arch. ges. Virusforsh. 23, 288-291 *and* Tjøtta E. (1968) Arch. ges. Virusforsh. 25, 363-364*), but was now adapted to this system.*

### Experiment number 4:

### The compound 4-OH-OPB tested for possible clonal inhibition of cells seeded in soft agar.

120000 S100T1 cells of a polyoma virus transformed BHK21/c13 cell line, were seeded in 1 ml soft agar medium in Falcon 50 ml centrifuge tubes. This layer contained 1 ml 1.1 % Ultra-low gelling temperature agarose (Sigma, Type IX). On the top of the agar layer 0.5 ml fluid medium was added. It contained conditioned medium from BHK21/C13 cultures, 2.8% foetal calf serum and 0.22% of the serum extender Mito+. Both media contained Eagles MEM, the agar medium also RPMI 1640.

result: The results are seen in Fig. 1, experiment 4 and summarized in Table 1:

**Experiment 4, Table 1:**

| | |
|---|---|
| 4-OH-OPB added | Result day7 |
| 0-control (at 5°C) | no growth |
| No addition | Big colonies |
| 3µM 4-OH-OPB | Single cells only |

### Conclusion:

*Even after 7 days no growth was observed in cultures treated with 3µM 4-OH-OPB. It seemed to cause a perfect clonal inhibition.*

### Experiment number 5:

### Surface growth of BHK21/c13 (normal baby hamster kidney cell line) and its polyoma-transformed offspring, S100T1. Response on different doses of 4-OH-OPB.

The degree of confluence of polyoma virus transformed BHK21/c13 cells, the S100T1 cell strain, growing on plastic surface was observed 2-4 days after seeding 3000 or 10000 cells in appropriate wells of a 96 well tissue culture plate (Nunc). The dose range of 4-OH-OPB is indicated in Fig. 1-4.

### Conclusion:

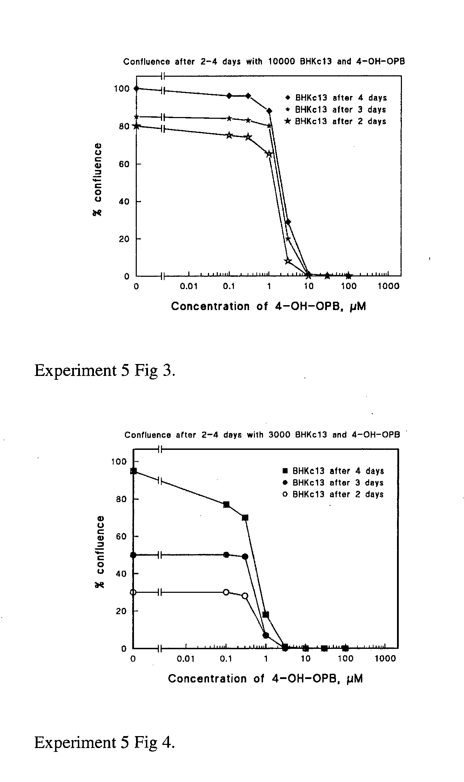

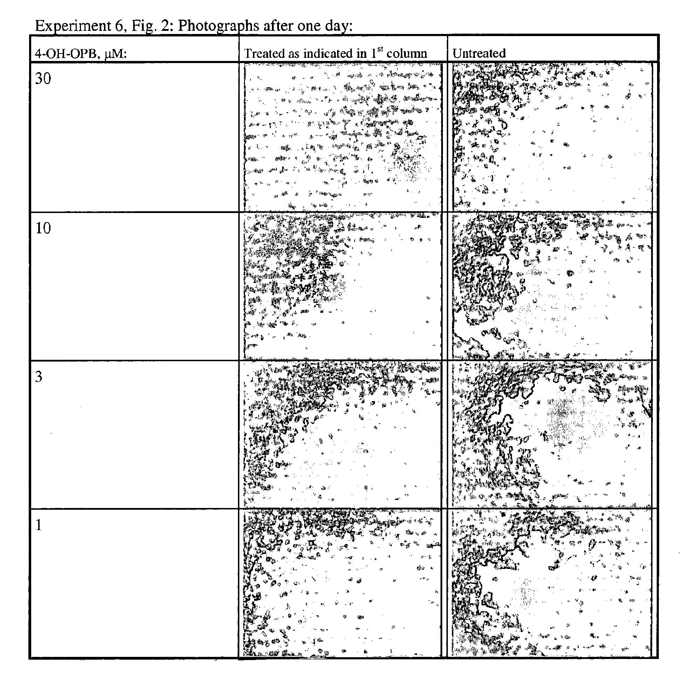

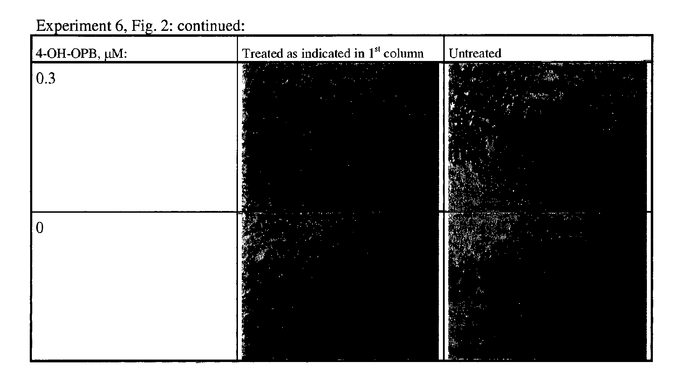

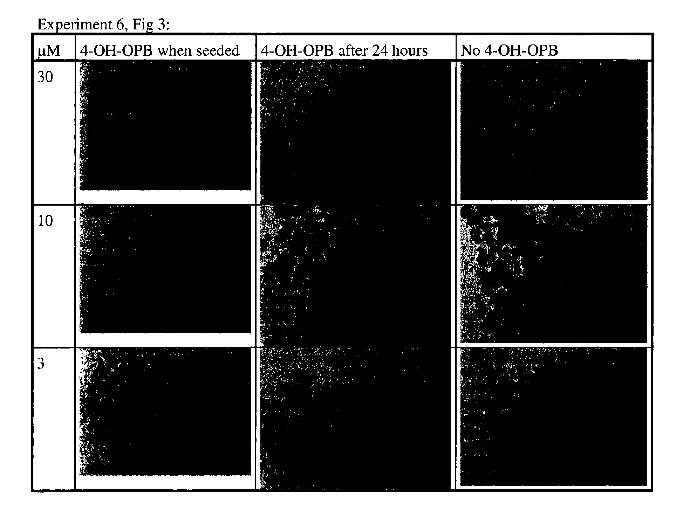

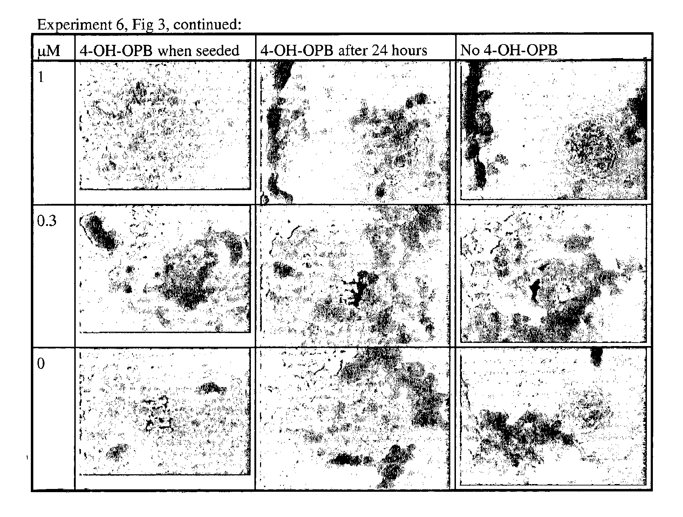

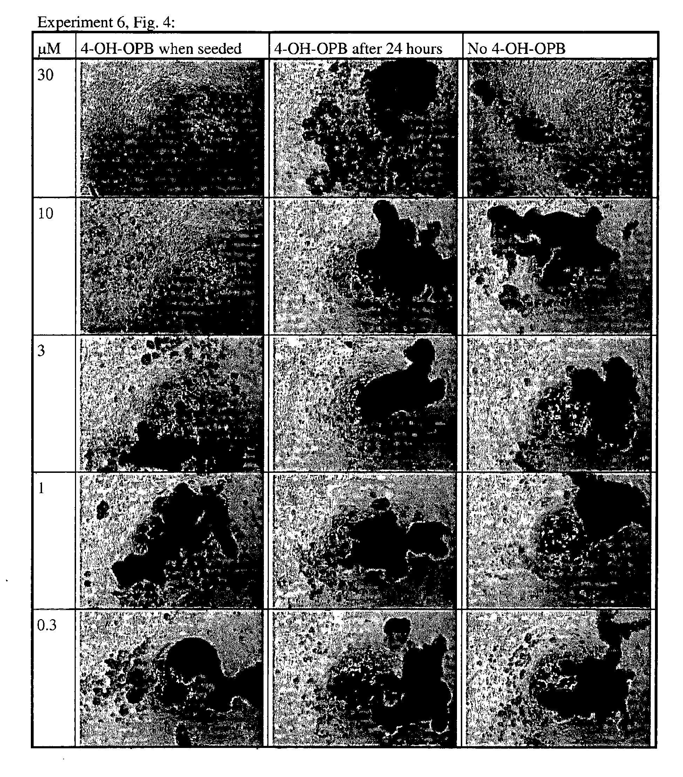

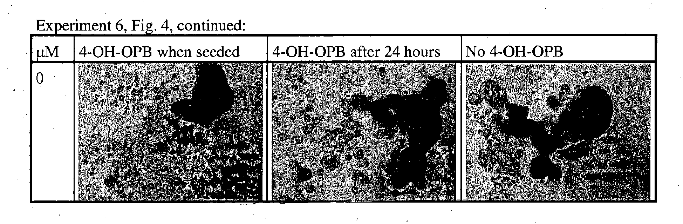

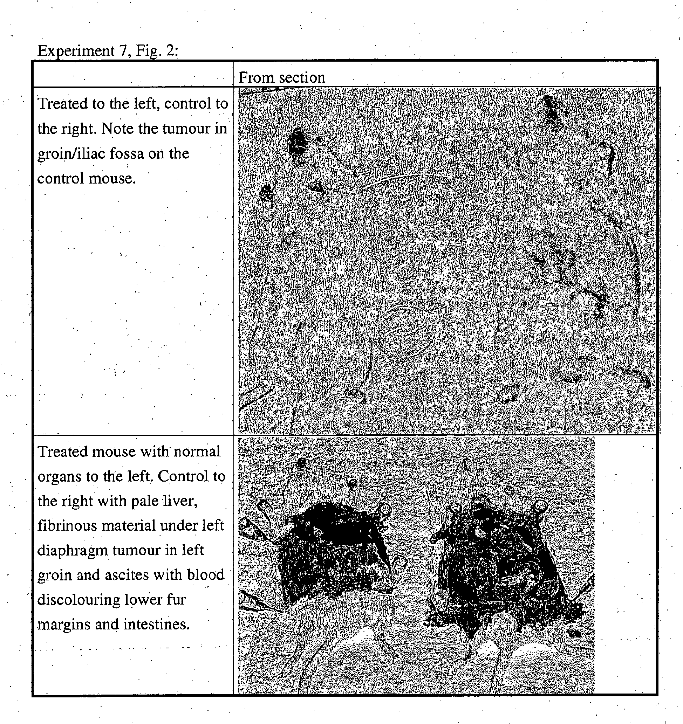

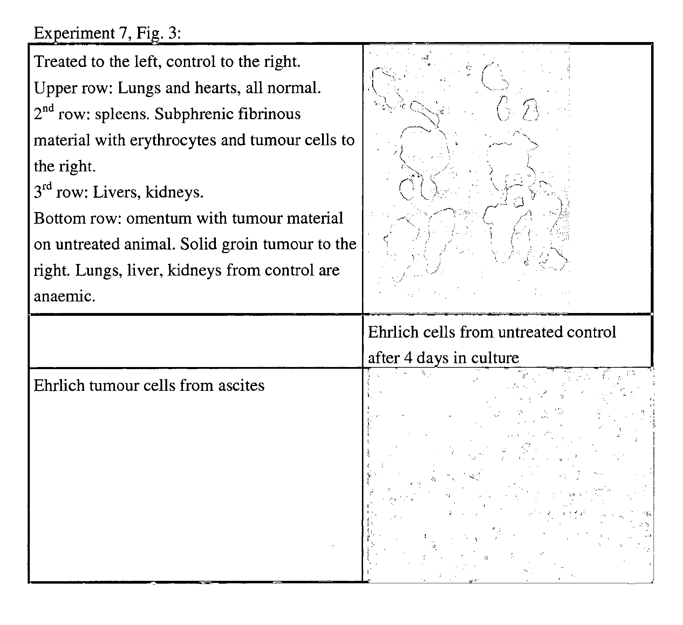

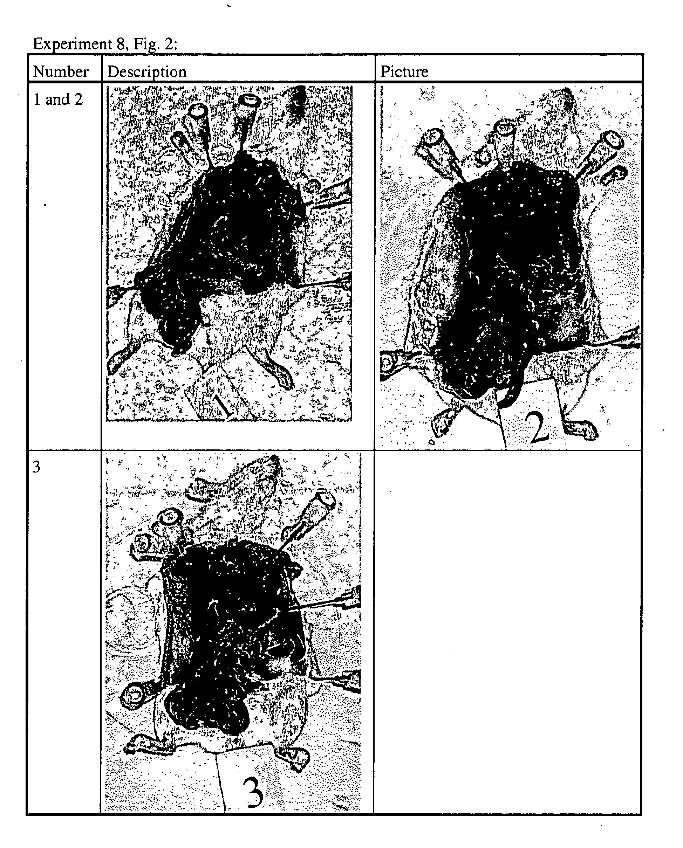

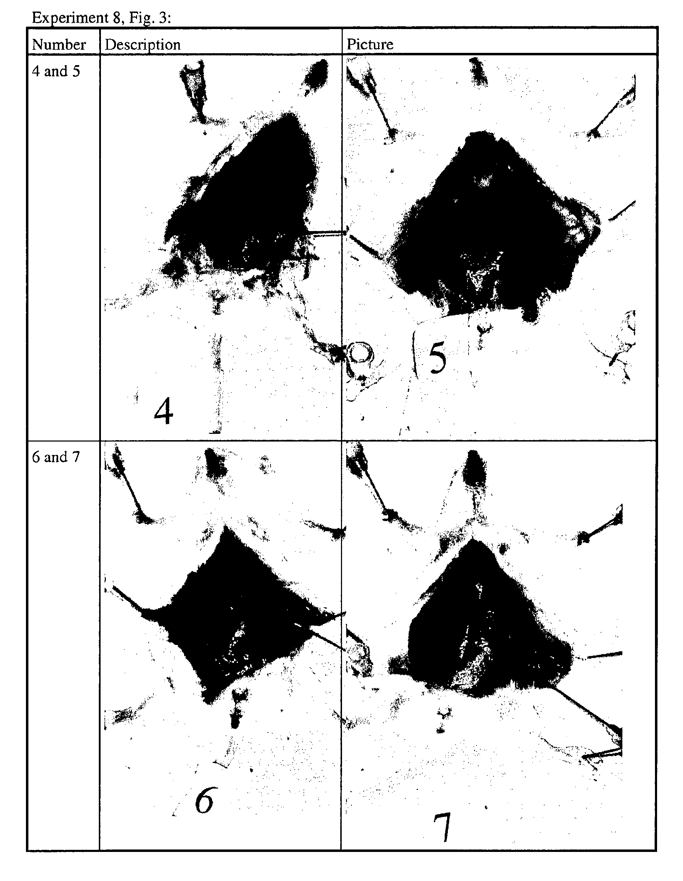

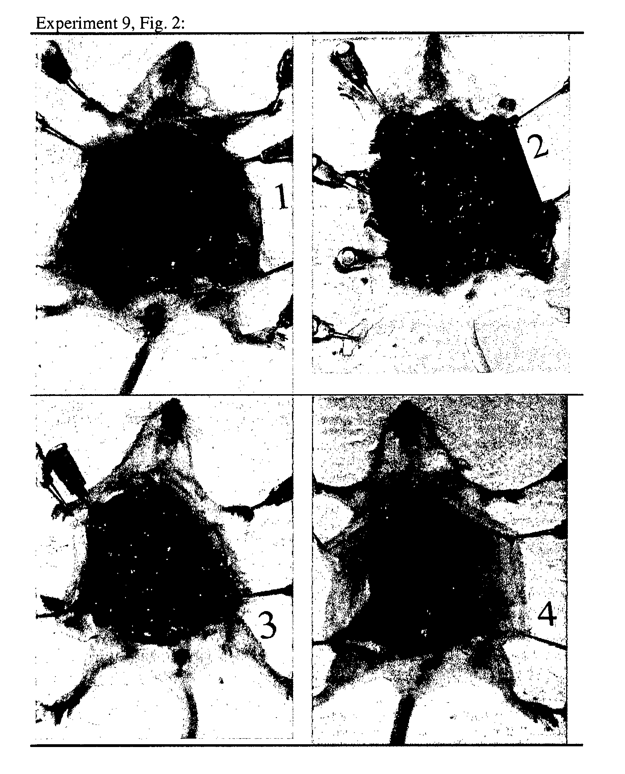

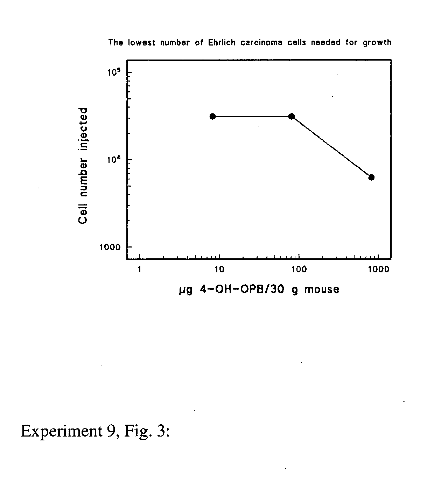

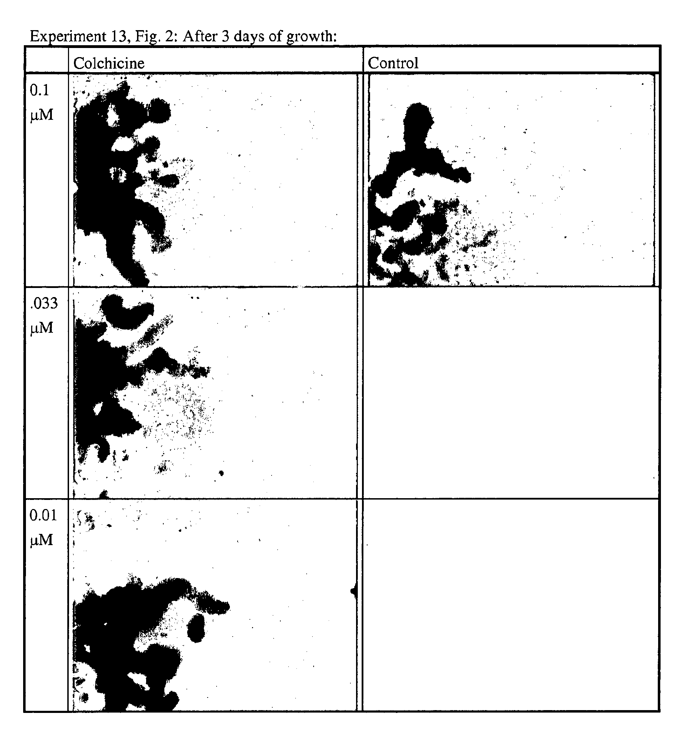

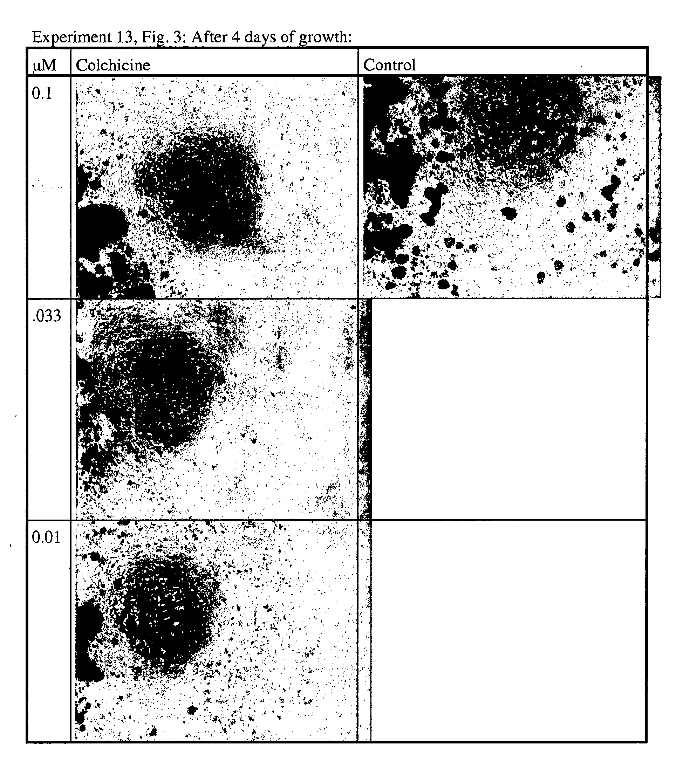

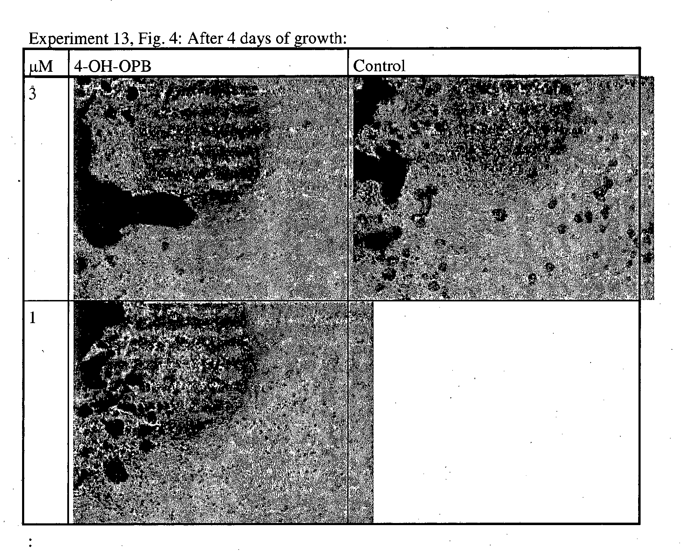

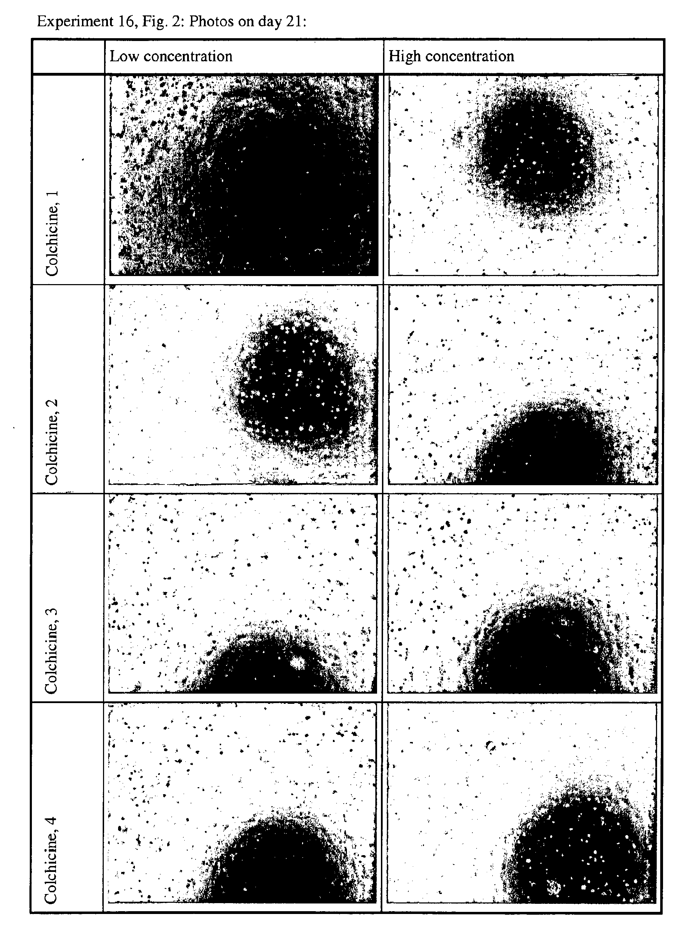

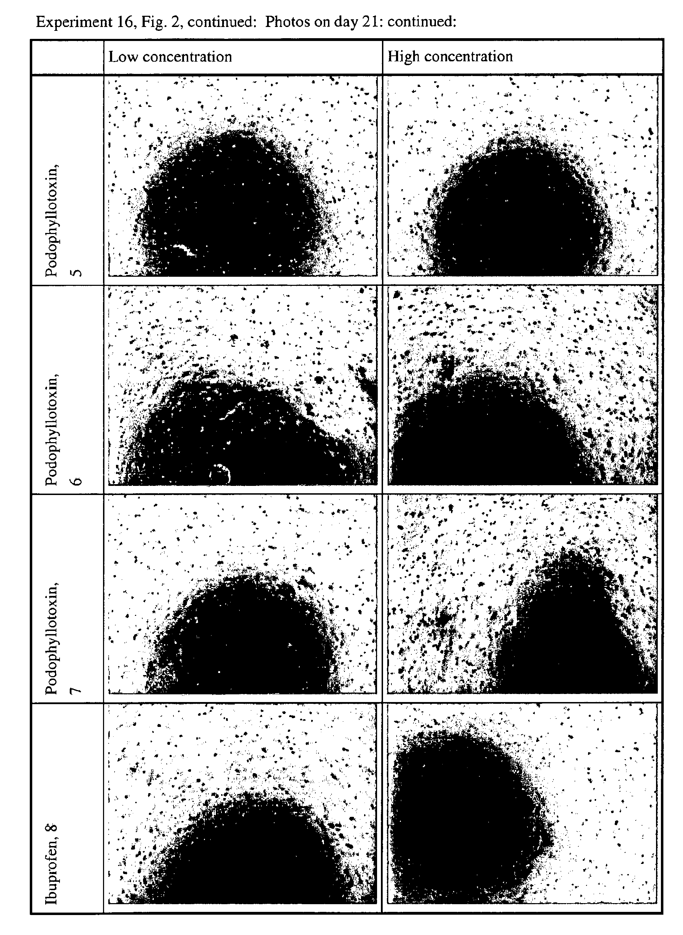

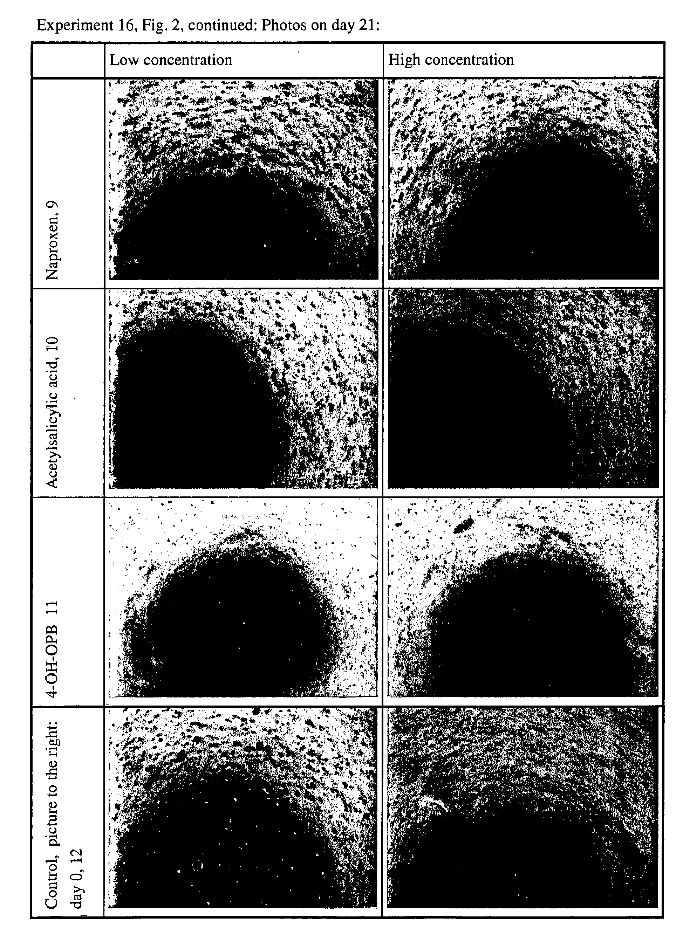

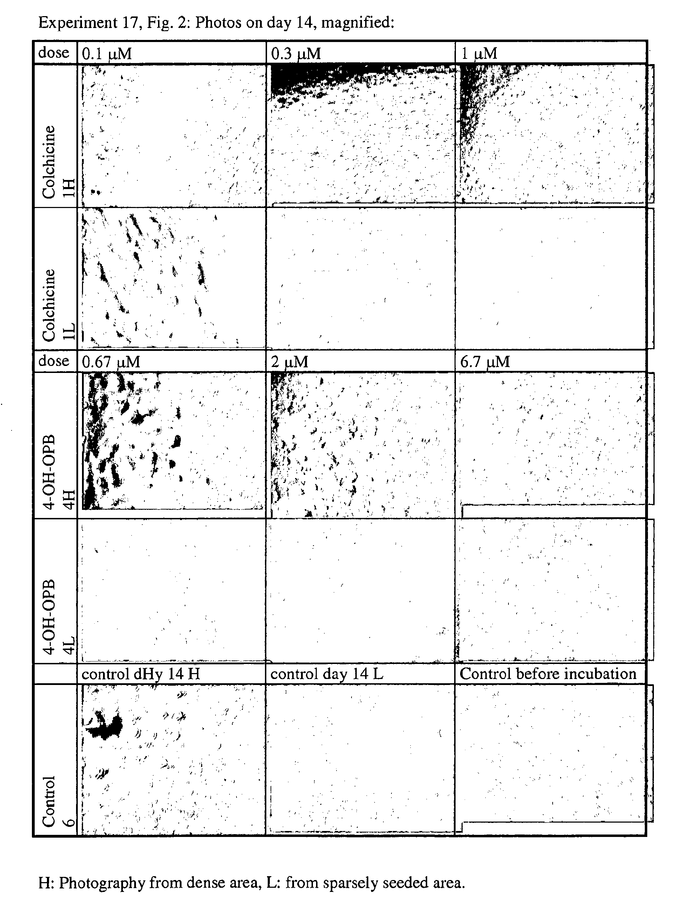

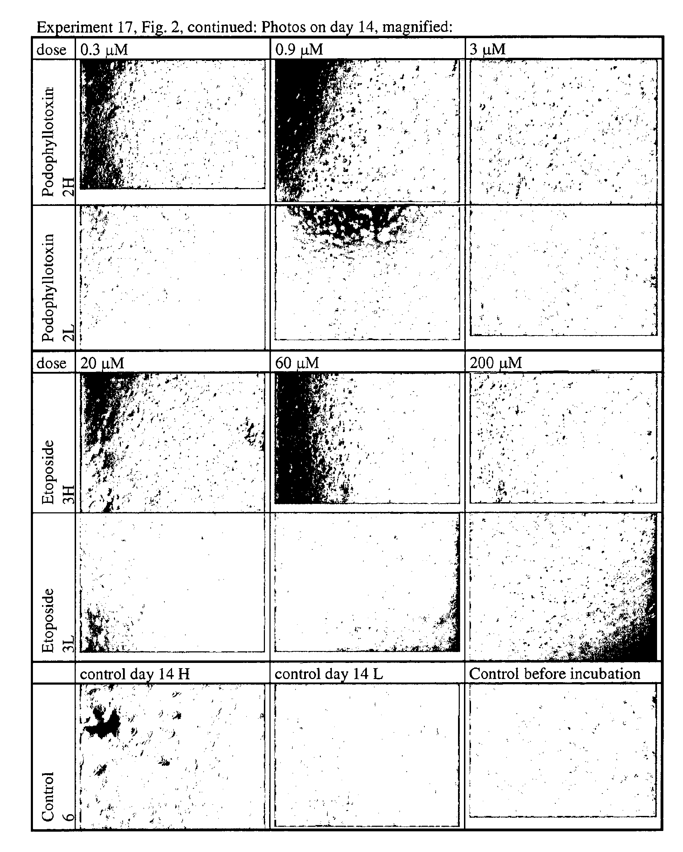

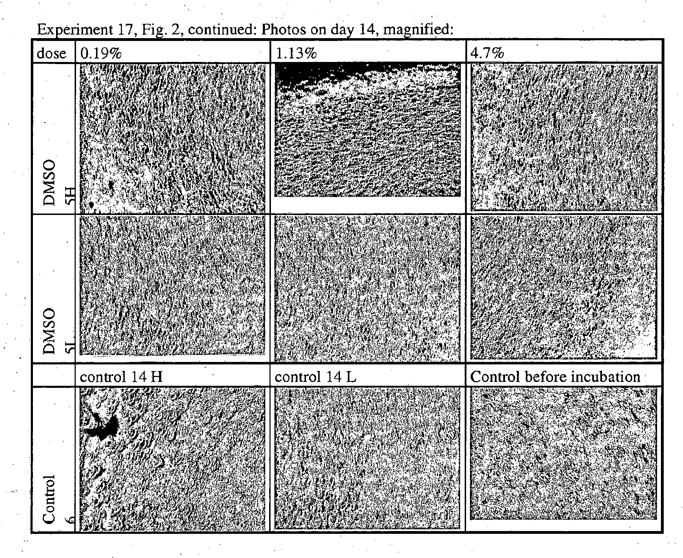

*As shown in the pictures, about 3 times higher concentration of 4-OH-OPB is needed for total inhibition of the growth on plastic surface of the normal baby hamster kidney cell line (BHK21*/*c13) (**Fig. 3 and 4**) compared to the polyoma transformed offspring (**Fig. 1* *and* *2**).*

*The experiments also show that cell density when starting is important for the effect of 4-OH-OPB. Shortening the distance to neighbour cells seems to increase the growth potential of clones and to decrease the effect of the clonal inhibitor 4-OH-OPB (**Fig. 1* *and* *3**). If this were true, cell cloning with other cells of the same kind far away would probably be inhibited most.*

This is in accordance with our animal experiments that show complete inhibition of Ehrlich ascites transplanted in the peritoneum of mice if the mice were treated at the time of transplantation. Treatment beginning the next day or 3 days after transplantation, when the cells have had a chance to multiple, did not cure the malignant disease even with doses about 9 times higher than the minimum dose that was necessary for the cure of the tumour when starting immediately (see experiment number 8).

Normal cells were inhibited less by 4-OH-OPB than the transformed ones. Growth inhibition by neighbour cells is supposed to be less for transformed cells than for normal cell lines, but if this might be the reason for greater 4-OH-OPB sensitivity of Transformed cells remains to be shown.

### Experiment number 6:

### Clonal growth depression in soft agar of BHK21/c13 (normal baby hamster kidney cells) stimulated by insulin (Novo Actrapid) and of S100T1 (polyoma-transformed offspring) as response to 4-OH-OPB dose.

The aim of this experiment was to see if the addition of 4-OH-OPB acted differently when added after the seeding of cells in agar compared to immediate treatment.

Agar cultures were prepared in wells of 24 well Falcon tissue culture plate. Each contained two agar layers of 0.3 ml. The bottom layer contained 1.75% Sigma agarose, type II, A-6877 and the top layer 120000 cells in 0.8% ultra low gelling temperature Sigma agarose type IX, A-5030. The medium contained about equal amounts of Eagle and RPMI 1640 with 10% FBS. Insulin (Actrapid, Novo) was added in a concentration of 0.1 i.u./ml to BHK21/c13 cell cultures only.

Summary of results as demonstrated in photographs of experiment 6, Fig. 1-4:

**Experiment 6, Table 1:**

| 4-OH-OPB, µM | S100T1, 1* | S100T1, 2* | S100T1, 3* | BHK21/c13, 1* | BHK21/c13, 2* | BHK21/c13, 3* |
|---|---|---|---|---|---|---|
| 30 | +++^{#} | +++ | + | +++ | ++ | 0 |
| 10 | +++ | + | 0 | + | + | 0 |
| 3 | ++ | +- | 0 | 0 | 0 | 0 |
| 1 | + | 0 | 0 | 0 | 0 | 0 |
| 0.3 | 0 | 0 | 0 | 0 | 0 | 0 |
| 0 | 0 | 0 | 0 | 0 | 0 | 0 |

| | | | | | | |
|---|---|---|---|---|---|---|
| *: 4-OH-OPB was added immediately and read after 24 hours (1). These cultures were also read after 48 hours (2) together with the set that obtained 4-OH-OPB after 24 hours (3). #: Clonal inhibition: Perfect: +++, good: ++, small: +, probably none: +-, none: 0. | | | | | | |

### Results and Conclusion:

*Appropriate wells of two 24 wells Falcon tissue culture plates obtained a solid bottom layer and a soft top agar layer containing 120000 cells each of either BHK21*/*c13 or its polyoma virus transformed offspring, S100T1.*

*Other experiments have provided evidence for reduced effect of clonal inhibition of 4-OH-OPB when the seeded cell number was high as in this experiment.*

*In a complete search for new compounds a smaller cell number should also be included in this agar test for comparison and evaluation of the effect of collocation and toxicity in agar.*

*If 4-OH-OPB treatment of S100T1 started immediately after seeding, a good clonal inhibition was obtained when the concentration of 4-OH-OPB was above 1 µM. However, a delay of treatment for 24 hours only induced a small growth inhibition when adding 30 µM 4-OH OPB, a very high dose (**Fig 1-4**, Table 1).*

*BHK21*/*c13 showed less inhibition than S100T1 cells when inhibited with the same concentrations of 4-OH-OPB. Photographs after 24 hours showed a small, but significant effect on clonal inhibition from 10 µM or higher when treated immediately on seeding. After 48 hours photographs of the cultures treated when seeded showed less effect of 10 µM 4-OH-OPB, but still an effect persisted (**Fig. 1-4**, Table 1).*

*However, if treated first after 24 hours, no effect was detected. Thus, only transformed cells showed a small effect when treated the first time 24 hours after seeding (**Fig. 3**).*

*It is evident also from this experiment that the transformed cell line S100T1 reacted better on clonal inhibition by 4-OH-OPB than the normal parent cell line BHK21*/*c13.*

*The other finding is that cells that have started to form small clones are no longer susceptible.*

*Only one exception to this rule was observed: S100T1 inhibited by 30 µM the day after seeding. At this high concentration of 4-OH-OPB also small colonies showed some inhibition ofgrowth. This may also indicate that not only single S100T1 cells can be inhibited if the 4-OH-OPB dose is high enough. But it was only the transformed cell line that showed this response, not the normal parental line.*

### Experiment number 7:

### Testing 4-OH-OPB as treatment of mouse transplanted with Ehrlich's mouse ascites.

The aim of this experiment was to see if 4-OH-OPB was able to inhibit or stop the growth of a transplantable mouse tumour, the Ehrlich ascites tumour.

The Ehrlich ascites cells stored in liquid nitrogen were thawed, washed and suspended in RPMI 1640 without FBS (pH~6.9) in a volume of 1 ml with 100000 cells for each mouse.

The experiment describes transplantation of the malignant mouse Ehrlich ascites tumour to two adult male mice (NMRI/Bom). Each of them received 100000 cells intraperitonally. One mouse was treated two times a week with 4-OH-OPB intraperitonally. After 19 days the untreated mouse was moribund (Fig. 1) and both were killed and examined (Fig. 2, experiment 7). No tumour was detected in the treated animal. The untreated mouse, however, had about 250 millions of malignant cells in the ascitic fluid and a pea/bean sized solid tumour in the abdominal wall where the needle penetrated during transplantation (Fig.3).

### Estimation of 4-OH-OPB dose:

The treated mouse was 45.5 g of weight. Estimated extracellular fluid is 45.5 g* 20/70= 13 ml. To obtain 2.5 µM of 4-OH-OPB in 13 g we need x ml of 20 mM solution of 4-OH-OPB in DMSO. Calculation: 13 ml * 2500 nM=20000000 nM * x. x=1.62 µl.

When solving 1.62 µl in 1 ml cell suspension, the concentration was

2.5 µM*13ml/1ml= 32.5 µM.

Cells were injected 90 minutes after the addition of 4-OH-OPB to the suspension. The control received the same cell suspension without 4-OH-OPB.

**Experiment 7, Table 1: Injection dates of 4-OH-OPB to treated mouse:**

| | day 0 | day 4 | day 7 | day 11 | day 14 | day 18 | day 19 |
|---|---|---|---|---|---|---|---|
| Dose | 1.62µl* | 1.62µl | 1.62µl | 1.62µl | 1.62µl | 1.62µl | Stop |
| Weight | 45.5 | | | | 42.6 | 44.3 | |
| Weight of control | 50.7 | | | | 52.1 | 52.7# | |

| | | | | | | | |
|---|---|---|---|---|---|---|---|
| *: From a 20 mM solution in DMSO, dissolved in 0.8 ml RPMI (pH~6.9) #: This mouse was now so sick that it should be killed to morrow. The other one is completely healthy clinically. | | | | | | | |

On day 19 the control mouse was still sick with extended stomach and was pale with few movements. In the left iliac fossa/left groin there was a tumour of about pea-bean size.

The treated mouse was completely normal without any palpable tumour or signs of ascites or anaemia. The mouse was very interested in escaping when the roof of the cage was lifted off.

Pictures (Fig 1-3) were taken before and after death and after opening abdomen and thorax.

The section showed no pathological signs in the treated animal. There was no fluid in the abdomen and no signs of tumours in the lungs, liver, kidneys, and spleen or elsewhere in abdomen, thorax, muscles or body surface.

The other mouse, however, had an extended abdomen full of haemorrhagic ascites (Table 2). Subphrenically and on the omentum there was lots of fibrinous material with erythrocytes and tumour cells. In the left groin/iliac fossa there was a tumour, obviously related to the point of injection of the abdomen. Size: pea/bean, lobulated, firm. Liver, kidneys and lungs were paler than in the treated animal, obviously caused by the heavy blood loss into the peritoneum. No tumour invasion of the liver, kidneys, spleen or lungs was observed (Table 2).

**Experiment 7, Table 2: Size of organs in grams:**

| | Treated animal | Control |
|---|---|---|
| Lungs | 0.3 | 0.25 |
| Hart | 0.20 | 0.157 |
| Spleen | 0.184 | 0.204 |
| Fibrinous material under diaphragm | 0 | 0.182 |
| Liver | 2.38 | 2.01 |
| Kidneys | 0.57 | 0.59 |
| Omentum | 2.36 | 3.71 (with tumour material) |
| Ascites | 0 | 2.6 ml with 234 mill. cells |
| Tumour in groin | 0 | 0.48 |

### Conclusion:

*There was no tumour or ascites observable in the mouse with transplanted Ehrlich carcinoma in peritoneum that was treated. This mouse also showed a completely normal behaviour.*

*The other one, the control that received no treatment, was anaemic, slow and moved with difficulty in the cage after 19 days. The developed ascites contained almost a quarter of a billion malignant cells and fibrinous material several places intraperitonally. In addition this mouse had a fairly big solid tumour in the groin*/*iliac fossa, the site where tumour cells were injected.*

*This experiment showed that there was a possibility that treatment with 4-OH-OPB was able to completely stopping the developntent of a malignant tumour when transplanted to peritoneum.*

*However, a final conclusion can not be drawn without more experiments.*

### Experiment number 8:

### Mouse test of 4-OH-OPB as treatment of mouse transplanted with Ehrlich's mouse ascites.

The aim of the experiment was to verify that 4-OH-OPB would stop the growth of the transplantable Ehrlich ascites tumour in mice. At the same time, dilutions of 4-OH-OPB were injected intraperitonally to the transplanted mice in order to find the smallest active dose.

The cells from ascites in the untreated control mouse was frozen on serum with 10% DMSO. The cells were thawed in water of 37°C, diluted in serum, and pelleted by low speed centrifugation after the addition of 2 ml FBS, to remove DMSO.

Each animal received such cells suspended in portions of 1 ml in RPMI (pH~6.9).

The amounts and time of injection of 4-OH-OPB are given in Table 1 and 2 of this experiment.

The results are indicated in Table 2 and 3 and in Fig. 1-3 of this experiment.

The weight of the mice was this time about 70% of the mouse that received 4-OH-OPB last time. Therefore, we reduced the cell number to about 75000 and the injected volume to 0.8 * 0.7=0.56 ml.

**Experiment 8, Table 1: Treated mice and 4-OH-OPB dosage:**

| :Number, g mouse weight | Total dose per mouse * | Time of treat ment | Day 1 | Day 4 | Day 7 | Day 8 | Day 10 |
|---|---|---|---|---|---|---|---|
| 1, 35.4 | 1.26µl | Day 0 | | 1.26µl | 1.26µl | | 1.26µl |
| 2, 39.9 | 0.42µl | " | | 0.42µl | 0.42µl | | 0.42µl |
| 3, 26.6 | 0.14µl | " | | 0.14µl | 0.14µl | | 0.14µl |
| 4, 28.1 | 0.047µl | " | | 0.047µl | 0.047µl | | 0.047µl |
| 5, 31.2 | 0 | | | 0 | 0 | | 0 |
| 6, 31.7 | 1.26 µl | Day 1 | 1.13 µl | 1.13 µl | 2.22 µl | | 1.13 µl |
| 7, 30.6 | 1.26 µl | Day 3 | | 1.09 µl | | 1.09µl. Tumour = grain | 1.09 µl. Tumour >grain |

| | | | | | | | |
|---|---|---|---|---|---|---|---|
| *: µl of 20 mM 4-OH-OPB in DMSO pr. gram mouse. If 2/7 of mouse weight was extracellular fluid with all 4-OH-OPB, the concentration in extra-cellular fluid was: 0.0356 µl 4-OH-OPB, 20 mM /(2/7) ml=0.0356 µl * 20000 µM / 285.71 µl = 2.49 µM in the mouse. The conc. in the injected 0.8 ml fluid with or without cells is: 2.49 µM * 13/0.8=40.5 µM. | | | | | | | |

**Experiment 8, Table 2: Continuation**

| Number, weight | Day 15 dose | Day 15 weight/initial weight | Day 15 | Section day 17 |
|---|---|---|---|---|
| 1, 35.4 | 1.26µl | 33.9, 0.96 | healthy | healthy, no tumour |
| 2, 39.9 | 0.42µl | 38.7, 0.97 | healthy | healthy, no tumour |
| 3, 26.6 | 0.14µl | 29.3, 1.1 | healthy | healthy, no tumour |
| 4, 28.1 | 0.047µl | 37.4, 1.33 | Ascites | 4.5 ml ascites |
| 5, 31.2 | 0 | 42.2, 1.33 | Ascites | 6.8 ml ascites |
| 6, 31.7 | 1.13 µl | 34.5, 1.09 | Healthy | 3.2 ml ascites, inj. site tumour |
| 7, 30.6 | 1.09 µl. lens-sized tumour | 30.5, 1.00 | lens-sized tumour at injection site | Pea-size tumour at inj. site, 15 µl ascites, typical cells. |

Tumour growth related to dosage and time of medication is shown in:

**Experiment 8, Table 3:**

| Number, weight in gram | 4-OH-OPB dose | Conc. intra-peritonally# | Estimated extra-cellular conc. ¤ | Time of first injection | Result |
|---|---|---|---|---|---|
| 1, 35.4 | 1.26µl*, | 42 µM | 2.5 µM | Same as for tumour cells | No tumour |
| 2, 39.9 | 0.42µl | 14 µM | 0.74 µM | " | " |
| 3, 26.6 | 0.14µl | 4.7 µM | 0.37 µM | " | " |
| 4, 28.1 | 0.047µl | 1.6 µM | 0.12 µM | " | Ascites (0.66 of contr.) + solid |
| 5, 31.2 | 0 µl | 0 µM | 0 µM | | Ascites (control) |
| 6, 31.7 | 1.13 µl | 37.7 µM | 2.5 µM | 24 h after tumour | Ascites (0.47 of contr.) + solid |
| 7, 30.6 | 1.09 µl. | 36.3 µM | 2.5 µM | 3 days after tumour | Ascites § (0.00022% of contr.) + solid |

| | | | | | |
|---|---|---|---|---|---|
| *: From a solution in DMSO with 20 mM 4-OH-OPB #: 4-OH-OPB was diluted in 0.6 ml RPMI 1640. ¤: The amount extracellular fluid was calculated as 2/7 of the weight of the mouse. §: In contrast to the other ascitic fluids, there was no blood in it. | | | | | |

### Conclusion:

*The experiment showed that it is possible to stop the development of ascites after transplanting Ehrlich ascites to mice if 4-OH-OPB was injected together with the cells and then twice a week. The estimated concentration extracellularly had to be over 120 nM, here 370 nM or more to be effective and in the peritoneum over 1.6 µM, here 4.7 µM or more to be effective. The first treatment started by injecting the 4-OH-OPB into the cell suspension 90 minutes before transplantation.*

*If the therapy started 24 hours after transplantation or later with doses in abdomen of 36-38 µM, there was development of ascites or ascites with solid tumour in addition. But the neoplastic development was possibly somewhat inhibited compared to untreated control.*

### Experiment number 9

### 4-OH-OPB as treatment of mice with subcutaneously transplanted Ehrlich's mouse ascites cells.

The aim of the experiment was to see if 4-OH-OPB might loose its activity when transported through blood from the peritoneal cavity to subcutaneously transplanted tumour cells in 6 different locations with different cell numbers (Table 1).

Ehrlich ascites was stored in liquid nitrogen on serum with 10% DMSO. The ampoules were thawed in water of 37°C, diluted in serum, and pelleted by low speed centrifugation after the addition of 2 ml FBS, to remove DMSO.

The cells were diluted in volumes of 0.6 ml RPMI (pH about 6.9) for each animal with no serum added. The 4-OH-OPB was injected intraperitonally twice a week, see Table 2.

The clinical status and resulting ascites or tumours are described in Table 3-4 and shown in Fig. 1-2. The smallest tumour cell number necessary for growth of local tumour under the influence of 4-OH-OPB is shown in Fig. 3.

Cell numbers of Ehrlich's cells injected subcutaneously in each of 4 mice:

**Experiment 9, Table 1:**

| | Right side | Left side |
|---|---|---|
| Lower thorax | 10 | 1250 |
| Upper abdomen | 50 | 6250 |
| Lower abdomen | 250 | 31250 |

**Experiment 9, Table 2: The calendar of intraperitonal 4-OH-OPB injections:**

| Number, weight | day 0 | day 3 | day 7 | day 10 | day 14 | day 18 | day 21 |
|---|---|---|---|---|---|---|---|
| 1, 26.2 | 10.5 µl,0.2M* | 10.5 µl,0.2M | 10.5 µl,0.2M | 10.5 µl,0.2M | 10.5 µl,0.2M | 0µl# 0.2M | 0µl# 0.2M |
| 2, 29.2 | 0 µl | 0 µl | 0 µl | 0 µl | 0 µl | 0 µl | 0 µl |
| 3, 24.1 | 9.6 µl 20mM* | 9.6 µl 20mM | 9.6 µl 20mM | 9.6 µl 20mM | 9.6 µl 20mM | 9.6 µl 20mM | 9.6 µl 20mM |
| 4, 34.2 | 1.37 µl 20 mM | 1.37 µl 20 mM | 1.37 µl 20 mM | 1.37 µl 20 mM | 1.37 µl 20 mM | 1.37 µl 20 mM | 1.37 µl 20 mM |

| | | | | | | | |
|---|---|---|---|---|---|---|---|
| *: The amount and concentration of 4-OH-OPB solution in DMSO that was used for treatment after having been solved in 0.6 ml RPMI pH~6.9. #: The animal was too sick to receive treatment | | | | | | | |

**Experiment 9, Table 3: Status:**

| Number, weight | Total pr. mouse, µl 4-OH-OPB | Day 3-7, clinical status: | Clinical status, day 10 | Clinical status, day 14 | Clinical status, day 18 |
|---|---|---|---|---|---|
| 1,26.2 | 10.5 µl,0.2M | no tumour | Few drops from peritoneum | No Ehrlich cells in abdomen | 37.4 g, ascites, but no Ehrlich cells (Fig. 1) |
| 2,29.2 | 0 µl | " | No tumour | Ascites, 28.5 | Ascites |
| 3,24.1 | 9.6 µl 20mM | " | " | No tumour | No tumour |
| 4,34.2 | 1.37 µl 20 mM | " | " | " | " |

**Experiment 9, Table 4: Results:**

| Number, initial weight: | Extra-cell. conc. # | Day 21, pathology: | Experiment terminated after 24 days. Animal weight and pathology: |
|---|---|---|---|
| 1, 26.2 | 281 | Ascites | 40.2 g. Toxic ascites and thoracic effusion *. Sites injected with highest and next highest cell number developed solid tumours. |
| 2, 29.2 | 0 | " | 42.9 g. Ascites with Ehrlich cells |
| 3, 24.1 | 27.9 | Lens sized tumour in left lower abdomen | 25 g. Pea sized tumour in left lower abdominal wall. |
| 4, 34.2 | 2.8 | Small tumour in left lower abdomen. | 33.5 g. Tumour as discovered 3 days ago, now lens sized. |

| | | | |
|---|---|---|---|
| #: Estimated conc. if all 4-OH-OPB is solved in the extracellular compartment. *: No malignant cells were found in these compartments. The fluid showed hemolysis because the animal was frozen and thawed before examination. However, no Ehrlich carcinoma cells were found in the effusions of this mouse. Also fluid examined under microscope before death contained no Ehrlich carcinoma cells (day 14). | | | |

Number 2, the untreated control, was injected with the same number of tumour cells, but had many malignant cells in the fluid (see Fig. 1).

### Conclusion:

*The experiment was designed to see if 4-OH-OPB injected intraperitonally in mice could be transported through blood to transplantable tumour cells injected subcutaneously.*

*The dosage of the injected candidate drug 4-OH-OPB was very high in one mouse. Organs close to peritoneum as liver, spleen and possibly kidneys became affected by its toxicity. The organs in thorax were not affected macroscopically, but the animal died of haemorrhagic effusion in thorax, probably as a result of the same toxic reaction as produced similar effusion in the peritoneum.*

*However, this mouse was the only mouse with two tumours of the abdominal wall at the sites where 6250 and 31250 Ehrlich ascites cells had been injected.*

*The two mice receiving smaller doses had only one tumour and the control without any 4-OH-OPB had none such tumour at the injection sites.*

*The cause of this might be that the growth of solid tumours, but not ascites was stimulated by the treatment. The mature of this "stimulation" is discussed below.*

*The only animal getting true Ehrlich ascites was the control, the only one without medication. None of the treated ones got ascites.*

*Since the control showed development of ascites even after no cells having been injected in peritoneum, the cells must have been transported to peritoneum from the injection sites, probably by the circulation. That might indicate that when giving the animals 4-OH-OPB it reduced the export of Ehrlich cells from the injection sites to peritoneum. If this is true, a reduced export of cells would increase the number of cells in the primary injection site compared to untreated animals. If the local cell numbers reaches values above the limit necessary for development of local tumours, solid local tumours would develop. In addition the collocated tumour cells at the injection sites would inhibit the action of 4-OH-OPB if local density is high enough.*

*This experiment showed that 4-OH-OPB probably possessed a second ability in addition to inhibit cloning. This additional effect was an inhibition of metastatic export of injected malignant cells from the site of injection to the site where the tumour cells obviously preferred to grow, the peritoneum. Moreover, this inhibition of metastatic export ofcells seemed not to be reduced by many closely located collocated cells.*

This effect was completely unknown before having done the experiment and should be patented together with the ability of 4-OH-OPB to inhibit clonal growth in sparsely seeded or sparsely populated areas and the decrease of clonal inhibition by 4-OH-OPB due to higher collocation of (tumour) cells.

*When testing candidate anti-cancer drugs, this test on inhibition of metastases should be included in order to be able to fully evaluate each candidate. Versification by further experiments will be undertaken.*

*It is the opinion of the inventor that one of the criteria of malignancy, the ability to local infiltration of a malignant tumour, is based on the ability of the tumour cells to clonal growth outside the central tumour mass. In bernign tumours or normal organs single cells outside the tumour or organ would not be able to divide probably because an existing natural clonal inhibition. 4-OH-OPB will probably strengthen this natural ability and make many if not all-malignant tumours more benign or completely benign.*

### Experiment number 10:

### Testing the effect of 4-OH-OPB on number of cells in spleen with production of antibodies against sheep red cells after immunisation.

The test animals were 4 mice, white Bom/NMRI (Table 5). The drug that was tested in this experiment is 4-OH-OPB (Table 1-4). It is inhibiting Ehrlich ascites when injected together with the cells in peritoneum in a concentration of 4 µM or higher and clonal growth of BHK21/c13 and its polyoma virus transformed offspring, S100T1, in cell culture.

Therefore, it would be interesting to see if other cells, as spleen antibody producing cells, that are cloned *in vivo* when stimulated during a new immunisation, would show clonal inhibition. In addition, an experiment like this will give information about hematogenous transport of 4-OH-OPB to an organ containing more than average amount of blood.

Serious toxicity occurs at 280 µM estimated concentration in extracellular fluid (calculated as 2/7 of the animal weight). This is nearly two logs higher than needed for obtaining a good clonal inhibition. Therefore, the safety of the treatment is expected to be good.

The attempted transplantation 2 months before the sheep red cell immunisation:

**Experiment 10, Table 1:**

| Animal #: → | 1 | 2 | 3 | 4 |
|---|---|---|---|---|
| Initial weight | 33.8 | 30.1 | 31.3 | 36.2 |
| No Ehrlich cells: | 40000 | 40000 | 40000 | 40000 |
| Volume RPMI intraperitonally | 0.8 | 0.8 | 0.8 | 0.8 |
| 4-OH-OPB subcutaneously | 0 | 2 µl 20mM | 8.76 µl 20mM | 4.22 µl 200mM |
| Volume RPMI sucutaneously | 0 | 0.6 | 0.6 | 0.6 |
| Estimated maximal extracellular conc. | 0 | 4.6 µM | 19,6 µM | 67.7 µM |

| | | | | |
|---|---|---|---|---|
| The Batch 4-OH-OPB used was 060/56A from Syntagon. | | | | |

**Experiment 10, Table 2: Timetable of injections:**

| | day 0 | day 3 | day 8 | day 11 | day 14 | day 17 | day 21 |
|---|---|---|---|---|---|---|---|
| 1 | 0 | 0 | 0 | 0 | 0 | 0 | 0 |
| 2 | 2 µl 20 mM | 2 µl 20 mM | 2 µl 20 mM | 2 µl 20 mM | 2 µl 20 mM | 2 µl 20 mM | 2 µl 20 mM |
| 3 | 8.4 µl 20 mM | 8.4 µl 20 mM | 8.4 µl 20 mM | 8.4 µl 20 mM | 8.4 µl 20 mM | 8.4 µl 20 mM | 8.4 µl 20 mM |
| 4 | 3.5 µl 200 mM | 3.5 µl 200 mM | 3.5 µl 200 mM | 3.5 µl 200 mM | 3.5 µl 200 mM | 3.5 µl 200 mM | 3.5 µl 200 mM |
| Condition | all well | all well | all well | all well | all well | all well | Weight gain of 1+2 |

**Experiment 10, Table 3. Timetable continued.**

| | day 24 | day 28 | day 31 | day 57 | day 62 |
|---|---|---|---|---|---|
| 1 | 0 | 0 | 0 | 0 | Terminated |
| 2 | 2 µl 20 mM | 2 µl 20 mM | 2 µl 20 mM | 2 µl 20 mM | " |
| 3 | 8.4 µl 20 mM | 8.4 µl 20 mM | 8.4 µl 20 mM | 8.4 µl 20 mM | " |
| 4 | 3.5 µl 200 mM | 3.5 µl 200 mM | 3.5 µl 200 mM | 3.5 µl 200 mM | " |
| Condition | all well | all well | all well | all well | all well at end |

At the end all animals were clinically well. A section was performed; they were weighed and a search for tumour masses was undertaken without finding any. The spleens were removed and about half of it crushed in 0.5 ml Eagles for getting extracted, free spleen cells that were seeded in soft agar on top of a solid agar layer in a Petri dish of 8.5 cm in diameter. The agar layer with spleen cells also contained sheep red cells and complement was added to obtain hemolysis.

**Experiment 10, Table 4:**

| Animal | Spleen weight | Cells in 0.5 ml 1^{st} time (millions) | Cells in 0.5 ml 2^{nd} time (millions) | Million cells on dish 2^{nd} time | 1. number of plaques | 2. number of plaques | Plaque no. in 10 mill. cells |
|---|---|---|---|---|---|---|---|
| 1, control | 198 mg | 137 | 127 | 25.4 | many | 11910 | 4689 |
| 2. low | 148 " | 162 | 163 | 32.6 | many | 10218 | 3134 |
| 3. average* | 150 " | 50 | | | 42 | | 8.4 |
| 4. high * | 136 " | 86 | | | 0-2? | | <=0.23 |

| | | | | | | | |
|---|---|---|---|---|---|---|---|
| *: concentrations of 4-OH-OPB as indicated in Table 2-3. | | | | | | | |

**Experiment 10, Table 5: Mouse weight in gram before and after experiment.**

| Mouse number→ | 1 | 2 | 3 | 4 |
|---|---|---|---|---|
| Day 0 | 33.8 | 31.3 | 30.1 | 36.2 |
| After 2 months | 36.2 | 37.6 | 33.3 | 34 |
| µg 4-OH-OPB/30g | 0 | 11.3 | 51.5 | 210.2 |

Animals were killed by cervical dislocation on day 62 and spleens were immediately put on cold Eagle's medium for transportation. It lasted about 5 hours before the agar dishes were ready for incubation in 5% CO₂ and 37°C. However, the dishes from mouse #1 and #2 were too fluid in the top layers and developed two many plaques to be counted. Therefore, the next day, the second half of the two spleens from mouse #1 and #2 that were kept cold in the same medium were crushed in the same way and new dishes were prepared with less number of cells.

### Results:

The smallest dose of 4-OH-OPB did not significantly change the number of plaques developed in mouse spleen after immunisation with sheep read cells.

However, the average and high doses of 4-OH-OPB did reduce the number significantly.

The two plaques seen in the dish from the 4^{th} animal might not be absolutely true plaques. Therefore, there is a possibility that very high doses destroy or inactivate all preformed natural immune cells against sheep red cells.

Post mortem examination: The mice were sectioned and no tumour or ascites or other suspicious fluids were found. Number 3 had 0.15 ml blood in thorax after cervical dislocation. No suspicious cells were found in the blood before or after haemolysis of red cells.

### Conclusion:

*We now have more evidence (see experiment number 9) and probably proof that 4-OH-OPB can be transported to an organ, the spleen, with more than average amount of blood and produce biological effect in that organ. The effect is an inhibition of clonal growth of spleen antibody producing cells showing that not only malignant cells were inhibited and that the inhibition is very effective if the dose is high enough. An other important aspect is that spleen cells with other specificities seem not to be able to inhibit the effect. That was also the conclusion when injecting Ehrlich cells in peritoneum, a place with many other cells. The other abdominal cells seemed not to be able to abrogate the clonal inhibition of 4-OH-OPB. Therefore it is thought that only cells having the same specificity would be able to abrogate the inhibition of clonal growth of 4-OH.OPB if they are collocated in a concentration over a certain level.*

*However, the amount 4-OH-OPB being transported and the amount that was lost during the same time are not easy to estimate from these experiments.*

### Experiment number 11a:

### Testing Herpes virus type 1 (HSV1) for sensitivity against 4-OH-OPB.

It is needed to check the sensitivity since 4-OH-OPB may be effective against growth of arteriosclerotic plaques in arteries. If Herpes infection, that has been shown to be present in some, might contribute, 4-OH-OPB might hit even two targets affecting the disease.

RK13 cells and HSV1 v:11252 was obtained from Dr. G. Hoddevik, National Institute of Public Health, Oslo, Virus had been stored at -75°C.

**Experiment 11a, Table 1: Cytopathogenicity read after 24 hours:**

| 4-OH-OPB*→ | 0 | 0.1 | 0.3 | 1 | 3 | 10 | 30* |
|---|---|---|---|---|---|---|---|
| Inoculate↓: | | | | | | | |
| 1 µl/well | 1.7d | 1.7d | 1.7d | 1.7d | 1.7d | 4d | 3-4g 0d |
| 4 µl/well | 2.6d | 2.6d | 2.6d | 2.6d | 2.6d | 4d | 3-4g 0d |
| 16 µl/well | 3-4d | 3.4d | 3-4d | 3.4d | 4d | 4d | 3-4g 0.5d |

| | | | | | | | |
|---|---|---|---|---|---|---|---|
| *: µM 4-OH-OPB added. 4d=100% degenerated. 4g= unaffected and covering bottom of the well. | | | | | | | |

**Experiment 11a, Table 2: Row infected with 1 µl HSV (Table 1) was titrated and read after 4 days:**

| HIV from wells wirh 4-OH-OPB conc. (Table 1)→: | 0 | 0.1 | 0.3 | 1 | 3 | 10 | 30 | cell-control |
|---|---|---|---|---|---|---|---|---|
| Dilution of HSV↓ | | | | | | | | |
| ¼ | + | + | + | + | + | - | - | - |
| 1/16 | + | + | + | + | + | - | - | - |
| 1/64 | + | + | + | + | + | - | - | - |
| 1/256 | + | - | + | (+) | - | - | - | - |
| 1/1024 | - | - | ((+)? | - | - | - | - | - |
| 1/4096 | - | - | - | - | - | - | - | - |

### Results:

The cytopatogenicity of cultures that were infected with 1 µl HSV was moderate, but more pronounced in cultures with 10 µM or more of 4-OH-OPB probably as sign of toxicity (Table 1). The wells that received 1 µl HSV1 were titrated after 24 hours. 10 µM 4-OH-OPB inhibited HSV. (Table 1, Fig.1).

### Conclusion:

*10 µM 4-OH OPB seems necessary for the cure of the infection in cell culture. The cells in wells with titration of HSV from untreated cultures do not show significant differences compared to those with virus from sparsely treated wells after 4 days in culture. The inoculate was not removed before titration. But in the next experiment it was done.*

### Experiment number 11b:

### New experiment to see how sensitive Herpes virus 1 is for 4-OH-OPB.

The inoculated virus was not removed before the titration in the last experiment. This time, however, removal of inoculate was performed next day.

RK13 cells and HSV1 v:11252 was obtained from Dr. G. Hoddevik, National Institute of Public Health, Oslo, Virus had been stored at -75°C.

**Experiment 11b, Table 1: Cytopathogenicity etc. read after 2 days:**

| 4-OH-OPB→ | 0 | 0.1 | 0.3 | 1 | 3 | 10 | 30* |
|---|---|---|---|---|---|---|---|
| HSV↓ | | | | | | | |
| 2 µl/well | 0.28d 1g | 0.28d 1g | 0.28d 1g | 0.28d 1g | 0.28d 1g | 1d 0.4g | 0d 1g |

| | | | | | | | |
|---|---|---|---|---|---|---|---|
| *: µM 4-OH-OPB added | | | | | | | |

This time 1d is 100% degeneration. 1g= 100% coverage of the bottom.

### Medium: Eagle with 5% FBS.

HSV-1 was added undiluted, but 4-OH-OPB was diluted in 50 µl medium. The row seeded with 2000 n1 of HSV was titrated and read after 4 days:
4-OH-OPB: 20 mM in DMSO was prepared 8 days before the experiment started.

**Experiment 11b, Table 2: HSV titration read after 4 days:**

| Inoculate/AV: | 0 µM | 0.1 µM | 0.3 µM | 1 µM | 3 µM | 10 µM | 30 µM | cell-control |
|---|---|---|---|---|---|---|---|---|
| ¼* | + | + | (+)) | +- | - | - | - | - |
| 1/16 | + | (+) | - | - | - | - | - | - |
| 1/64 | (+) | - | - | - | | | | |
| 1/256 | ((+) | - | - | | | | | |
| 1/1024 | ((+) | | | | | | | |
| 1/4096 | ((+)) | | | | | | | |
| 1/16384 | - | | | | | | | |

### Results:

The cytopatogenicity was moderate, but increased for cultures with 10 µM of 4-OH-OPB, probably caused by its toxicity (Table 1). The less cytopatogenicity of 30 µM 4-OH-OPB is not fully understood. The wells that received 2 µl HSV1 got new medium after 24 hours and were titrated after 48 hours. 1 µM 4-OH-OPB was found to inhibit satisfactorily (Table 2, Fig.1).

### Conclusion:

*This time the inhibition is 10 times better than before. The difference from 1^{st} time was that the day after infecting cell cultures the medium was changed in order to get rid of remaining active HSV. In addition the original HSV 1 infected and treated cultures were incubated one day more, for 48 hours. The production of virus is then measured in titration of 4-fold dilution steps.*

*The result of this titration is considered adequate and showed a much better inhibition than last time without removal of the inoculate. 1000 nM 4-OH-OPB inhibited satisfactorily.*

### Experiment number 12:

### An experiment to see if Herpes virus 2 is sensitive for 4-OH-OPB.

The possibility needs to be investigated since the last two experiments using HSV1 showed an inhibition using concentrations between 1000 nM and 10000 nM of 4-OH-OPB. The best results was obtained when the inoculate was removed one day before titration.

RK13 cells and HSV2 v12225 was obtained from Dr. G. Hoddevik, National Institute of Public Health, Oslo, Virus had been stored at -75°C. It was propagated on RK13 cells.

After 24 hours the infected cultures showed changes indicated in Table 1. Then the medium, Eagles MEM with 5% FCS, was removed and replaced by new medium.

**Experiment 12, Table 1:Cytopathogenicity read after 24 hours**

| Inoculate/AV: | 0 | 0.1 | 0.3 | 1 | 3 | 10 | 30* | 0-contr. |
|---|---|---|---|---|---|---|---|---|
| 2 µl/well | O.5d 1g | 0.6d 1g | 0.7d 1g | 0.7d 1g | 0.95d 0.8g | 0.95d 0.9g | 0.2d 0.7g | 0d 1g |
| 8 µl/well | 0.9d 1g | 0.9d 1g | 0.9d 1g | 0.9d 1g | 0.95d 0.8g | 0.95d 0.86g | 0.2d 0.7g | 0d 1g |

| | | | | | | | | |
|---|---|---|---|---|---|---|---|---|
| *: µM 4-OH-OPB added | | | | | | | | |

This time 1d is 100% degeneration. 1g= 100% coverage of the bottom.

Experiment 12, Table 2, HSV 2 titration read day 4:

| Inoculate/AV: | 0 | 0.1 | 0.3 | 1 | 3 | 10 | 30 | cell-control |
|---|---|---|---|---|---|---|---|---|
| ¼* | + | + | + | + | + | + | - | - |
| 1/16 | + | + | + | + | + | + | - | - |
| 1/64 | + | + | + | + | - | - | | - |
| 1/256 | + | + | + | | | - | | - |
| 1/1024 | + | - | - | | | | | - |
| 1/4096 | + | - | - | | | | | - |
| 1/16384 | + | | | | | | | - |
| 1/65528 | + | | | | | | | |
| 1/262112 | - | | | | | | | |

### Results:

The inoculated virus was removed by changing the medium the day before titration. The titration was read after 3 days and is showing a logarithmic titre loss parallel to logarithmic increase of the concentration of 4-OH-OPB that was added to the cultures Fig. 1, esperiment 12.

Herpes type 2 infection in cultures treated with 4-OH-OPB showed inhibited production of HSV2 for concentrations above 0.1 µM. At 3 µM the inhibition was read to about 3 log after incubating the titration for 3 days. Maximal inhibition occurred after treating with 30 µM and was 5.5 log or greater.

### Conclusion:

*If a HSV infection might occur in arteries in connection with arteriosclerosis or even contribute to the vascular disease, there is reason to believe that 4-OH-OPB might "hit" the disease twice.*

### Experiment number 13:

### Colchicine and 4-OH-OPB as inhibitors in a density gradient of BHK21/c13 S100T1 cells in soft agar culture.

The aim of the experiment was to see if the addition of 4-OH-OPB and Colchicine acts similarly when added to BHK21/c13 S100T1 cells in agar. If this is true, anti-cancer effect might be obtained in vivo with a drug (Colchicine) that is in use in humans for the treatment of other diseases. The next possible aim should be to see if cells seeded as a density gradient in agar might demonstrate directly different growth retardation in the high or low cell density areas of the agar culture with 4-OH-OPB or Colchicine.

### Agar with cells:

Cells: The polyoma virus transformed offspring of BHK21/c13, S100T1, was grown on 75 cm² flasks on Eagles medium with 5% foetal calf serum. The cell layer of the culture flasks was rinsed with a mixture of equal volumes versene 2 mM and trypsin 0.25% and then treated with 1.5 ml of the same mixture for 16 minutes in 37°C when the cells were totally dispersed. Foetal calf serum (0.7 ml) was added to each harvest. After low speed centrifugation the supernatant Was discarded and the cells were dispersed in 1 ml FBS.

Plate: 24 wells Falcon tissue culture plate.

**Experiment 13, Table 1: Bottom layer preparation:**

| Agar conc.1.75% bottom layer | Each well | Total (x10) |
|---|---|---|
| Made from agar* 4.5 % in water, volume | 0.117 | 1.17 |
| 2 x conc. RPMI | 0.117 | 1.17 |
| 1 x Eagle | 0.067 | 0.67 |
| total | 0.3 | 3 |
| Added to each well | | 0.3 |

| | | |
|---|---|---|
| *: Sigma agarose, type II, A-6877 | | |

**Experiment 13, Table 2: Top layer preparation, ¾ of it without cells:**

| Agar conc.0.8%, top layer | Each well | Total (x10) |
|---|---|---|
| Agar # 4.5% in water | 0.040 | 0.4 |
| 2 x conc. RPMI | 0.040 | 0.4 |
| Eagle | 0.1 | 1 |
| Total volume before FBS: | 0.18 | 1.8 |
| FBS, volume | 0.045 | 0.45 |
| Total volume to each well | 0.225 | |
| Cell number pr. well | 0 | 0 |

| | | |
|---|---|---|
| #: Ultra-low gelling temperature agarose (Sigma, A2576) | | |

**Experiment 13, Table 3: Top layer preparation, ¼ of it with cells:**

| Agar conc.0.8%, top layer | Each well | Total (x10) |
|---|---|---|
| Agar # 4.5% in water | 0.0133 | 0.133 |
| 2 x conc. RPMI | 0.0133 | 0.133 |
| Eagle | 0.033 | 0.333 |
| Total volume before cells: | 0.06 | 0.6 |
| Cell number | 193000 | 1930000 |
| Cells in FBS, volume | 0.015 | 0.15 |
| Total volume after cells and serum | 0.075 | 0.75 |

| | | |
|---|---|---|
| #: Ultra-low gelling temperature agarose (Sigma, A2576) | | |

¾ of the top layer was added without cells containing 0.8% of the ultra low gelling temperature agarose (Sigma) (Table 2). The transformed cells in this experiment did not receive insulin since experiments do not indicate any need for that when using polyoma virus transformed cells. In this experiment 193000 cells were added to each top layer in the soft agar mixture described here (Table 3). The concentration of ultra low gelling temperature agarose (Sigma) of the top agar layer with cells was also 0.8%. In the bottom layer there was 1.75% of the type II agarose (A-6877, Sigma) Table 1. The addition of cells was performed at the same side of the wells in order to create similar cell density gradients. Finally the top layer agar was consolidated in the refrigerator.

Before the addition of 4-OH-OPB and Colchicine the plates were put in the refrigerator for ¾ hour in 5% CO₂ atmosphere for consolidating the top agar layer. Thereafter the wells received the specified dilutions (Table 4) of the inhibitor in the centre of top layer. The control did not get inhibitor. After that it was incubated at 37 degrees in 5% CO₂ atmosphere.

### Results:

Day 3and 4: Pictures were taken of the 6 wells. It is evident that only the control shows clonal growth at a certain distance from the central cell mass. This central mass seemed to grow at about the same rate in all wells. But the growth difference was easily observed between the control and wells with inhibitor where the cell density was low (Fig. 1-4, Table 5-6).

**Experiment 13, Table 4:**

| | Control | Low conc. | Medium conc. | High conc. |
|---|---|---|---|---|
| 4-OH-OPB, µM | 0 | 1 | 3 | nt |
| Colchicine, µM | | 0.01 | 0.033 | 0.1 |

**Experiment 13, Table 5: Summary of results after 3 days incubation:**

| Inhibitor conc.→ | Low | Medium | High | Comment |
|---|---|---|---|---|
| | 1 µM | 3 µM | | |
| 4-OH-OPB H* | +++ | +++ | nt | Very good growth in high density areas |
| 4-OH-OPB L* | +- | - | nt | Insignificant growth at low, none at medium conc. |
| | 10 nM | 33 nM | 100 nM | |
| Colchicine H | +++ | +++ | +++ | Very good growth in high density areas |
| Colchicine L | - | - | - | No growth in low density areas |
| Control H | +++ | | | Very good growth in high density areas |
| Control L | + | | | Probably significant growth in low density areas |

| | | | | |
|---|---|---|---|---|
| *: H: High cell density area, L: Low cell density area | | | | |

**Experiment 13, Table 6: Summary of results after 4 days incubation:**

| Inhibitor conc.→ | Low | Medium | High | Comment |
|---|---|---|---|---|
| | 1 µM | 3 µM | | |
| 4-OH-OPB H | +++ | +++ | nt | Very good growth in high density areas |
| 4-OH-OPB L | +- | - | nt | Insignificant growth at low, none at medium conc. |
| | 10 nM | 33 nM | 100 nM | |
| Colchicine H | +++ | +++ | +++ | Very good growth in high density areas |
| Colchicine L | +- | - | - | Insignificant growth at low, none at higher conc. |
| Control H | +++ | | | Very good growth in high density areas |
| Control L | ++ | | | Significant growth in low density areas |

### Conclusion:

*This experiment shows that: Colchicine and 4-OH-OPB did not inhibit growth in locations with high cell density. Colchicine and 4-OH-OPB inhibited growth in locations with low cell density even when using low doses, but not in high cell density locations.*

*Cell culture and animal experiments described in this application showed that high collocation of cells decreased or abolished the growth inhibiting effect of 4-OH-OPB. This is also true in this experiment. But in the same picture from the same well is demonstrated full inhibition on sparsely seeded cells.*

*This is probably also a good illustration of what has been tried to be demonstrated for the export of metastatic cells from a region rich in such cells. At a certain distance from the main cell mass, single cells were not able to grow when under the influence of a clonal inhibitor like 4-OH-OPB or may be Colchicine (not demonstrated in animal experiments jet).*

*This "peripheral" growth inhibition probably is what is needed to stop metastasizing from growing malignant cells or tumours. And high collocation in one region does not affect the effect at a short distance outside. This has also been verified in other experiments in this paper when using 4-OH-OPB e.g. experiment 9.*

### Experiment 14:

### The effect of cell density on clonal growth of MT4 cells inhibited by 4-OH-OPB.

### Question:

1. Is there a cell density below which no cell growth will take place?
2. Will 4-OH-OPB act differently on clonal growth in low-density cultures compared to high-density cultures?

Set-up: Cross dilution of cells (MT4 grown on Gibco foetal bovine serum, Cat. No. 10106-169, Lot No. 40F5426F) and 4-OH-OPB (Syntagon):

**Experiment 14, Table 1. Growth the second day:**

| | | | | | | |
|---|---|---|---|---|---|---|
| 4-OH-OPB* ↓ | | | | | | |
| 10 | | | | - | - | - |
| 3 | | | | - | - | + |
| 1 | | | | - | + | + |
| 0.3 | | | | - | + | + |
| 0.1 | | | | - | + | + |
| 0 | - | - | - | + | + | + |
| Cells/well→ | 5 cells | 50 cells | 500 cells | 5000 cells | 50000 cells | 500000 cells |

| | | | | | | |
|---|---|---|---|---|---|---|
| *: Final concentration in µM | | | | | | |

Ten fold cell dilutions indicatet in the last row (Table 1) were undertaken in Falcon tissue culture tray using 6x6 wells.

The 4-OH-OPB dilutions were prepared minutes before being used from 20 mM stock solution in DMSO.

The 10 µM and 3 µM dilutions were made first and each of them were diluted further in 1/10 steps.

### Conclusion:

*The answer of the first question: "Is there a cell density below which no cell growth will take place?" is not easy from these data since we have probably not waited long enough for growth of cells in wells with less than 5000 cells. However, it is probably much less likely that these few cells are able to form colonies than those being more abundantly seeded.*

*The last question: "Will 4-OH-OPB act differently on clonal growth in low-density areas compared to high-density areas?" will now be discussed:*

*The lowest inhibiting dose of 4-OH-OPB that inhibits the growth of clones of MT4 cells in a cell culture with fluid mediurn was found (**Fig. 1**). The concentration of 4-OH OPB needed for arresting colony growth was about 2 logs less in MT4 cultures with 5000 cells than in cultures with 500000 cells (**Fig. 2**). The interpretation of this is probably not that each cell needed a certain amount 4-OH-OPB to be bound to postulated receptors of the cell forfull clonal inhibition, since in agar cultures seeded with cells in a concentration gradient, the colony inhibition was strongest in sparsely seeded areas.*

*It is expected that within the same culture the availability of 4-OH-OPB should be equal for all cells. Therefore, the most likely explanation must be that cells in sparsely seeded areas are more sensitive to 4-OH-OPB than cells in densely seeded areas.*

*Also the results of animal experiments must be taken into consideration. These experiments were indicating that the inoculate consisting ofsingle tumour cells might form aggregates soon after transplantation or the injected tumour cells might be so concentrated that the anti-clonal effect of 4-OH-OPB treatment was abrogated Under these circumstances the Ehrlich tumour became able to continue the growth and finally killing the animal even when treated with 4-OH-OPB.*

*On the other hand, a large number of other cells in the neighbourhood of single tumour cells transplanted to an animal did not exert the same abrogating effect on tumour cells.*

*At the moment this hypothesis seems appropriate:*

*Close cell to cell interaction between tumour cells would abrogate the anticlonal effect of 4-OH-OPB. If the distance between tumour cells increases, a weaker interaction is expected between these related cells. Even if many other normal cells, that were unrelated to the tumour cells were present in the same area, the effect of 4-OH-OPB on tumour cells would not be abrogated.*

*Thus, interactive effects of related cells in close contact might blockpostulated cell receptors for 4-OH-OPB and allow clonal growth.*

### Experiment 15

### Screening non-steroid anti-inflammatory drugs (NSAIDs) or other potential drugs for anti-clonal effect using polyoma virus transformed BHK21/c13 cells, the S100T1 line, growing in soft agar medium.

Question: Are there substances other than 4-OH-OPB that have anticlonal activity? Will they be found among mitotic inhibitors, NSAIDs or common painkillers (Table 1)?

**Experiment 15, Table 1: Table showing the examples that were included in this study:**

| No | Name, Sigma catalogue no. | synonym | MW | Amount weighed | Vol. DMSO |
|---|---|---|---|---|---|
| 1 | 4-OH-OPB | AV-1101 | 340. 4 | 5.0 | 0.734 |
| 2 | Colchicine | Colchicine | 399. 4 | 3.3 | 0.413 |
| 3 | Diphenylhydantoin, D4007 | Fenantoin | 252. 3 | 5.5 | 1.090 |
| 4 | Podophyllotoxin P4405 | | 414. 4 | 4.8 | 0.579 |
| 5 | Piroxicam P5654 | Piroxicam | 331. 4 | 8.8 | 1.328 |
| 6 | Diclofenak D6899 | Voltaren | 318. 1 | 4.0 | 0.629 |
| 7 | Ibuprofen I 4883 | Brufen | 206. 3 | 3.1 | 0.751 |
| 8 | Naproxen M 4015 | Naprosyn | 230. 3 | 3.6 | 0.782 |
| 9 | Acetylsalicylic acid A 5376 | Aspirin | 180. 2 | 4.9 | 1.360 |
| 10 | Control | | | | |

**Experiment 15, Table 2: Dilutions and average dose:**

| NO | Name, Sigma catalogue no. | MW | Human dose, mg | µM in 20 litres* | 20 mM diluted # | Amount to well | µM in wells | Dilutions |
|---|---|---|---|---|---|---|---|---|
| 1 | 4-OH-OPB | 340.4 | 100 | 14.7 | 1/50 | 3,30 | 2,20 | 4/196 |
| 2 | Colchicine C-9754 | 399.4 | 0.5 | 0.063 | 1/10000 | 3,30 | 0.01,0.1 | 2/198,2/198¤ |
| 3 | Diphenylhydantoin, D4007 | 252.3 | 100 | 19.8 | 1/10 | 3,30 | 10,100 | 10/90 |
| 4 | Podophyllotoxin P4405 | 414.4 | 0.3-3 | 0.04-0.4 | 1/3333 | 3,30 | 0.03,0.3 | 6/194,2/19,8 |
| 5 | Piroxicam P5654 | 331.4 | 20 | 3.02 | 1/20 | 3,30 | 5,50 | 5/95 |
| 6 | Diclofenak D6899 | 318.1 | 50 | 7.86 | 1/10 | 3,30 | 10,100 | 10/90 |
| 7 | Ibuprofen I 4883 | 206.3 | 400 | 96.9 | 1/1 | 3,30 | 100,1000 | |
| 8 | Naproxen M 4015 | 230.3 | 250 | 54.3 | ½ | 3,30 | 50,500 | 50/50 |
| 9 | Acetylsalicylic acid A 5376 | 180.2 | 500 | 138.7 | 1/1 | 3,30 | 100,1000 | |
| 10 | Control | | | | | | | |

| | | | | | | | | |
|---|---|---|---|---|---|---|---|---|
| *: If the indicated human dose is solved in 20 litres that is equal to extracellular fluid of a person weighing 70 kg. #: This is the dilution needed of the DMSO solution of 20 mM of the indicated compound to obtain the indicated concentration of extra-cellular fluid that corresponds to the indicated human dose. ¤: Example: Dilutions 2/198 indicates 2 µl to 198 µl medium (Eagles MEM with 10% FBS) or 1:100. 2 µl is taken from this dilution and diluted further (1:100) giving 1:10000 final dilution. | | | | | | | | |

Agar cultures were prepared in wells of Falcon 24 well tissue culture plate. Each contained two agar layers of 0.3 ml. The bottom layer contained 1.75% Sigma agarose, type II, A-6877 lot 49H0380 and the top layer 32000 cells in 0.8% ultra low gelling temperature Sigma agarose type IX-A, A-2576 lot 19H0821. The medium contained both Eagle and RPMI 1640 with 10% FBS (Table 3-4).

Cells: The polyoma virus transformed S100T1 were grown on 75 cm² flasks on Eagles medium with 5% foetal calf serum. The cell layer of the culture flasks was rinsed with a mixture of equal volumes Versene 2 mM and Trypsin 0.25% and then treated with 1.5 ml of the same mixture for 30 minutes in 37°C until the cells were totally dispersed. Foetal calf serum (0.7 ml) was added to each harvest. After centrifugation the supernatant was discarded and the cells were dispersed in 1 ml FBS. The cells were all single cells. Each well received 32000 cells (Table 4). Plate: 24 wells Falcon tissue culture plate received a bottom layer composed as in Table 3:

**Experiment 15, Table 3:**

| Agar conc.( %) bottom layer | 1.75% |
|---|---|
| Made from agar 4.5 % in water, volume* | 3.888 |
| 2 x RPMI | 3.888 |
| 1 x Eagle | 2.22 |
| total | 10 |
| Added to each well | 0.3 |

| | |
|---|---|
| *: Agar, Sigma agarose, type II, A-6877 lot 49H0380 | |

The next table indicates the composition of top layers containing 0.8 % agar.

**Experiment 15, Table 4:**

| Agar conc.(%), top layer | 0.8% | Total preparation |
|---|---|---|
| Agar 4.5% in water # | 0.0533 | 2.005 |
| 2 x RPMI | 0.0533 | 2.005 |
| Eagle | 0.133 | 5.002 |
| Total volume: | 0.24 | 9 |
| Volume taken to each of the wells: | 0.24 | 0.24 |
| Cell number pr. well | 32000* | 1205000 |
| Cells in FBS, volume | 0.06 | 2.26 |

| | | |
|---|---|---|
| *: 533333 per ml #: ultra low gelling temperature agarose type IX-A, Sigma A-2576, Lot19H0821 | | |

Before the addition of 4-OH-OPB or the other compounds (Table 2), the plates were put into the refrigerator for 1 hour for consolidating the top agar layer. The control did not receive anything.

**Experiment 15, Table 5: The plate read the 5^{th} day:**

| No | Low conc. | Clonal growth | Significance# | High conc. | Clonal growth | Significance |
|---|---|---|---|---|---|---|
| 1 | 2 | - | yes | 20 | - | yes |
| 2 | 0.01 | ++ | no | 0.1 | +(+) | no |
| 3 | 10 | ++ | " | 100 | +(+) | " |
| 4 | 0.03 | +(+) | " | 0.3 | ((+)) | probably |
| 5 | 5 | ++ | " | 50 | + | no |
| 6 | 10 | ++(+) | probably* | 100 | ++ | " |
| 7 | 100 | +(+) | no | 1000 | - | ? |
| 8 | 50 | +(+) | " | 500 | - | ? |
| 9 | 100 | + | " | 1000 | - | ? |
| 10 | 0 | ++ | | | | |

| | | | | | | |
|---|---|---|---|---|---|---|
| *: Diclofenak probably stimulated clonal growth significantly at this concentration ?: The DMSO concentration is too high for deciding significance #: Significance: + or ++: no, (+: doubtful, (+): possible, (+)): probable, -: yes | | | | | | |

### Conclusion:

*As shown in* *Fig. 1-2**, experiment 15, 4-OH-OPB was inhibiting clonal growth at the concentrations tested, 2 and 20 µM.*

*Colchicine did not inhibit clonal growth at concentrations from 0,01-0,1 µM.*

*Diphenhydantoin did not inhibit clonal growth significantly at concentrations 10-100 µM.*

*Podophyllotoxin inhibited significantly at 0.3 µM, but still some few clones persisted. There was no significant inhibition at 0. 03 µM.*

*Piroxicam inhibited doubtfully at 50 µM, but not at 5 µM.*

*Diclofenac showed no significant inhibition at 10-100 µM. However, a probable significant increased in clonal growth was observed at 10 µM.*

*Ibuprofen showed insignificant inhibition at 100 µM. The inhibition at 1000 µM might not be real since it involved the addition of 30 µl of DMSO to the well. That might be toxic (see next experiments).*

*Naproxen inhibited significantly at 500 µM, but DMSO toxicity might interfere as mentioned for Ibuprofen.*

*Acetylsalicylic acid possibly inhibited significantly, but not completely at 100 µM. At 1000 µM the inhibition is complete, but might partially be caused by DMSO as mentioned for Ibuprofen.*

### Experiment 16:

### Testing drugs or potential drugs for anti-clonal effect using polyoma virus transformed BHK21/c13 cells, the clone S100T1, growing in soft agar.

Question: Are there other substances than 4-OH-OPB that might have anticlonal activity? Will they be found among inhibitors of mitosis or NSAIDs or common painkillers (Table 1)? What about Colchicine that tested positive before? The test became negative last time. Also the other that tested positive using high concentrations should be tested again.

**Experiment 16, Table 1:**

| No | Name, Sigma catalogue no. | synonym | MW | Amount weighed | Vol. DMSO |
|---|---|---|---|---|---|
| 1# | Colchicine, C-9754, Lot 60K1932 | Colchicine | 399. 4 | 3.3 | 0.413 |
| 2* | Colchicine, C-9754, Lot 60K1932 | Colchicine | 399. 4 | 2.1 | 0.263 |
| 3,4* | Colchicine, C-9754, Lot 28H1229. | Colchicine | 399. 4 | 3.2 | 0.401 |
| 5# | Podophyllotoxin P4405, Lot 12K1562 | | 414. 4 | 4.8 | 0.579 |
| 6,7* | Podophyllotoxin P4405, Lot 12K1562 | | 414. 4 | 2.4 | 0.290 |
| 8* | Ibuprofen I 4883, Lot 26H1386 | Brufen | 206. 3 | 3.1 | 0.751 |
| 9* | Naproxen M 4015, Lot 111K1991 | Naprosyn | 230. 3 | 3.6 | 0.782 |
| 10* | Acetylsalicylic acid A 5376, Lot 31K0013 | Aspirin | 180. 2 | 4.9 | 1.360 |
| 11* | 4-OH-OPB, Syntagon | AV-1101 | 340. 4 | 5.0 | 0.734 |
| 12 | Control | | | | |

| | | | | | |
|---|---|---|---|---|---|
| *: These were solved in DMSO the same day. #: Solved in DMSO 8 days in beforehand, but was stored frozen. | | | | | |

**Experiment 16, Table 2: Dilutions, concentrations:**

| No | Name, catalogue no. | MW | Human dose, mg | µM in 20 litres | Dil. of 20 mM | Amount to wells | µM in wells | Dilutions |
|---|---|---|---|---|---|---|---|---|
| 1# | Colchicine | 399.4 | 0.5 | 0.063 | 1/1000 | 3,30 | 0.1,1 | 20/180,2/198 |
| 2* | Colchicine | 399.4 | 0.5 | 0.063 | 1/1000 | 3,30 | 0.1,1 | 20/180,2/198 |
| 3* | Colchicine | 399.4 | 0.5 | 0.063 | 1/1000 | 3,6.46 | 0.1,0.22 | 20/180,2/198 |
| 4* | Colchicine | 399.4 | 0.5 | 0.063 | 1/1000 | 13.92,30 | 0.46,1 | 20/180,2/198 |
| 5# | Podophyllotoxin | 414.4 | 0.3-3 | 0.04-0.4 | 1/333,3 | 3,30 | 0.3, 3 | 60/140,2/198 |
| 6* | Podophyllotoxin | 414.4 | 0.3-3 | 0.04-0.4 | 1/3333 | 3,6.46 | 0.03,0.065 | 6/194,2/198 |
| 7* | Podophyllotoxin | 414.4 | 0.3-3 | 0.04-0.4 | 1/3333 | 13.92,30 | 0.139,0.3 | 6/194,2/198 |
| 8* | Ibuprofen | 206.3 | 400 | 96.9 | 1/1 | 6.46,13.92 | 215,464 | none |
| 9* | Naproxen | 230.3 | 250 | 54.3 | ½ | 6.46,13.92 | 108,232 | 50/50 |
| 10* | Acetylsalicylic acid | 180.2 | 500 | 138.7 | 1/1 | 6.46,13.92 | 215,464 | none |
| 11* | 4-OH-OPB | 340.4 | 100 | 14.7 | 1/150 | 3,30 | 0.67,6.7 | 2/300 |
| 12 | Control | | | | | 0,30 DMSO ¤ | | |

| | | | | | | | | |
|---|---|---|---|---|---|---|---|---|
| *: These were solved in DMSO the same day. #: Solved in DMSO 8 days in beforehand, but was stored frozen. ¤: It was added 30 µl DMSO on this control. | | | | | | | | |

Cells: The polyoma virus transformed line S100T1 was grown on 75 cm² flasks on Eagles medium with 5% foetal calf serum. The cell layer of the culture flasks was rinsed with a mixture of equal volumes Versene 2 mM and Trypsin 0.25% and then treated with 1.5 ml of the same mixture for 16 minutes in 37°C until the cells were totally dispersed. Foetal calf serum (0.7 ml) was added to each harvest. After centrifugation the supernatant was discarded and the cells were dispersed in FBS. The cells were all single cells.

**Experiment 16, Table 3: Top layer preparation, total preparation per plate:**

| Agar conc.(%), top layer | 0.8% | Total preparation |
|---|---|---|
| Agar 4.5% in water # | 0.0533 ml | 2.00 ml (90 mg agar) |
| 2 x RPMI | 0.0533 ml | 2.00 ml |
| Eagle | 0.133 ml | 5.00 ml |
| Total volume before addition of cells: | 0.24 ml | 9 ml |
| Cell number pr. well | 40000* | 1500000* |
| Cells in FBS, volume of 666667/ml: | 0.06 ml* | 2.25 ml* |
| Volume taken to each of the wells: | 0.3 ml | |

| | | |
|---|---|---|
| *: 0.66667 mill. cells/ml #: Ultra low gelling temperature agarose type IX-A, Sigma A-2576, Lot19H0821 | | |

**Experiment 16, Table 4: Bottom layer preparation:**

| Final agar conc.(%) in bottom layer | 1.75% |
|---|---|
| Made from agar* 4.5 % in water, volume | 3.89 |
| 2 x conc. RPMI | 3.89 |
| Eagle's MEM | 2.22 |
| Total | 10 |
| Added to each well | 0.3 |

| | |
|---|---|
| *: Sigma agarose, type II, A-6877 | |

### Agar layers:

Agar cultures (Table 3-4) were prepared in wells of Falcon 24 well tissue culture plate. The wells contained two layers, each of 0.3 ml agar. The medium contained both Eagle and RPMI 1640 with 10% FBS.

Ultra-low gelling temperature agarose (Sigma A-2576, Lot19H0821) was used in the top layer and the Sigma agarose, type II, A-6877, Lot 49H0380 in the bottom layer. Concentrations are indicated in the tables above. The agarose was added to double distilled water and solved by heating in micro oven.

Before the addition of 4-OH-OPB or the other compounds (Table 2), the plates were put into the refrigerator for 1 hour for consolidating the top agar layer.

The control did not receive anything unless indicated.

**Experiment 16, Table 5: Table summarising status on day 14:**

| No | Compound | µM in wells | Growth | Significance# | µM in wells | Growth | Significance# |
|---|---|---|---|---|---|---|---|
| 1 | Colchicine | 0.1 | (+ | doubtful | 1 | (+ | doubtful |
| 2 | Colchicine | 0.1 | (+ | " | 1 | (+) | possible |
| 3 | Colchicine | 0.1 | (+) | possible | 0.22 | (+) | " |
| 4 | Colchicine | 0.46 | (+) | " | 1 | (+)) | probable |
| 5 | Podophyllotoxin | 0.3 | (+)) | probable | 3 | (+)) | " |
| 6 | Podophyllotoxin | 0.03 | ++) | no | 0.065 | (+) | possible |
| 7 | Podophyllotoxin | 0.139 | (+)) | probable | 0.3 | (+) | " |
| 8 | Ibuprofen | 215 | (+) | possible | 464 | (+)) | doubtful* |
| 9 | Naproxen | 108 | (+ | doubtful | 232 | (+) | possible* |
| 10 | Acetylsalicylic acid | 215 | (+)) | probable | 464 | (+)) | doubtful* |
| 11 | 4-OH-OPB | 0.67 | - | yes | 6.7 | - | yes |
| 12 | Control | 0 | ++ | | 30 DMSO ¤ | - | DMSO effect |

| | | | | | | | |
|---|---|---|---|---|---|---|---|
| *: Number 8-10, see conclusion #: Significance: + or ++: no, (+: doubtful, (+): possible, (+)): probable, -: yes ¤: Here 30 µl were added. | | | | | | | |

**Experiment 16, Table 6: Table summarising status on day 21:**

| No | Compound | µM in wells | Growth | Significance# | µM in wells | Growth | Significance# |
|---|---|---|---|---|---|---|---|
| 1 | Colchicine | 0.1 | + | no | 1 | (+ | doubtful |
| 2 | Colchicine | 0.1 | (+ | doubtful | 1 | (+) | possible |
| 3 | Colchicine | 0.1 | (+ | " | 0.22 | (+ | doubtful |
| 4 | Colchicine | 0.46 | (+) | possible | 1 | (+ | " |
| 5 | Podophyllotoxin | 0.3 | (+ | doubtful | 3 | (+)) | probable |
| 6 | Podophyllotoxin | 0.03 | ++ | no | 0.065 | (+ | doubtful |
| 7 | Podophyllotoxin | 0.139 | (+ | doubtful | 0.3 | (+) | possible |
| 8 | Ibuprofen | 215 | (+)) | probable | 464 | (+)) | doubtful* |
| 9 | Naproxen | 108 | (+ | doubtful | 232 | (+) | possible* |
| 10 | Acetylsalicylic acid | 215 | (+) | possible | 464 | (+)) | doubttul* |
| 11 | 4-OH-OPB | 0.67 | - | yes | 6.7 | - | yes |
| 12 | Control | 0 | ++ | | 30 DMSO ¤ | - | DMSO effect |

| | | | | | | | |
|---|---|---|---|---|---|---|---|
| *: Number 8-10, see conclusion #: Significance: + or ++: no, (+: doubtful, (+): possible, (+)): probable, -: yes ¤: Here 30 µl were added. | | | | | | | |

### Conclusions:

The significance of results for Colchicine read after 14 days is shown in Table 7:

**Experiment 16, Table 7:**

| | | 0.1 µM | 0.22 µM | 0.46 µM | 1 µM |
|---|---|---|---|---|---|
| 1# | Lot 60K1932 | doubtful | | | doubtful |
| 2* | Lot 60K1932 | doubtful | | | possible |
| 3* | Lot 28H1229 | possible | possible | | |
| 4* | Lot 28H1229 | | | possible | probable |

| | | | | | |
|---|---|---|---|---|---|
| *: These were solved in DMSO the same day. #: Solved in DMSO 8 days in beforehand, but were stored frozen. | | | | | |

***This table shows clearly that there might be difference between the two Colchicine lots. The lot 60K1932 was not so effective and storage and freezing further reduced the effect.***

***It might be possible that therapeutic doses of Colchicine could inhibit cloning.** But **the effect is not as good as 4-OH-OPB and some few clones might escape the inhibition.***

**Experiment 16, Table 8: Table summarising the significance of results for Colchicine read after 21 days:**

| | | 0.1 µM | 0.22 µM | 0.46 µM | 1 µM |
|---|---|---|---|---|---|
| 1# | Lot 60K1932 | no | | | doubtful |
| 2* | Lot 60K1932 | doubtful | | | possible |
| 3* | Lot 28H1229 | doubtful | doubtful | | |
| 4* | Lot 28H1229 | | | possible | doubtful |

| | | | | | |
|---|---|---|---|---|---|
| *: These were solved in DMSO the same day. #: Solved in DMSO 8 days in beforehand, but were stored frozen. | | | | | |

*After 21 days the lot 60K1932 behaved as read after 14 days. The lot 28H1229, however, seemed to having lost some activity the last week.*

**Experiment 16, Table 9: For Podophyllotoxin the significance of results are summarised in this table when read after 14 days:**

| | | 0.03µM | 0.065 µM | 0.139 µM | 0.3 µM | 3 µM |
|---|---|---|---|---|---|---|
| 5# | Lot 12K1562 | | | | probable | probable |
| 6* | Lot 12K1562 | no | possible | | | |
| 7* | Lot 12K1562 | | | probable | possible | |

| | | | | | | |
|---|---|---|---|---|---|---|
| *: These were solved in DMSO the same day. #: Solved in DMSO 8 days in beforehaitd, but were stored frozen. | | | | | | |

*Storage infreezer for 8 days did not reduce activity, but might increase the activity instead.*

*Dosage within a range that might be therapeutic revealed a probable anti-clonal activity that was not as good as for 4-OH-OPB.*

**Experiment 16, Table 10: For Podophyllotoxin the significance of results are summarised in this table when read after 21 days:**

| | | 0.03µM | 0.065 µM | 0.139 µM | 0.3 µM | 3 µM |
|---|---|---|---|---|---|---|
| 5# | Lot 12K1562 | | | | doubtful | probable |
| 6* | Lot 12K1562 | no | doubtful | | | |
| 7* | Lot 12K1562 | | | doubtful | possible | |

| | | | | | | |
|---|---|---|---|---|---|---|
| *: These were solved in DMSO the same day. #: Solved in DMSO 8 days in beforehand, but were stored frozen. *Storage in freezer for 8 days might indicate a slight decrease of activity when read after 3 weeks.* | | | | | | |

*Dosage within a range that might be therapeutic revealed a possible anti-clonal activity that was not as good as for 4-OH-OPB and less significant than results read after 2 weeks (Table 5-6, 8-11).*

**Experiment 16, Table 11: The significance of results for Ibuprofen, Naproxen, Acetylsalicylic acid and 4-OH-OPB read after 14 days:**

| | | 0,67 µM | 6.7 µM | 108 µM | ca. 223 µM | 464 µM |
|---|---|---|---|---|---|---|
| 8 | Ibuprofen | | | | possible | doubtful* |
| 9 | Naproxen | | | doubtful | possible | |
| 10 | Acetyl salicylic acid | | | | probable | doubtful* |
| 11 | 4-OH-OPB | yes | yes | | | |

| | | | | | | |
|---|---|---|---|---|---|---|
| *: Doubtful because the solvent DMSO had a concentration in these wells that might be inhibiting. These high DMSO concentrations make new tests necessary. | | | | | | |

*The DMSO concentration for compounds number 8 and 10 could interfere as a clonal growth inhibitor since the concentrations was above about 1% as found in a later experiment.*

*The results concerning number 9, Naproxen, could be accepted as possibly significant since the concentration of DMSO did not exceed a critical level.*

*The results concerning highest concentrations of Ibuprofen and Acetyl salicylic acid, however, could not be accepted since DMSO itself might inhibit. But these drugs possibly orprobably showed significant clonal inhibition when added to the cultures less concentrated and accompanied of less DMSO concentration.*

*The results showed that Naproxen and Acetylsalicylic acid probably had a colony inhibiting activity for dosage within a range that might be therapeutic. For Ibuprofen, Colchicine and Podophyllotoxin the same activity possibly existed But the cell number in each well was low and made it difficult to estimate the possibility of toxicity on cells in densely seeded areas or on the organism.*

*The control 4-OH-OPB was clearly better than any other.*

### Experiment 17:

### Verifying observations of the mitotic inhibitors Colchicine and Podophyllotoxin inhibiting clonal growth and introducing Etoposide, a derivative of Podophyllotoxin.

Question: How effectively do Colchicine, Podophyllotoxin and its derivative Etoposide inhibit clonal growth compared to the effect of 4-OH-OPB, and what level of the solvent DMSO might interfere with the results?

**Experiment 17, Table 1: Dilutions, related to average human doses:**

| No | Name, catalogue no. | MW | Human dose, mg | µM in 20 litres | Dilutions of 20 mM | Dilutions | Amount to wells | µM in wells |
|---|---|---|---|---|---|---|---|---|
| 1 | Colchicine, Sigma C-9754 Lot 28H1229 | 399. 4 | 0.5 | 0.063 | 20/180, 2/198# | 1/1000 | 3.5,10.5,3 5¤ | 0.1,0.3, 1¤ |
| 2 | Podophyllotoxin, Sigma P-4405, Lot 12K1562 | 414. 4 | 0.3-3 | 0.04-0.4 | 60/140, 2/198 | 1/333,3 | 3.5,10.5,3 5 | 0.3, 0.9, 3 |
| 3 | Etoposide Sigma E-1383 Lot 111K1538* | 588. 6 | 180 | 15.3 | 3/57 | 1/10 | 3.5,10.5,3 5 | 20,60,200 |
| 4 | 4-OH-OPB Syntagon | 340. 4 | 100 | 14.7 | 2/300 | 1/150 | 3.5,10.5,3 5 | 0.67, 2, 6.7 |
| 5 | DMSO Sigma D-5979 | | | | | | 1.16,6.8, 29.2 | |
| 6 | Control | | | | | | 0 | 0 |

| | | | | | | | | |
|---|---|---|---|---|---|---|---|---|
| #: Serial dilutions: 20 µl are taken from 20 mM DMSO solution. After mixing with 180 ml medium 2 µl of this dilution are diluted further by mixing with 198 µl medium giving a final dilution of the DMSO solution of 1/1000. *: Unlike the others that were solved in DMSO to 20 µM, Etoposide is diluted in DMSO to 40 mM. ¤: The amounts and concentrations for low, medium and high doses separated by commas. | | | | | | | | |

In the two previous experiments it was used about 40000 cells in each well of 0.6 ml. In these experiments the initial growth was retarded more than expected when compared to other experiments. Therefore, the cell number in the well was increased to about 144000 (Table 4). This cell number was expected to be more optimal.

The aim of this experiment was to see if the addition of other compounds than 4-OH-OPB inhibited clonal growth when added to S100T1 cells, a polyoma virus transformed line of BHK21/c13, seeded in agar (Table 4-5).

Agar cultures were prepared in Falcon 24 well tissue culture plate. Each well contained two agar layers of 0.3 ml. The bottom layer contained 1.75% Sigma agarose, type II, A-6877, Lot 49H0380 and the top layer 144000 cells in 0.8% ultra low gelling temperature Sigma agarose type IX-A, Sigma A-2576, Lot19H0821.

### Agar with cells:

Cells: Polyoma virus transformed BHK21/c13 cells, the S100T1 cell line, was grown on 75 cm² flasks on Eagles medium with 5% foetal calf serum. The cell layer of the culture flasks was rinsed with a mixture of equal volumes Versene 2 mM and Trypsin 0.25% and then treated with 1.5 ml of the same mixture for 16 minutes in 37°C until the cells were totally dispersed. Foetal calf serum (0.7 ml) was added to each harvest. After centrifugation the supernatant was discarded and the cells were dispersed in 1 ml FBS. The cells were all single cells.

**Experiment 17, Table 2: Bottom layer preparation in Falcon 24 well plates:**

| Agar conc.(%) bottom layer | 2.25% |
|---|---|
| Made from agar* 4.5 % in water, volume | 10 |
| 2 x RPMI | 10 |
| 1 x Eagle | 0 |
| total | 20 |
| Added to each well | 0.3 |

| | |
|---|---|
| *: Sigma agarose, type II, A-6877 | |

**Experiment 17, Table 3: Top layer preparation:**

| Top layer preparation without cells: Final agar conc.0.8% | Each well | Total preparation |
|---|---|---|
| Agar 4.5% in water (ultra low...#) | 0.04 | 2.7 |
| 2 x RPMI | 0.04 | 2.7 |
| Eagle | 0.1 | 6.75 |
| FBS, volume | 0.045 | 3.04 |
| Total volume: | 0.225 | 15.19 |
| Volume taken to each of the wells: | 0.225 | 0.225 |

| | | |
|---|---|---|
| #: Ultra-low gelling temperature agarose (Sigma A-2576) | | |

**Experiment 17, Table 4: The cell containing part of the top layer used for making cell gradient:**

| Agar conc.0.8%, top layer | Each well | Total (x25) |
|---|---|---|
| Agar # 4.5% in water | 0.0133 | 0.333 |
| 2 x conc. RPMI | 0.0133 | 0.333 |
| Eagle | 0.033 | 0.825 |
| Cells in FBS, volume | 0.0174 | 0.436 |
| Cell number | 144000 | 3.6 mill. |
| Total volume before cells: | 0.06 | 1.5 |
| Total volume after cells and serum | 0.075 | 1.878 |

| | | |
|---|---|---|
| #: Ultra-low gelling temperature agarose (Sigma A-2576) | | |

Before the addition of 4-OH-OPB or one of the other compounds (Table 1), the plates were put into the refrigerator for 1 hour for consolidating the soft agar layer on the top.

The compounds were added to the different S100T1 agar cell cultures. Control wells did not receive anything or DMSO in the µl amounts indicated in Table 1.

The cell preparation did not contain aggregated cells after having been trypsinised for 16 min. and contained a little more than 3.6 mill cells. Each well received 144000 cells.

**Experiment 17, Table 5: Clonal growth read on the 3^{rd} day after seeding:**

| # | Low* | Medium* | High* |
|---|---|---|---|
| 1 | ++ | ++ | - |
| 2 | ((+) | ((+) | ((+) |
| 3 | ((+) | (+) | (+) |
| 4 | + | - | - |
| 5 | +(+ | +(+ | +(+ |
| 6 | ++ control | - (control day 0) | |

| | | | |
|---|---|---|---|
| *: Low medium or high concentrations of the substances tested for activity, see the first table | | | |

**Experiment 17, Table 6: Clonal growth read on the 6^{th} day after seeding:**

| # | Low* | Medium* | High* |
|---|---|---|---|
| 1 | + | + | ((+) |
| 2 | + | (+ | ((+) |
| 3 | + | ((+) | - |
| 4 | + | ((+)) | ((+)) |
| 5 | ++ | +(+ | (+ |
| 6 | ++ control | - (control day 0) | |

| | | | |
|---|---|---|---|
| *: Low medium or high concentrations of the substances tested for activity, see the first table | | | |

**Experiment 17, Table 7: Results read on the day 14:**

| | Low* | Significance# | Medium* | Significance | High* | Significance |
|---|---|---|---|---|---|---|
| Colchicine a | ++ | No | ++ | No | (+) | Possible |
| Colchicine b | ++ | No | ++ | No | (+) | Possible |
| Podophyllotoxin a | (+ | Doubtful | (+ | Doubtful | (+) | Possible |
| Podophyllotoxin b | + | No | + | No | (+) | possible |
| Etoposide a | + | No | + | No | (+) | Possible |
| Etoposide b | (+) | Possible | (+) | Possible | (+) | Possible |
| 4-OH-OPB a | ++ | No | ++ | No | ((+)) | Probably |
| 4-OH-OPB b | + | No | - | Yes | - | Yes |
| DMSO a | ++ | No | ++ | No | ((+) | Probably |
| DMSO b | ++ | No | +(+) | No | | Yes |

| | | | | | | |
|---|---|---|---|---|---|---|
| a: Clonal growth located close to densely seeded cell areas b: Clonal growth in sparsely seeded areas in the same agar culture *: Low medium or high concentrations of the substances tested for activity, see the first table #: Significance: + or ++: no, (+: doubtful, (+): possible, (+)): probable, -: yes | | | | | | |

**Experiment 17, Table 8: Controls read on day 14:**

| a | b | Control before incubation* |
|---|---|---|
| ++ | ++ | - |

| | | |
|---|---|---|
| a: Clonal growth located close to dense cell areas b: Clonal growth in sparsely seeded areas *: 0 control from day 0 just after seeding. | | |

### Discussion and Conclusion: None of the tested inhibitors (Colchicine,

*Podophyllotoxin, or Etoposide) did inhibit clonal cell growth as effectively as 4-OH-OPB (Table 7-8). All three seented to allow the growth of a few small clones in sparsely seeded locations of the agar (**Fig. 1**, experiment 17).*

### Etoposide seemed to promote the best inhibition of cells in crowded areas. Therefore, this might be the best candidate for combination with 4-OH-OPB during short-term periods of treatment.

*But when treated over long time, Podophyllotoxin and Etoposide is known to have high toxicity. This is in agreement with the significant growth inhibition found in crowded areas.*

*It is known that Colchicine is tolerated during longer time periods, an advantage if needed in treatment.*

*Thus, the tested growth inhibitors consisted of two groups, the inhibitors that inhibited cell growth in both high and low cell density locations, the Podophyllotoxin group, and the inhibitors that only inhibited clonal growth in low density areas, 4-OH-OPB and Colchicine. Especially for average 4-OH-OPB concentration there is good growth of colonies in high-density areas, but no growth in low-density areas (Table 7,* *Fig. 1**).*

*Colchicine did not inhibits the high-density areas significantly. Only high dose seemed to inhibit the gr owth of cells inlow-density areas, but no complete clonal inhibition could be observed.*

*Both compounds in the Podophyllotoxin group behaved similarly with growth inhibition of both high and low-density areas. But complete inhibition of clonal growth in low-density areas was not observed. Therefore, effective control of metastases would not be expected when treating animal or man.*

*These findings indicated that Podophyllotoxin or Etoposide might be toxic also to cells in densely populated areas and in the body as is already known.*

*Testing DMSO resulted in approximately unaffected growth inhibition up to about 1% concentration in soft agar cultures. 4.7% inhibited formation of clones, but not completely.*

The table below indicates what is supposed to become the main advantages or disadvantages of the tested substances if used in cancer treatment (Etoposide already is in use):

**Experiment 17, Table 9:**

| | Toxicity for body or dense cell populations | Inhibition of tumour cell colonies* | Control of metastatic cells leaving tumour# | Control of settlement of metastatic cells ¤ | Tolerating long duration of treatment |
|---|---|---|---|---|---|
| Colchicin | none or small | Possibly, but only partly | probably not | partly | yes |
| 4-OH-OPB | none or small | Possibly, but only partly | yes | completely | yes |
| Etoposide | yes | yes, but not completely | probably not | partly | no |
| Podophyllotoxin | yes | yes, but not completely | probably not | partly | no |

| | | | | | |
|---|---|---|---|---|---|
| *: Inhibition of colonies or tumours like metastases or primary tumours greater than a certain size. #: Control of the ability of cells to leave a tumour, grow and infiltrate locally outside tumour or grow in sparsely seeded areas. ¤: Control of settlement of single cells or very few cells spread to distant sites from a tumour or tumour cell rich area. | | | | | |

In situations where clones are about to grow from single cells or oligocellular clumps, as supposed to happen if effective anti-clonal treatment is not jet started, it might be advantageous to combine 4-OH-OPB with Etoposide. Toxicity is limiting Etoposide from being applied for more than a few days each time. It is expected, however, that 4-OH-OPB would be tolerated for much longer periods.

The situation for Colchicine is not clear after this experiment since there is doubt about its effect on clonal growth in sparsely seeded areas.

When bigger tumours appear, either palpable or detected by x-rays or similar techniques, 4-OH-OPB alone is probably not advisable alone. However, 4-OH-OPB would be expected to prevent metastatic cells leaving the tumour. This is especially important during surgery, but would probably be valuable also during chemical treatment or irradiation.

Experiments in mice transplanted with Ehrlich's carcinoma subcutaneously did indicate stop in tumour cells leaving the tumour cell deposits when given 4-OH-OPB. In addition this treatment will probably stop the settlement of tumour cells reaching distant organs.

*In a new experiment we will see if mice transplanted with Ehrlich carcinoma in peritoneum would be rescued even if the treatment is started the day after transplantation if not only 4-OH-OPB is given to the animal, but a combination of Etoposide and 4-OH-OPB. This last compound should be given in a dose not effective alone.*

*A control animal should receive a moderate dose of 4-OH-OPB the day after transplantation to confirm earlier established findings of missing cure.*

We would also like to ask if Colchicine alone will stop settlement of transplanted Ehrlich tumour cells when mixing the drug with the transplanted cells before injecting them as done with 4-OH-OPB.

### Experiment 18:

### Treating mice with 4-OH-OPB in combination with Tarivid and trying Colchicine against Ehrlich carcinoma transplanted to peritoneum.

*Question: Will mice transplanted with Ehrlich carcinoma in peritoneum be rescued even if the treatment is started the day after transplantation if not only 4-OH-OPB is given to the animal, but a combination of Etoposide and 4-OH-OPB, the last in a dose not effective alone?*

*A control animal will receive a moderate dose of 4-OH-OPB the day after transplantation to confirm earlier established findings of missing cure.*

*Will ,Colchicine alone stop settlement of transplanted Ehrlich tumour cells when mixing the drug with the transplanted cells before injecting them as done with 4-OH-OPB?*

The frozen and thawed Ehrlich ascites cells are diluted in a volume of 1 ml RPMI (pH∼6.9) for each animal. No calf serum was added to the medium to avoid immunisation of the mice.

Cell number: 90000 tumour cells in 0.7 ml RPMI were injected intraperitonally to each animal.

The total cell number in the ampoule was 7.5 million cells. The cells were thawed, and 2 ml FBS was added before low speed centrifugation. The supernatant was decanted and cells mixed with RPMI 1640 medium to obtain a concentration 10 times the final: 1.286 mill/ml. Than separate vessels were prepared for each animal. The cell tube for animal number 3 received Colchicine as indicated in the table on day 0 one hour before the cells were injected.

Each animal received about 0.7 ml of the cell suspension of 1.286 mill/ml or 0.9 million cells injected to each.

**Experiment 18, Table 1: Treated mice:**

| Number, g mouse weight | Treatment* | Dose from 20 mM in DMSO (Etoposide: 40 mM) | Estimated µM in extra-cellular fluid. | Time for first treatment | µM in Ehrlich cells for 1 h. |
|---|---|---|---|---|---|
| 1, 29.4 | 4-OH-OPB | 1 .05 µl | 2.5 | day 1 | |
| 2, 27.8 | Etoposide + 4-OH-OPB | 1.69 µl¤ and 0.99 µl | 8.5 and 2.5 | day 1 | |
| 3, 26.5 | Colchicine | 0.212 µl# | 0.56 | day 0 | 6.1 |
| 4, 26.2 | Control | | | not treated | |

| | | | | | |
|---|---|---|---|---|---|
| ¤: This solution in DMSO of Etoposide was 40 mM. *: The compounds in doses indicated in the next column were solved in 0.7 ml RPMI and given as intraperitoneal injections on days indicated in the next table. The injection on day 0 contained cells, see #. #: Calculated as equivalent to 4.5 mg for a 70 kg human given into cell suspension first time and repeated as intraperitonal injection 3 times a week. The cell suspension was prepared with Colchicine 1 hour before the injection into peritoneum of mouse number 3 on day zero. | | | | | |

**Experiment 18, Table 2: Calendar for injections, day number: →**

| Mouse#: ↓ | 0 | 1 | 3 | 4 | 6 | 8 | 11 | 13 | 14 | 16 |
|---|---|---|---|---|---|---|---|---|---|---|
| 1 | | 4-OH-OPB | 4-OH-OPB | 4-OH-OPB | 4-OH-OPB | 4-OH-OPB | 4-OH-OPB | 4-OH-OPB | Ascites 0502> | |
| 2 | | 4-OH-OPB+ Etoposide | 4-OH-OPB | 4-OH-OPB | 4-OH-OPB | 4-OH-OPB | 4-OH-OPB | 4-OH-OPB | Ascites 0602? | Ascites 0602> |
| 3 | Colchicine | | Colc hicine | Colchicine | Colchicine | Colchicine | Colchicine | Colchicine | Ascites 0502? | Ascites 0502> |
| 4 | | | | | | | | | Ascites 0502> | |

| | | | | | | | | | | |
|---|---|---|---|---|---|---|---|---|---|---|
| >: Killed because of signs of ascites being observed, especially for number 4, the control. | | | | | | | | | | |

### Dosages:

Colchicine: 434 µl Eagles medium was filled onto two tubes. 9.7 µl 20 mM Colchicine in DMSO was added to the first tube. After mixing the same volume was transferred from this tube to the last tube. The content of this last tube was injected intraperitonally to mouse number 3 on the day indicated in the table above.

4-OH-OPB: 478 µl Eagles medium was pipetted into each of tree tubes and it was added 22,25 µl of 20 mM 4-OH-OPB in DMSO to the first tube. After mixing the content of this first tube the same volume and 23.6 µl was transferred from this tube to each of the next two tubes. The content of the last two tubes was injected intraperitonally to each of the mice number 1 and 2 respectively. Time calendar of injections is indicated in the Table 2 above and the start in Table 1.

**Experiment 18, Table 3: Results:**

| Mouse number :↓ | weight gain in % | day 14 | day 16 | Animal weight and findings on section. |
|---|---|---|---|---|
| 1 | 32.3 | Ascites 0502> | | 38.9 g. 0.1 ml ascites with Ehrlich cells. Lens sized tumour in left side of abdominal wall. Normal organs. |
| 2 | 30.9 | Ascites 0602? | Ascites 0802> | 36.4 g. 1 ml ascites with Ehrlich cells. Lens sized tumour in left abdominal wall. Normal organs. |
| 3 | 23.8 | Ascites 0502? | Ascites 0802> | 32.8 g. 1.5 ml ascites with Ehrlich cells. Lens sized tumour in left abdominal wall. Normal organs. 0.5 ml subcutaneous fluid |
| 4 | 42.4 | Ascites 0502> | | 37.3 g. 4 ml ascites with Ehrlich cells. 2 x 3 mm tumour in left abdominal wall. Normal organs. Cells frozen at - 196 degrees. |

| | | | | |
|---|---|---|---|---|
| >: Killed because of signs of ascites being observed, especially for number 4, the control. | | | | |

### Conclusion:

*The answer of the first question: "Will mice transplanted with Ehrlich carcinoma in peritoneum be rescued even if the treatment is started the day after transplantation if not only 4-OH-OPB is given to the animal, but a combination of Etoposide and 4-OH-OPB, the last one in a dose not effective alone?" is NO".*

This result corresponds to the results of *in vitro* experiment indicating that Etoposide did not completely inhibit sparsely seeded polyoma transformed S100T1 cells. However, an *in vitro* experiment studying combined effect of the two inhibitors has not been undertaken.

*As expected: A control animal that received a moderate dose of 4-OH-OPB the day after transplantation conformed earlier established findings of missing cure.*

The answer of the last question: " Will Colchicine alone stop settlement of transplanted Ehrlich tumour cells when mixing the drug with the transplanted cells before injecting them as done with 4-OH-OPB?" is also NO. The concentrations, thought to be quite high, did not provide complete protection against the development of transplantable Ehrlich tumour Table 1, Fig. 1).

This result corresponds to *in vitro* experiments with Colchicine showing no complete clonal inhibition for sparsely seeded cells. This is in contrast to what was found for 4-OH-OPB that effectively inhibited such growth.

### Summary and conclusions mainly based on the last 5 experiments:

### Based on experiments including number 14, this hypothesis might be appropiate:

*Close cell to cell interaction between tumour cells would abrogate the anticlonal effect of 4-OH-OPB. If the distance between tumour cells increases, a weaker interaction is expected between these related cells. Even if many other normal cells, which were unrelated to the tumour cells were present in the same area, the effect of 4-OH-OPB on tumour cells would not be abrogated.*

*Thus, interactive effects of related cells in close contact might block postulated cell receptors for 4-OH-OPB.*

### Based on experiment number 15 the following conclusion seems possible:

4-OH-OPB was inhibiting clonal growth at the concentrations tested, 2 and 20 µM. Colchicine did not inhibit clonal growth at concentrations from 0,01-0,1 µM. Diphenhydantoin did not inhibit clonal growth significantly at concentrations 10-100 µM.

Podophyllotoxin inhibited significantly at 0.3 µM, but had probably some few remaining clones. There was no significant inhibition at 0.03 µM.

Piroxicam possibly inhibited significantly at 50 µM, but not at 5 µM.

Diclofenac showed no significant inhibition at 10-100 µM. However, a probable significant increase in clonal growth was observed at 10 µM. This might indicate a cancer promoting ability having consequences for its use as a human drug.

Ibuprofen showed insignificant inhibition at 100 µM. Clonal inhibition at 1000 µM might not be real since it involved the addition of 30 µl of DMSO to the well. That might be toxic (see next experiment).

Naproxen probably inhibited significantly at 500 µM, but DMSO toxicity might interfere as mentioned for Ibuprofen.

Acetylsalicylic acid possibly inhibited significantly, but not completely at 100 µM. At 1000 µM the inhibition is complete, but might partially be caused by DMSO as mentioned for Ibuprofen.

### Based on experiment number 16 the following conclusion seems possible:

The results were read after two or tree weeks. The results read after 3 weeks were not included in this abbreviated conclusion since the initial medication was not continued.

The results seemed most relevant when read after 2 weeks.

Table 1 summarising the significance of results for Colchicine read after 14 days:

**Experiment 16, summarising Table 1:**

| | | 0.1 µM | 0.22 µM | 0.46 µM | 1 µM |
|---|---|---|---|---|---|
| 1# | Colchicine ¤ | doubtful | | | doubtful |
| 2* | Colchicine ¤ | doubtful | | | possible |
| 3* | Colchicine § | possible | possible | | |
| 4* | Colchicine§ | | | possible | probable |

| | | | | | |
|---|---|---|---|---|---|
| #: Solved in DMSO 8 days in beforehand, but were stored frozen. *: These were solved in DMSO the same day. ¤: Lot 60K1932 §: Lot 28H1229 | | | | | |

***This table shows clearly that there might be difference between the two Colchicine lots. The lot 60K1932 was not so effective and storage and freezing further reduced the effect.***

***It might be Possible that therapeutic doses of Colchicine could inhibit cloning But the effect is not as good as of 4-OH-OPB.***

For Podophyllotoxin the significance of results are summarised in this Table 2 when read after 14 days:

**Experiment 16, summarising Table 2:**

| | | 0.03µM | 0.065 µM | 0.139 µM | 0.3 µM | 3 µM |
|---|---|---|---|---|---|---|
| 5# | Podophyllotoxin¤ | | | | probable | probable |
| 6* | Podophyllotoxin¤ | no | possible | | | |
| 7* | Podophyllotoxin¤ | | | probable | possible | |

| | | | | | | |
|---|---|---|---|---|---|---|
| #: Solved m DMSO 8 days m betorehand, but were stored frozen. *: These were solved in DMSO the same day. ¤: Lot 12k1562 | | | | | | |

*Storage in freezer for 8 days did not reduce activity, but might increase the activity instead.*

*Dosage within a range that might be therapeutic revealed a probable anti-clonal activity that was not as good as for 4-OH-OPB.*

*Dosage within a range that might be therapeutic revealed a possible anti-clonal activity that was not as good as for 4-OH-OPB and less significant than results read after 2 weeks.*

The significance of results for Ibuprofen, Naproxen, Acetylsalicylic acid and 4-OH-OPB read after 14 days, Table 3:

**Experiment 16, summarising Table 3:**

| | | 0,67 µM | 6.7 µM | 108 µM | ca. 223 µM | 464 µM |
|---|---|---|---|---|---|---|
| 8 | Ibuprofen | | | | possible | doubtful* |
| 9 | Naproxen | | | doubtful | possible | |
| 10 | Acetyl salicylic acid | | | | probable | doubtiul* |
| 11 | 4-OH-OPB | yes | yes | | | |

| | | | | | | |
|---|---|---|---|---|---|---|
| *: Doubtful because the solvent DMSO had a concentration in these wells that might be inhibiting. These high DMSO concentrations make new tests necessary. | | | | | | |

*The DMSO concentration for compounds number 8 and 10 could interfere as a clonal growth inhibitor since the concentrations were above about 1% as found in a later experiment.*

*The results concerning number 9, Naproxen, could be accepted as possibly significant since the concentration of DMSO did not exceed a critical level.*

*The results concerning highest concentrations of Ibuprofen and Acetyl salicylic acid, however, could not be accepted since DMSO itself might inhibit. But these drugs possibly or probably showed significant clonal inhibition when added to the cultures less concentrated*

*The results showed that Acetylsalicylic acid probably had a colony inhibiting activity for dosage within a range that might be therapeutic. For Naproxen and Ibuprofen the same activity possibly existed.*

*The control 4-OH-OPB was clearly better than any other.*

### Based on experiment number 17 the following conclusion seems possible:

*None of the tested inhibitors (Colchicine, Podophyllotoxin, or Etoposide) did inhibit clonal cell growth as effectively as 4-OH-OPB. All three seemed to allow the growth of a few small clones in sparsely seeded locations of the agar.*

*Etoposide seented to promote the best inhibition of cells in crowded areas. Therefore, this might be the best candidate for combination with 4-OH-OPB during short-term periods of treatment.*

*It is known that Colchicine is tolerated during longer periods, an advantage if needed in treatment.*

*Long time toxicity for Podophyllotoxin and Etoposide is known to be high and agrees with the significant growth inhibition found in crowded areas.*

*The tested growth inhibitors consisted of two groups, the inhibitors that inhibited cell growth in both high and low cell density locations, the Podophyllotoxin group, and the inhibitors that only inhibited clonal growth in low density areas, 4-OH-OPB and Colchicine. Especially for medium 4-OH-OPB concentrations there is good growth of colonies in high-density areas, but no growth in low-density areas.*

*Colchicine did not inhibit the high-density areas significantly. Only high dose seemed to inhibit significantly the growth of cells in low-density areas. But in this experiment no complete clonal inhibition could be observed in these areas.*

*Both compounds in the Podophyllotoxin group behaved similarly, growth in high-density areas was significantly inhibited, and the low-density areas, that also showed inhibition, failed to demonstrate complete clonal inhibition.*

*These findings indicate that Podophyllotoxin or Etoposide might be toxic also to cells in densely populated areas. This is in agreement with known relatively high toxicity in the body. In addition they inhibit clonal growth in sparsely populated areas, but probably not completely as needed for effective control of metastases.*

*Testing DMSO resulted in approximately unaffected growth inhibition up to about 1% concentration in soft agar cultures. 29.2 µl*/*700 µl or 4% inhibited formation of clones, but not completely.*

The Table 1 below indicates what is supposed to become the main advantages or disadvantages of the tested substances if used in cancer treatment (Etoposide already is in use):

**Experiment 17, summarising Table 1:**

| | Toxicity for body or dense cell populations | Inhibition of tumour cell colonies* | Control of metastatic cells leaving tumour# | Control of settlement of metastatic cells ¤ | Tolerating long dural of treatment |
|---|---|---|---|---|---|
| Colchicin | none or small | Possibly, but only partly | probably not | partly | yes |
| 4-OH-OPB | none or small | Possibly, but only partly | yes | completely | yes |
| Etoposide | yes | yes, but not completely | probably not | partly | no |
| Podophyllotoxin | yes | yes, but not completely | probably not | partly | no |

| | | | | | |
|---|---|---|---|---|---|
| *: Colonies or tumours like metastases or primary tumours greater than a certain size. #: The ability of cells to leave a tumour, grow and infiltrate locally or grow in sparsely seeded areas. ¤: Settlement of single cells or very few cells spread to distant sites from a tumour or tumour cell rich area. | | | | | |

*In situations where clones are about to grow from single cells or oligocellular clumps, as supposed to happen if effective anti-clonal treatment is not jet started, it might be advantageous to combine 4-OH-OPB with Etoposide. Toxicity is limiting Etoposide from being applied for more than a few days each time. It is expected, however, that 4-OH-OPB would be tolerated for much longer periods.*

*The situation for Colchicine is not clear after this experiment since there is doubt about its effect on clonal growth in sparsely seeded areas.*

*When bigger tumours appear, either palpable or detected by x-rays or similar techniques, 4-OH-OPB alone is probably not advisable alone. However, 4-OH-OPB would be expected to prevent metastatic cells leaving the tumour. This is especially important during surgery, but could be valuable also during chemical treatment or irradiation.*

*Experiments in mice transplanted with Ehrlich's carcinoma subcutaneously did indicate stop in tumour cells leaving the tumour cell deposits when given 4-OH-OPB. In addition this treatment will probably stop the settlement of tumour cells reaching distant organs.*

### Based on experiment number 18 the following conclusion seems possible:

*The answer of the first question: "Will mice transplanted with Ehrlich carcinoma in peritoneum be rescued even if the treatment is started the day after transplantation if not only 4-OH-OPB is given to the animal, but a combination of Etoposide and 4-OH-OPB, the last one in a dose not effective alone? " is NO".*

*This result corresponds to the results of in vitro experiment indicating that Etoposide did not completely inhibit sparsely seeded polyoma transformed S100T1 cells. However, an in vitro experiment studying combined effect of the two inhibitors has not been undertaken.*

*As expected: A control animal that received a moderate dose of 4-OH-OPB the day after transplantation confirmed earlier established findings of missing cure.*

*The answer of the last question: " Will Colchicine alone stop settlement of transplanted Ehrlich tumour cells when mixing the drug with the transplanted cells before injecting them as done with 4-OH-OPB?" is also NO. The concentrations, thought to be quite high, did not provide complete protection against the development of transplantable Ehrlich tumour.*

This result corresponds to in vitro experiments with Colchicine showing no complete clonal inhibition for sparsely seeded cells. This is in contrast to what was found for 4-OH-OPB that effectively inhibited such growth.

### Experiment 19

### The effect of Mycophenolic acid and Cyclophosphamide on S100T1 cells in soft agar culture.

Question: What is the effect of Mycophenolic acid and Cyclophosphamide on cloning in soft agar of these cells?

The aim of this experiment was to see if the addition of the two drugs mentioned (Table 1) inhibited or stimulated clonal growth when added to the polyoma virus transformed cell line S100T1 of BHK21/c13 cells seeded in the top soft agar layer.

Agar cultures were prepared in wells of Falcon 24 well tissue culture plate. Each contained two agar layers of 0.3 ml. The bottom layer contained 2.16% Sigma agarose, type II, A-6877 and the top layer 120000 cells in 0.8% ultra low gelling temperature Sigma agarose type IX, A-5030. The medium contained Eagles MEM and 10% foetal bovine serum (FBS).

The compounds were first solved in DMSO in 20 mM concentration and then diluted in medium. The top layer needed 60-90 minutes in the refrigerator for solidification before incubation in 37 ° C with 5% CO₂ atmosphere.

The cell gradient was made by adding all the cells in the last 75 µl of the 300 µl top layer in the periphery of the well.

The resulting growth was photographed using an inverted Olympus microscope CK 40, 4 x-objective and Olympus Camedia digital camera C-3040 zoom.

**Experiment 19, Table 1: Estimated concentration after average human dose:**

| No | Name | MW | Human dose, mg | µM in 20 litres* |
|---|---|---|---|---|
| 1 | Mycophenolic acid | 320.3 | 1000 mg | 156.1 µM |
| 2 | Cyclophosphamide | 279,1 | 500 mg | 89.6 µM |
| 3 | 4-OH-OPB | 340.4 | 100 mg | 14.7 µM |

| | | | | |
|---|---|---|---|---|
| *: If solved in 20 litres extracellular fluid of a man of 70 kg. The concentrations in experiment, see Fig 1. | | | | |

Results after 48 hours incubation using high, medium or low concentration of the compunds tested are shown in Fig.1 and summarised in Table 2.

**Experiment 19, Table 2:**

| Concentration → | low | medium | high* |
|---|---|---|---|
| Mycophenolic ac, H | (+) | (+) | (+) |
| Mycophenolic ac, L | (+) | (+) | (+) |
| Cycloph. amide, H | ++(+) | ++(+) | ++ |
| Cyclophosphamide, L | ++ | ++ | ++ |
| 4-OH-OPB, H | ++ | ++ | ++ |
| 4-OH-OPB, L | - | - | - |
| DMSO control, H | ++ | ++ | ++ |
| DMSO control, L | ++ | ++ | ++ |
| Control, H | ++ | - | |
| Control, L | ++ | - | |
| | Control after 48 h | Control at 0 hours | |

| | | | |
|---|---|---|---|
| *: See figures for exact concentrations. (H-L): H=High cell density part of the cell gradient in agar, L=Low cell density part of the cell gradient in agar | | | |

***Results and Conclusion:** The growth of polyoma virus transformed BHK21 C13 cells might be influenced by adding the compounds under test to the cultures. The cultures were observed in both sparsely and densely seeded areas of the soft agar cultures (**Fig. 1**). The inhibition of cellular growth in these areas was significant at all concentrations of 4-OH-OPB when observing the sparsely seeded areas. However, there was no significant inhibition in the densely seeded areas.*

*The tested substances, Mycophenolic acid and Cyclophosphamide showed different results. The first one, Mycophenolic acid, showed significant growth inhibition both in densely and sparsely seeded areas of the agar cultures at all concentrations.*

*Cyclophosphamide, however, showed no inhibition ofgrowth in any of these areas. But the opposite, a stimulation of clonal growth probably was significant. This was observed in densely seeded areas of wells with the two smallest concentrations of the compound.*

*The results indicated that Mycophellolic acid probably would have both a toxic effect and an inhibiting effect on metastatic growth and carcinogenesis and the profile corresponded to that of a typical cytostatic drug.*

*The Cyclophosphamide, however, did not show an inhibiting effect even after relatively large doses compared to the amount administered to people. Since this is not what is expected if it was active in culture. In the organism, Cyclophosphamide is activated in the liver. This did obviously not happen in cell culture. The moderate increase in clonal growth observed in cultures treated by Cyclophosphamide could in theory become a problem if the transformation to active substance in the liver is too slow or inhibited in some way and if the concentration before activation reaches values that could liberate and*/*or stimulate existing metastatic cells to initiate malignant growth.*

*DMSO did not show growth inhibition in concentrations corresponding to those administered to the cultures along with the test substances. The highest concentration of Mycophenolic acid received more DMSO than the highest concentration added to the DMSO control because of the extra DMSO necessary for solving the compound. It is possible that at least part of the inhibition of growth observed in well with highest Mycophenol concentration could be caused by DMSO. But since wells with both medium and low concentration of Mycophenolic acid showed about the same degree of inhibition of clonal growth it is unlikely that this is a DMSO effect.*

### Experiment 20

### An experiment to see if sparsely seeded S100T1 cells are inhibited more efficient than densely seeded cells and to see which concentration of 4-OH-OPB that is needed.

Question: Different concentrations of cells will grow differently when treated with the same amount of 4-OH.OPB. What are the limits?

In this experiment the inhibitor was added to the bottom layer in order to get an easier way for making the cell density gradient. One side of the Falcon 24 well plate was elevated 12 degrees in the refrigerator for 60-90 minutes to solidify the bottom layer in an oblique position. When then solidifying the cell containing top soft agar layer in horizontal position, the cell gradient constantly became good across the well.

Ultra low gelling temperature agarose (Type IX-A, Sigma A-2576, lot. 19H0821) was used both as bottom and top layer. Dilutions of 4-OH-OPB was added to this bottom layer before solidification in the refrigerator. Another 90 minutes were spent in the refrigerator to consolidate the soft agar layer with cells in a horizontal position. Both layers contained 0.8% agarose, Eagles MEM and 10% FBS.

**Experiment 20, Table 1: Clonal growth after 48 hours, see Fig. 1:**

| Conc. of 4-OH-OPB | 1 µM | 6 µM | 18 µM | Cell number |
|---|---|---|---|---|
| Low H* | +-# | +-# | +-# | 25000 (42'/ml) |
| Low L * | +-# | +-# | +-# | 25000 (42'/ml) |
| Medium H | ++(+) | + | + | 75000 (125'/ml) |
| Medium L | - | - | - | 75000 (125'/ml) |
| High H | +++ | + | + | 225000 (375'/m |
| High L | - | - | - | 225000 (375'/m |

| Cell density of control | LOW | medium | High | |
|---|---|---|---|---|
| Control H | | ++ | | 75000 (125'/ml) |
| Control L | | ++ | | 75000 (125'/ml) |
| Control day 0 | - | - | - | |

| | | | | |
|---|---|---|---|---|
| #: Very small increase in cell size, not new clones. *:(H-L): H=High cell density part of the cell gradient in agar, L=Low cell density part of the cell gradient in agar | | | | |

***Discussion and conclusion:** In this experiment of cross-titration of cell number and dose of 4-OH-OPB, there was found a very strong inhibition of growth in low density areas of high and medium concentration of S100T1 cells (3 75000 or 125000 cells*/*ml) with a small dose of 4-OH-OPB. However, the smallest number of seeded cells, 42000 cells*/*ml, did not grow even in dense areas of the cell gradient if treated. Only the cell size might have increased a little.*

*The control with medium number of cells and no inhibitor grew well even in sparsely seeded areas and represented the basis for the conclusion that even 1 µM 4-OH-OPB had a tremendous effect even on wells with large cell numbers, but only in the zone with few cells.*

*In order to getting fast results, experiments intended to screen for anti growth effects need to have cell numbers above 42000, probably between 125000 and 375000 cells*/*ml. In this experiment the conclusion that an anti clonal effect is present at a concentration of 4-OH-OPB of 1 µM seems valid. No significant growth inhibition on denser populated areas of the agar was observed in wells with 1-18 µM 4-OH-OPB.*

*Therefore the toxicity on the organism is expected to be low. Spleen cells producing anti-sheep red cell antibodies (see Experiment 10) could be an exception. But it is thought that the growth inhibiting effect is limited to immunity cells that were of the same kind, such as those with activity against red sheep cells. But if such cells are gathered in follicles with many collocated cells of the same specificity it is expected that the effect would be abrogated just as in densely seeded areas of the agar or when tumour cells were collocated in clumps larger than a few cells. The treated animals do not show any sign of organ toxicity or destruction of the immune system or other organs. However, since the anticipated growth of the immune cells was inhibited new or recent infections might be harmful under such treatment. Development of new clones also is important in the organogenetic period of foetal development and therefore treatment is expected to be harmful for the foetus.*

*We expect 4-OH-OPB to be a substance capable of inhibiting growth of single or very few cells even if these cells are close to, but different from other cells that were highly collocated. This view is in concordance with the observations made when treating mice transplanted with Ehrlich carcinoma.*

*In the experiment cells in the top layer is expected to have about the same access to the inhibitor from the bottom layer in the same well. Therefore, it is hard to explain the differences of clonal growth between cells in densely and sparsely seeded areas as caused by different access to the compound.*

*Low cell numbers are easily inhibited to grow. But the control shows much better growth in sparsely seeded areas than in treated cultures. Therefore, the treatment is to be blamed for the specific growth inhibition in sparsely seeded areas (**Fig. 1**).*

*But there might be a combined effect of the drug and low density seeding. The threshold of clonal growth is normally at a certain level. The organism probably protects itself by this mechanism. However, if cells obtain greater capability to grow, cancer might develop since development of new clones might end up with some having carcinogenic properties.*

*It is expected that the candidate drug, 4-OH-OPB, will both inhibit the first clonal growth of a cancer and cells released from an established malignant tumour that is capable of forming metastases.*

*The inhibiting effect on densely seeded areas seemed to be abscent for low 4-OH-OPB doses (1µM) and small or moderate for higher doses (6-18 µM).*

*In this experiment there might be a stimulation of clonal growth at 1-6 µM, but this is not found in other experiments. Therefore the effective therapeutic dose would probably be less than 6 µM, and this dose would not affect established tumour or normal organs rich in cells with the already mentioned exceptions.*

### Experiment 21

### The effect of Actinomycin D, Cytarabin and Fluorouracil on S100T1 cells in soft agar culture.

Question: What is the effect of the cytostatics Actinomycin D, Cytarabin and Fluorouracil on cloning in soft agar of these cells?

This time the cell number seeded in each well is 117000. Tilting the plate 12 degrees when solidifying the bottom layer with the drug and casting the top layer with cells horizontally made a stable cell gradient.

*Results, see* *Fig. 1* *and Table 1:*

**Experiment 21, Table 1:**

| | Low | Medium | High | |
|---|---|---|---|---|
| Actino-mycin D, H* | ++ | ++ | + | Good, but probably no complete |
| Actino-mycin D, L* | + | (+) | ((+)) | inhibition of clonal growth at low toxicity |
| Cytarabin, H | ++ | ++ | (+) | Incomplete inhibition of clonal growth |
| Cytarabin, L | + | + | (+) | and significant toxicity |
| Fluorouracil, H | ++ | ++ | ++ | Rel. moderate inhibition of clonal growth and |
| Fluorouracil L | + | + | + | low toxicity independent of dosage |
| 4-OH-OPB, H | ++(+) | ++(+) | + | Arrest in clonal growth in sparsely seeded areas without significant toxic effect on |
| 4-OH-OPB, L | +- | - | - | densely seeded areas at medium and low dose |

| | Control after 48 h | Control before incubation | | |
|---|---|---|---|---|
| Control, H | +++ | - | | Control shows excellent growth in H and L |
| Control, L | +++ | - | | Negative by definition before incubation |

| | | | | |
|---|---|---|---|---|
| *: H: Area with high cell density, L: Area with low cell density +: Klonal growth present. - and +- considered negative, then: (+),+,+(+),++,++(+),+++ in increasing ability to clonal growth. | | | | |

### Conclusion:

*The results showed that Actinomycin was similar to 4-OH-OPB. But no absolute clonal inhibition of sparsely seeded cells was observed in this experiment. Therefore, the drug would probably not be able to completely inhibiting growth of metastatic cells.*

*Cytarabin would probably have a significant toxic effect at dosage that showed significant inhibition of clonal growth.*

*Fluorouracil showed a relatively moderate clonal inhibition and low toxicity. The effect was not dependent on concentration within the range tested.*

### Experiment 22

### The effect of analogues of 4-OH-OPB, #4, #6 and #7 on S100T1 cells in soft agar culture.

Question: What is the effect of analogues of 4-OH-OPB, #4, #6 and #7 on cloning in soft agar of these cells?

Experiment 22, Table 1: Available 4-OH-OPB analogues to be tested in experiments 22-25:

| No | |
|---|---|
| 1 | p-hydroxy-azobenzene A |
| 2 | 2-Butyl-2-hydroxy-N-(4-hydroxy-phenyl)-N'-phenyl malonamide |
| 3 | 2-oxo-hexanoic acid phenylamide |
| 4 | 2-Butyl-N-(4-hydroxy-phenyl)-N'-phenylmalonamide |
| 5 | 4-OH-PBOMe |
| 6 | 1,2-diphenyl-4-hydroxy-4-[2-(phenylsulfinyl)ethyl]-3,5-pyrazolidinedione. |
| 7 | 4-(1-butyl)4-hydroxy-1-(4-methoxyphenyl)-2-phenyl-3,5-pyrazolidinedione |
| 8 | 1,2-diphenyl-4-hydroxy-4-(4-methylphenyl)-3,5-pyrazolidinedione |
| 9 | 4-benzyl-1,2-diphenyl-4-hydroxy-3,5-pyrazolidinedione |
| 10 | 4-hydroxy-4-aldehyd-1,2-diphenyl-pyrazolidinedione |
| 11 | 4-butyl-4-hydoxy-1,2-diphenyl-3,5-pyrazolidnedione |

In this experiment number 4, 6 and 7 were tested.

The indicated compounds were added to soft agar cultures of the polyoma virus transformed cell line S100T1 of BHK21/c13 cells (Table 1). Agar cultures were prepared in wells of a Falcon 24 well tissue culture plate. The wells contained two agar layers of 0.3 ml. The top layer contained 117000 cells in 0.8% ultra low gelling temperature Sigma agarose type IX, A-5030.in Eagles MEM with 10% foetal bovine serum (FBS). The bottom medium was identical, but without cells.

The compounds were first solved in DMSO in 20 mM concentration and then diluted in bottom medium. The agar layers needed 60-90 minutes in the refrigerator for consolidation before incubation at 37 ° C in 5% CO₂ atmosphere.

At the first consolidation the plate was tilted 12 degrees to one side using a "China pen" under one of the long sides of the plate. Next time, when casting cells in top agar layer the plate was positioned horizontally in the refrigerator in order to produce the cell gradient.

The compounds were added to the bottom medium, diluted in the same medium. Therefore, the control did not receive anything.

The cells had no clumps after having been trypsin/versene treated for 16 minutes. The cell suspension was centrifuged after the addition of serum at low speed (1100 rpm for 6 minutes) and suspended in FBS.

### Results:

This time the growth in dense areas of the control 4-OH-OPB was absent in high concentration of the inhibitor and doubtful in medium concentration (Table 2, Fig. 1).

The best inhibitor of clonal growth of sparsely seeded cells was #6 1,2-diphenyl-4-hydroxy-4-[2-(phenylsulfinyl)ethyl]-3,5-pyrazolidinedione. However, at low concentration no effect was observed. Therefore, if this compound should become an anti-cancer drug in the future, the dose is expected to be higher than for 4-OH-OPB. #4, 2-Butyl-N-(4-hydroxy-phenyl)-N'-phenylmalonamide showed an effect, but even higher concentration was needed than for #6.

Also #7, 4-(1-butyl)-4-hydroxy-1-(4-methoxyphenyl)-2-phenyl-3,5-pyrazolidinedione, might have a small similar effect.

**Experiment 22, Table 2: Test results:**

| No | | Low | Medium | High |
|---|---|---|---|---|
| 4 H* | 2-Butyl-N-(4-hydroxy-phenyl)-N'-phenylmalonamide A | ++ | ++ | ++ |
| 4 L* | 2-Butyl-N-(4-hydroxy-phenyl)-N'-phenylmalonamide B | ++ | ++ | ((+)) |
| 6 H | 1,2-diphenyl-4-hydroxy-4-[2-(phenylsulfinyl)ethyl]-3,5-pyrazolidinedione. A | ++ | ++ | ++ |
| 6 L | 1,2-diphenyl-4-hydroxy-4-[2-(phenylsulfinyl)ethyl]-3,5-pyrazolidinedione B | ++ | ((+)) | - |
| 7 H | 4-(1-butyl)-4-hydroxy-1-(4-methoxyphenyl)-2-phenyl-3,5-pyrazolidinedione A | ++ | ++ | ++ |
| 7 L | 4-(1-butyl)-4-hydroxy-1-(4-methoxyphenyl)-2-phenyl-3,5-pyrazolidinedione B | ++ | ++ | (+) |
| H | 4-OH-OPB A | ++ | (+) | - |
| L | 4-OH-OPB B | - | - | - |
| H | Control A | | | +++ |
| L | Control B | | | ++ |
| H | 0-control A | | | - |
| L | 0-control B | | | - |

| | | | | |
|---|---|---|---|---|
| *: H: Area with high cell density, L: Area with low cell density +: Clonal growth present - and +- considered negative, then: (+),+,+(+),++,++(+),+++ in increasing ability to clonal growth. | | | | |

### Conclusion:

*This time the growth in dense areas of the control 4-OH-OPB was absent in high concentration of the inhibitor and doubtful in medium concentration. Compared to the other experiments, the growth was less this time. The reason was unknown. Growth in other wells seemed to be unaffected.*

*The results of this experiment were accepted since there was a good difference between growth in sparsely and dense areas when treating cells with low concentrations of the control inhibitor 4-OH-OPB.*

*The best inhibitor of clonal growth of sparsely seeded cells was* #*6 1,2-diphenyl-4-hydroxy-4-[2-(phenylsulfinyl)ethyl]-3,5-pyrazolidinedione. However, at low concentration no effect was observed. Therefore, if this compound should become an anti-cancer drug in the future, the dose is expected to be higher than for 4-OH-OPB. #4, 2-Butyl-N-(4-hydroxy-phenyl)-N'-phenylmalonamide showed an effect, but even higher concentration was needed than for #6.*

*Also #7,4-(1-butyl)-4-hydroxy-1-(4-methoxyphenyl)-2-phenyl-3,5-pyrazolidinedione, might have a small similar effect.*

### Experiment 23

### The effect of analogues of 4-OH-OPB, #1, #2 and #3 on S100T1 cells in soft agar culture.

Question: What is the effect of analogues of 4-OH-OPB? What is the effect of number 1, 2 or 3 (Table 1) on cell cloning in soft agar?

**Experiment 23, Table 1: Available 4-OH-OPB analogues to be tested in experiments 22-25:**

| No | |
|---|---|
| 1 | p-hydroxy-azobenzene A |
| 2 | 2-Butyl-2-hydroxy-N-(4-hydroxy-phenyl)-N'-phenyl malonamide |
| 3 | 2-oxo-hexanoic acid phenylamide |
| 4 | 2-Butyl-N-(4-hydroxy-phenyl)-N'-phenylmalonamide |
| 5 | 4-OH-PBOMe |
| 6 | 1,2-diphenyl4-hydroxy-4-[2-(phenylsulfinyl)ethyl]-3,5-pyrazolidinedione. |
| 7 | 4-(1-butyl)-4-hydroxy-1-(4-methoxyphenyl)-2-phenyl-3,5-pyrazolidinedione |
| 8 | 1,2-diphenyl-4-hydroxy-4-(4-methylphenyl)-3,5-pyrazolidinedione |
| 9 | 4-benzyl-1,2-diphenyl-4-hydroxy-3,5-pyrazolidineilione |
| 10 | 4-hydroxy-4-aldehyd-1,2-diphenyl-pyrazolidinedione |
| 11 | 4-butyl-4-hydoxy-1,2-diphenyl-3,5-pyrazolidnedione |

These analogues are: #1, #2 and #3 (Table 1). In addition Methotrexate (Emthexat) MW 454,4 is tested

This time the cell number seeded in each well is 180000. Tilting the plate 12 degrees when solidifying the bottom layer with the compound to be tested and casting the top layer with cells horizontally made the cell gradient

**Experiment 23, Table 2: Results also in Fig. 1:**

| No | | Low | Medium | High |
|---|---|---|---|---|
| #1 | p-hydroxy-azobenzene A | +++ | +++ | +(+) |
| #1 | p-hydroxy-azobenzene B | ++ | ++ | +- |
| #2 | 2-Butyl-2-hydroxy-N-(4-hydroxy-phenyl)-N'-phenyl malonamide A | ++(+) | ++ | ++ |
| #2 | 2-Butyl-2-hydroxy-N-(4-hydroxy-phenyl)-N'-phenyl malonamide B | ++ | ++ | - |
| #3 | 2-oxo-hexanoic acid phenylamide A | ++(+) | ++(+) | ++ |
| #3 | 2-oxo-hexanoic acid phenylamide B | ++(+) | ++(+) | ++ |
| | Methotrexate A | ++ | ++ | +(+) |
| | Methotrexate B | + | +(+) | + |
| | 4-OH-OPB A | ++(+) | + | (+) |
| | 4-OH-OPB B | - | - | - |
| | | | Control before incubation | Control after 48 hours |
| | Control A | | - | +++ |
| | Control B | | - | +++ |

| | | | | |
|---|---|---|---|---|
| *: H: Area with high cell density, L: Area with low cell density +: Klonal growth present. - and +- considered negative, then: (+),+,+(+),++,++(+),+++ in increasing ability to clonal growth. | | | | |

### Results:

The 4-OH-OPB analogues 1, 2 and 3 (Table 1) were tested in agar cultures as described in the last experiment and showed results as seen in Table 2.

### Conclusion:

*The control, the clone inhibitor 4-OH-OPB, showed some inhibition of total growth in densely seeded areas of the soft agar culture when added in high or medium concentrations (**Fig. 2**, Table 2). However, clonal growth was present even in wells with high concentrations of the compound but only in densely seeded areas. All wells with this Compound showed complete inhibition of clonal growth in sparsely seeded areas.*

*The compound #2: 2-Butyl-2-hydroxy-N-(4-hydroxy-phenyl)-N'-phenyl malonamide and probably also #1, p-hydroxy-azobenzene had an effect very similar to 4-OH-OPB, but at at least 66 times higher concentration (Table 2,* *Fig. 1**). They showed an effective clone inhibition selectively in sparsely seeded areas of the polyoma virus transformed cells S100T1 in soft agar cultures for high concentrations of the compounds. The possibility of using these two analogues in treatment of diseases would very much depend on toxicological factors.*

*#3, 2-oxo-hexanoic acid phenylamide and methotrexate did not show significant growth inhibition at all*

### Experiment 24

### The effect of analogues of 4-OH-OPB, #5, #8 and #9 on S100T1 cells in soft agar culture.

Question: What is the effect of analogues of 4-OH-OPB, #5, #8 and #9 (Table 1) on cloning in soft agar of these cells?

Experiment 24, Table 1: Available 4-OH-OPB analogues to be tested in experiments 22-25:

| No | |
|---|---|
| 1 | p-hydroxy-azobenzene A |
| 2 | 2-Butyl-2-hydroxy-N-(4-hydroxy-phenyl)-N'-phenyl malonamide |
| 3 | 2-oxo-hexanoic acid phenylamide |
| 4 | 2-Butyl-N-(4-hydroxy-phenyl)-N'-phenylmalonamide |
| 5 | 4-OH-PBOMe |
| 6 | 1,2-diphenyl-4-hydroxy-4-(2-(phenylsulfinyl)ethyl]-3,5-pyrazolidinedione. |
| 7 | 4-(1-butyl)-4-hydroxy-1-(4-methoxyphenyl)-2-phenyl-3,5-pyrazolidinedione |
| 8 | 1,2-diphenyl-4-hydroxy-4-(4-methylphenyl)-3,5-pyrazolidinedione |
| 9 | 4-benzyl-1,2-diphenyl-4-hydroxy-3,5-pyrazolidinedione |
| 10 | 4-hydroxy-4-aldehyd-1,2-diphenyl-pyrazolidinedione |
| 11 | 4-butyl-4-hydoxy-1,2-diphenyl-3,5-pyrazolidnedione |

As seen above this time was the analogues: #5, #8 and #9 (Table 1) tested.

The cell number seeded in each well was 180000. The plate was tilted 12 degrees when consolidating the bottom layer containing each of the analogues and the top layer with cells was molded horizontally for getting the cell density gradient.

**Experiment 24, Table 2: Results:**

| No | | Low | Medium | High |
|---|---|---|---|---|
| #5 H | 4-OH-PBOMe | ++ | +(+) | + |
| #5 L | 4-OH-PBOMe | ++(+) | ++(+) | + |
| #8 H | 1,2-diphenyl-4-hydroxy-4-(4-methylphenyl)-3,5-pyrazolidinedione | ++ | ++ | +(+) |
| #8 L | 1,2-diphenyl-4-hydroxy-4-(4-methylphenyl)-3,5-pyrazolidinedione | + | +(+) | +(+) |
| #9 H | 4-benzyl-1,2-diphenyl4-hydroxy-3,5-pyrazolidinedione | ++(+) | +++ | ++ |
| #9 L | 4-benzyl-1,2-diphenyl-4-hydroxy-3,5-pyrazolidinedione | +(+) | + | + |
| H | 4-OH-OPB | ++(+) | + | + |
| L | 4-OH-OPB | - | - | - |
| | | XX-low | X-low | |
| H | 4-OH-OPB | ++ | +(+) | |
| L | 4-OH-OPB | (+) | - | |
| | | Control after 48 hours | Control before incubation | |
| | Control A | ++ | - | |
| | Control B | ++(+) | - | |

| | | | | |
|---|---|---|---|---|
| *: See Fig. for exact concentration of compounds **H:** Area with high cell density, L: Area with low cell density +: Klonal growth present - and +- considered negative, then: (+),+,+(+),++,++(+),+++ in increasing ability to clonal growth. | | | | |

### Results and conclusion:

*None of the analogous compounds to 4-OH-OPB tested, list number 5,8 and 9 (Table 1), did show significant inhibition of clonal growth (Table 2,* *Fig. 1**). In this experiment the control, 4-OH-OPB, showed reduced clonal growth in the densely seeded areas of high or medium concentrations (9 and 3 µM). Clonal growth, however, was significantly inhibited in the concentration range 9 to 0.33 µM. The effect was gone at 0.11 µM.*

### Experiment 25

### The effect of analogues of 4-OH-OPB, #10, #11, Somatotropin and, zathioprin on S100T1 cells in soft agar culture.

Question: What is the effect of analogues of 4-OH-OPB, #10 and #11, Somatotropin and Azathioprin on cloning in soft agar of these cells?

**Experiment 25, Table 1: Available 4-OH-OPB analogues to be tested in experiments 22-25:**

| No | |
|---|---|
| 1 | p-hydroxy-azobenzene A |
| 2 | 2-Butyl-2-hydroxy-N-(4-hydroxy phenyl)-N'-phenyl malonamide |
| 3 | 2-oxo-hexanoic acid phenylamide |
| 4 | 2-Butyl-N-(4-hydroxy-phenyl)-N'-phenylmalonamide |
| 5 | 4-OH-PBOMe |
| 6 | 1,2-diphenyl-4-hydroxy-4-[2-(phenylsulfinyl)ethyl]-3,5-pyrazolidinedione. |
| 7 | 4-(1-butyl)-4-hydroxy-1-(4-methoxyphenyl)-2-phenyl-3,5-pyrazolidmedione |
| 8 | 1,2-diphenyl-4-hydroxy-4-(4-methylphenyl)-3,5-pyrazolidinedione |
| 9 | 4-benzyl-1,2-diphenyl-4-hydroxy-3,5-pyrazolidinedione |
| 10 | 4-hydroxy-4-aldehyd-1,2-diphenyl-pyrazolidinedione |
| 11 | 4-butyl-4-hydoxy-1,2-diphenyl-3,5-pyrazolidnedione |

| | |
|---|---|
| This time the tested analogues are: #10 and #11. In additionwere tested Somatotropin (Gentropin, Pharmacia), MW 20000 and Azathioprin (Imurel) MW, 277.3 | |

As seen above this time was the analogues: #10 and #11 (Table 1) and Somatotropin and Azathioprin tested. The cell number in each well was 180000. The plate was tilted 12 degrees when consolidating the bottom layers containing each of the analogues. The top layer with cells was molded horizontally for getting the cell density gradient. Both layers contained 0.8% Sigma agarose ultra low gelling temperature Sigma agarose type IX-A, A-2576, Eagles MEM and 10% FBS

Azathioprine: 2 tablets of 25 mg each were solved in distilled water and sterile filtered. It was relevant also to test lower dilutions of 4-OH-OPB than done before (Fig. 1).

The aim of this experiment was to see if the addition of the two analogues of 4-OH-OPB and the two drugs inhibited or stimulated clonal growth when added to polyoma virus transformed BHK21/c13 cells, the line S100T1 cells seeded in soft agar. 4-OH-OPB was diluted in 4 steps ending at 0.111 µM.

**Experiment 25, Table 2: Results after 48 hours incubation:**

| No | | Low* | Medium | High |
|---|---|---|---|---|
| #10, H | 4-hydroxy-4-aldehyd-1,2-diphenyl-pyrazolidinedione | ++ | ++ | ((+)) |
| #10, L | 4-hydroxy-4-aldehyd-1,2-diphenyl-pyrazolidinedione | ++ | - | - |
| #11, H | 4-butyl-4-hydoxy-1,2-diphenyl-3,5-pyrazolidnedione | ++ | ++ | ++(+) |
| #11, L | 4-butyl-4-hydoxy-1,2-diphenyl-3,5-pyrazolidnedione | ++ | ++ | ++ |
| H | Somatotropin (Gentropin, Pharmacia) | ++(+) | ++(+) | ++ |
| L | Somatotropin (Gentropin, Pharmacia) | ++(+) | ++(+) | ++ |
| H | Azathioprin (Imurel) | ++ | ++ | ++ |
| L | Azathioprin (Imurel) | ++ | ++ | ++ |
| H | 4-OH-OPB A | ++(+) | ++ | (+) |
| L | 4-OH-OPB B | - | - | - |
| | | Control after 48 h. incub. | Control before incub. | X-low 4-OH-OPB |
| H | Control | ++ | - | ++ |
| L | Control | ++ | - | ++ |

| | | | | |
|---|---|---|---|---|
| *: See Fig. for exact concentration of compounds H: Area with high cell density, L: Area with low cell density +: Klonal growth present. - and +- considered negative, then: (+),+,+(+),++,++(+),+++ in increasing ability to clonal growth. | | | | |

### Results and conclusion

*There has been found 5-6 substances with effect.*

*First: #10, 4-hydroxy-4-aldehyd-1,2-diphenyl pyrazolidinedione inhibited clonal growth of sparsely seeded polyoma virus transformed cell line (S100T1 of BHK21 c13) using about 50 µM without affecting the same cells in densely seeded areas of the same well (**Fig. 1**, Table 2).*

*Next: Use of Somatotropine on these cells seemed to create some large colonies. But other wells with other substances added might have large colonies too. Other pictures might not show big colonies because they were all rare. Therefore, the large colonies could be, but may not be specific for this hormone (**Fig. 1**, Table 2) .*

*This time the control substance 4-OH-OPB showed the specific growth inhibition of only sparsely seeded cells in the range of 1-0.3 µM. When using 0.11 µM no growth inhibition was observed In the well where 3 µM was added, there was a substantial inhibition of the growth in densely seeded areas. But some significant clonal growth occurred This time the preparation was stored for 24 hours in the refrigerator after being solved in DMSO from batch 13 from Syntagon, Sweden.*

*Azathioprin showed no clonal inhibition or stimulation of the cells.*

### Summary of results testing 4-OH-OPB analogues for growth inhibition or possible stimulation of polyoma virus transformed baby hamster cell line.

The tests have been done in different experiments reported elsewhere. The controls are omitted. Therefore, these results can not be evaluated properly, but are useful for developing overview.

Number 1, 2, 4, 6 and 10 may inhibit clonal growth in sparsely seeded areas. None can do so at a low concentration and it seems to be a narrow concentration gap. Only one, number 11, did indicate a possible stimulation at high concentration.

**Experiment 25, Table 3:**

| No | | Low | Medium | High |
|---|---|---|---|---|
| #1 H | p-hydroxy-azobenzene A | +++ | +++ | +(+) |
| #1 L | p-hydroxy-azobenzene B | ++ | ++ | +- |
| #2 H | 2-Butyl-2-hydroxy-N-(4-hydroxy-phenyl)-N'-phenyl malonamide | ++(+) | ++ | ++ |
| #2 L | 2-Butyl-2-hydroxy-N-(4-hydroxy-phenyl)-N'-phenyl malonamide | ++ | ++ | - |
| #3 H | 2-oxo-hexanoic acid phenylamide | ++(+) | ++(+) | ++ |
| #3 L | 2-oxo-hexanoic acid phenylamide | ++(+) | ++(+) | ++ |
| #4 H | 2-Butyl-N-(4-hydroxy-phenyl)-N'-phenylmalonamide | ++ | ++ | ++ |
| #4 L | 2-Butyl-N-(4-hydroxy-phenyl)-N'-phenylmalonamide | ++ | ++ | ((+)) |
| #5 H | 4-OH-PBOMe | ++ | +(+) | + |
| #5 L | 4-OH-PBOMe | ++(+) | ++(+) | + |
| #6 H | 1,2-diphenyl-4-hydroxy-4-[2-(phenylsulfinyl)ethyl]-3,5-pyrazolidinedione. | ++ | ++ | ++ |
| #6 L | 1,2-diphenyl-4-hydroxy4-[2-(phenylsulfinyl)ethyl]-3,5-pyrazolidinedione | ++ | ((+)) | - |
| #7 H | 4-(1-butyl)-4-hydroxy-1-(4-methoxyphenyl)-2-phenyl-3,5-pyrazolidinedione | ++ | ++ | ++ |
| #7 L | 4-(1-butyl)-4-hydroxy-1-(4-methoxyphenyl)-2-phenyl-3,5-pyrazolidmedione | ++ | ++ | (+) |
| #8 H | 1,2-diphenyl-4-hydroxy-4-(4-methylphenyl)-3,5-pyrazolidinedione | +(+) | ++ | ++ |
| #8 L | 1,2-diphenyl-4-hydroxy-4-(4-methylphenyl)-3,5-pyrazolidinedione | +(+) | +(+) | + |
| #9 H | 4-benzyl-1,2-diphenyl-4-hydroxy-3,5-pyrazolidinedione | ++(+) | +++ | ++ |
| #9 L | 4-benzyl-1,2-diphenyl-4-hydroxy-3,5-pyrazolidinedione | +(+) | + | + |
| #10 H | 4-hydroxy-4-aldehy4-1,2-diphenyl-pyrazolidinedione | ++ | ++ | ((+)) |
| #10 L | 4-hydroxy-4-aldehyd-1,2-diphenyl-pyrazolidinedione | ++ | - | - |
| #11 H | 4-butyl-4-hydoxy-1,2-diphenyl-3,5-pyrazolidnedione | ++ | ++ | ++(+) |
| #11 L | 4-butyl-4-hydoxy-1,2-diphenyl-3,5-pyrazolidnedione | ++ | ++ | ++ |

*Conclusion: 4 of the 4-OH-OPB analogues, #1, #2, #6 and # 10 showed complete inhibition of growth in sparsely seeded areas of agar culture of S100T1 cells when the growth in densely seeded areas of the same culture showed significant growth.*

### Experiment 26

### The effect of Benzo(a)pyrene, Altretamine and Sulindac on S100T1 cells in soft agar culture.

Question: What is the effect of Benzo(a)pyrene, Altretamine and Sulindac on S100T1 cells in soft agar? Benzo(a)pyrene is a known carcinogenic agent.

**Experiment 26, Table 1**

| Number | | MW |
|---|---|---|
| 1 | Benzo(a)pyrene | 252.3 |
| 2 | Altretamine | 210.3 |
| 3 | Sulindac | 356.42 |
| 5 | 4-OH-OPB | 340.4 |
| 6 | Control | |

This time the cell number seeded in each well was 172800 in each well. The Falcon 24 well plate was tilted 12 degrees as before when solidifying the bottom layer of 0.3 ml with the test substance. The top layer with the polyoma virus transformed cell line S100T1 was consolidated in a horizontal position as described earlier. Both layers contained 0.8% Sigma agarose ultra low gelling temperature Sigma agarose type IX-A, A-2576, Eagles MEM, 10% FBS.

The aim of this experiment was to see if the addition of the three compounds (Table 1) inhibited or stimulated clonal growth when added to polyoma virus transformed BHK21/c13 cells, the line S100T1 when seeded in soft agar. 4-OH-OPB was also diluted to 0.333 µM.

**Experiment 26, Table 2: Resulting clonal growth of treated cultures.**

| No | | Low* | Medium | High |
|---|---|---|---|---|
| 1 H | Benzo(a)pyzene | ++ | ++ | ++ |
| 1 L | Benzo(a)pyrene | ++ | ++ | +++ |
| 2 H | Altretamine | ++ | ++ | ++ |
| 2 L | Altretamine | ++ | ++ | ++ |
| 3 H | Sulindac | ++(+) | ++ | ++ |
| 3 L | Sulindac | +++ | ++ | + |
| 5 H | 4-OH-OPB | ++ | ++ | ++ |
| 5 L | 4-OH-OPB | (+) | - | - |

| | | Control after 24 hours | Control before incubation | |
|---|---|---|---|---|
| | Control A | ++ | - | |
| | Control B | ++ | - | |

| | | | | |
|---|---|---|---|---|
| *: See Fig. 1 for concentration of compounds H: Area with high cell density, L: Area with low cell density +: Clonal growth present - and +- is considered negative, then: (+),+,+(+),++,++(+),+++ in increasing ability to clonal growth. | | | | |

### Results and Conclusion:

*The results were read after 24 hours. The 4-OH-OPB showed nice and complete inhibition for high and medium concentrations on sparsely seeded cells (**Fig. 1**, Table 2). Low concentration showed break through of clonal growth close to the sparsely seeded limit in the right hand side of the picture. High concentration did not affect the densely seeded parts of the gradient, which might indicate a low toxicity for densely collocated cells. It is our opinion that these cells were analogous to most of the organs in the organism and would indicate organ toxicity in the body. However, after storing 4-OH-OPB solved in DMSO in the refrigerator, a cytotoxic effect had occasionally been observed of high concentrations added to densely seeded S100T1 cells. This is important since in treatment of patients, only toxic or growth-inhibiting effect on sparsely seeded cells is desired.*

*The carcinogenic Benzo(a)pyrene in high dose gave increased clonability (**Fig. 1**, Table 2). This phenomenon is believed to relate to the carcinogenic effect of the compound Sulindac showed both a weak anti-cloning effect on sparsely seeded cells when adding high dose and a clone-inducing effect on the same cell density if the added dose was low. Low dose seemed also to increase growth in densely seeded areas of the same well. No effect on cell growth was observed when adding Altretamine.*

## Claims

1. Use of 4-OH-OPB for preparing a pharmaceutical preparation for the treatment of psoriasis or for the treatment or prophylactics of arteriosclerosis or cancer, with the provision that said cancer is not malignancies derived from CD4 lymphocytes and HIV related Kaposis sarcoma.

2. Use according to claim 1 wherein the diseases are selected from cancer and arteriosclerosis.

3. Use according to claim 1 for preparing a pharmaceutical preparation for the treatment or prophylaxis of cancer.

4. Use according to claim 3 for preparing a pharmaceutical preparation either for the prophylaxis of malignant growth of cancer or for the treatment or prophylaxis of metastatic spread and local infiltration of malignant tumors.

5. Use according to claim 3 for preparing a pharmaceutical preparation for the prophylaxis of malignant growth of cancer.

6. Use according to claim 3 for preparing a pharmaceutical preparation for the treatment or prophylaxis of metastatic spread and local infiltration of malignant tumors.

7. Use according to claim 1 for the preparation of a pharmaceutical preparation for the treatment or prophylaxis of arteriosclerosis.

8. Use according to claim 1 for the preparation of a pharmaceutical preparation for the treatment of psoriasis.

## Patentansprüche

1. Verwendung von 4-OH-OPB zur Herstellung eines pharmazeutischen Präparats für die Behandlung von Psoriasis oder für die Behandlung oder Vorbeugung von Arteriosklerose oder Krebs, mit der Maßgabe, dass der Krebs nicht zu bösartigen Tumoren gehört, die von CD4-Lymphozyten und HIV-bezogenem Kaposi-Sarkom stammen.

2. Verwendung nach Anspruch 1, wobei die Krankheiten aus Krebs und Arteriosklerose ausgewählt sind.

3. Verwendung nach Anspruch 1 zur Herstellung eines pharmazeutischen Präparats für die Behandlung oder Vorbeugung von Krebs.

4. Verwendung nach Anspruch 3 zur Herstellung eines pharmazeutischen Präparats entweder für die Vorbeugung eines bösartigen Krebswachstums oder für die Behandlung oder Vorbeugung einer metastatischen Ausbreitung und Lokalinfiltration von bösartigen Tumoren.

5. Verwendung nach Anspruch 3 zur Herstellung eines pharmazeutischen Präparats für die Vorbeugung eines bösartigen Krebswachstums.

6. Verwendung nach Anspruch 3 zur Herstellung eines pharmazeutischen Präparats für die Behandlung oder Vorbeugung einer metastatischen Ausbreitung und Lokalinfiltration von bösartigen Tumoren.

7. Verwendung nach Anspruch 1 zur Herstellung eines pharmazeutischen Präparats für die Behandlung oder Vorbeugung von Arteriosklerose.

8. Verwendung nach Anspruch 1 zur Herstellung eines pharmazeutischen Präparats für die Behandlung von Psoriasis.

## Revendications

1. Utilisation de 4-OH-OPB pour la préparation d'une préparation pharmaceutique pour le traitement du psoriasis ou pour le traitement ou la prophylaxie de l'artériosclérose ou du cancer, à condition que ledit cancer n'est pas de tumeurs malignes dérivées des lymphocytes CD4 et du sarcome de Kaposi lié au VIH.

2. Utilisation selon la revendication 1, dans laquelle les maladies sont sélectionnées du cancer et de l'artériosclérose.

3. Utilisation selon la revendication 1 pour la préparation d'une préparation pharmaceutique pour le traitement ou la prophylaxie du cancer.

4. Utilisation selon la revendication 3 pour la préparation d'une préparation pharmaceutique soit pour la prophylaxie de la croissance maligne du cancer soit pour le traitement ou la prophylaxie de la propagation métastatique et l'infiltration locale des tumeurs malignes.

5. Utilisation selon la revendication 3 pour la préparation d'une préparation pharmaceutique pour la prophylaxie de la croissance maligne du cancer.

6. Utilisation selon la revendication 3 pour la préparation d'une préparation pharmaceutique pour le traitement ou la prophylaxie de la propagation métastatique et l'infiltration locale de tumeurs malignes.

7. Utilisation selon la revendication 1 pour la préparation d'une préparation pharmaceutique pour le traitement ou la prophylaxie de l'artériosclérose.

8. Utilisation selon la revendication 1 pour la préparation d'une préparation pharmaceutique pour le traitement du psoriasis.
